# EUROPÄISCHE PATENTSCHRIFT

(11) **EP 2 283 021 B1**
(45) Veröffentlichungstag und Bekanntmachung des Hinweises auf die Patenterteilung: **08.01.2014**
(21) Anmeldenummer: 09749778.8
(22) Anmeldetag: 18.05.2009
(51) Int. Cl.: C07D 487/22, C07D 519/00, C09B 47/067

(54) **SCHALTBARE EFFEKTSTOFFE**
SWITCHABLE SPECIAL EFFECT SUBSTANCES
SUBSTANCES À EFFET COMMUTABLES

(30) Priorität: 19.05.2008 EP 08156466
(43) Veröffentlichungstag der Anmeldung: 16.02.2011
(73) Patentinhaber: BASF SE, 67056 Ludwigshafen (DE)
(72) Erfinder: GEßNER, Thomas, 69120 Heidelberg (DE); SENS, Rüdiger, 67069 Ludwigshafen (DE); EBERT, Sophia, 68165 Mannheim (DE)
(86) Internationale Anmeldenummer: PCT/EP2009/055961
(87) Internationale Veröffentlichungsnummer: WO 2009/141288

(56) Entgegenhaltungen:
- WO-A-02/077081
- WO-A-2007/099059
- WO-A-2009/040000
- WO-A1-2007/071957
- DE-A1- 2 429 258
- DE-A1-102006 011 271
- US-A1- 2008 036 373

## Beschreibung

Die vorliegende Erfindung betrifft Verfahren zur Änderung der Absorption elektromagnetischer Strahlung durch ausgewählte Verbindungen. Die Erfindung umfasst ebenfalls die Verwendung dieser Verfahren zum Nachweis der Markierung von Materialien, zum Hervorrufen einer Farbänderung, zum Laserschweißen, im Wärmemanagement oder zur Bereitstellung von Radikalfängern oder Photoinitiatoren, sowie die Verwendung zum Nachweis von Sauerstoff. Weiterhin betrifft die Erfindung Verfahren zur Reaulierung der Absorption oder Transmission von elektromagnetischer Strahlung durch Materialien. Bestimmte ausgewählte Verbindungen, sowie Materialien sind ebenfalls Gegenstand der Erfindung. Weiterhin sind Polymere, Lacke oder Gläser, die ausgewählte Verbindungen enthalten ebenfalls Gegenstand der vorliegenden Erfindung.

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstandes nicht nur in der jeweils konkret angegebenen Kombination, sondern auch in anderen Kombinationen verwendbar sind, ohne den Rahmen der Erfindung zu verlassen. Bevorzugt bzw. ganz bevorzugt sind die Ausführungsformen der vorliegenden Erfindung in denen alle Merkmale die bevorzugten bzw. ganz bevorzugten Bedeutungen haben.

Es ist bekannt, dass Perylentetracarbonsäure(di)imide, die am Imidstickstoff kovalent gebundene Donorreste enthalten, wie z. B. Amino (Langhals et al., Chem. Eur. J. 1998, 4, 2110 - 2116; Mohr et al., Anal. Chem. 2000, 72, 1084- 1087) oder Anilino (Zhang et al., J. Am. Chem. Soc. 2002, 124, 10640 -10641.), einen photoinduzierten intramolekularen Elektronentransfer zeigen können und deshalb wenig zur Fluoreszenz neigen. Durch Protonierung des Donorrestes oder Wechselwirkung des Donorrestes mit Metallionen kann die Fluoreszenzlöschung aufgehoben werden. Deswegen wurden solche Verbindungen zum Beispiel als pH- oder metallionenempfindliche Fluoreszenzsensoren vorgeschlagen (Sauer et al., Angew. Chem. 2003, 115, 1834 - 1835).

Mit 4-Carbazol-9-yl-butan-1-sulfonsäure als Donor wurde ein intermolekularer Elektronentransfer auf N,N'-Di-(2-dimethylaminoethyl)-1,6,7,12-tetrachlorperylen-3,4,9,10-tetracarbonsäurediimid (Akzeptor) in Lösung beschrieben, wenn sich die beiden Moleküle durch elektrostatische Kräfte bedingt, nahe kommen (Bonnet et al., Synthetic Metals 156 (2006) 1292 - 1298).

O'Neil et al. (Science 257 (1992) 63 - 65) beschreiben N,N'-diphenyl-3,4,9,10-perylenbis(dicarboximide), die an den Phenylgruppen Porphyrinsubstituenten tragen. Nach der Absorption von Laserpulsen mit einer Wellenlänge von 585 nm wird eine transiente Absorption bei 713 nm und eine weitere Absorption bei 546 nm beobachtet. Eine Absorption im Infrarot wird nicht beschrieben. Die Lebensdauer der transienten Absorption liegt im Bereich von wenigen 100 Picosekunden.

Aus den Patentanmeldungen DE 10 2006 011 269 A1, DE 10 2006 011 270 A1 und DE 10 2006 011 271 A1 sind Radikalanionen bekannt, die durch die Reduktion von Perylenfarbstoffen, Perylen-3,4:9,10-tetracarbonsäurebisimiden erhältlich sind. Die Radikalanionen bzw. deren Salze werden unter anderem als Farbmittel eingesetzt.

In der DE 24 29 258 A1 werden Polyimidverbindungen beschrieben, die zur Massefärbung von Kunststoffen geeignet sind. Diese Polyimidverbindungen können Rylengruppen enthalten.

In keinem der oben angeführten Dokumente des Standes der Technik wurde in Verbindung mit der Anregung durch elektromagnetische Strahlung eine anhaltende und kontrollierbare Änderung der Absorption elektromagnetischer Strahlung durch Moleküle beschrieben.

Um die Absorption von elektromagnetischer Strahlung, beispielsweise die IR-Absoption von Materialien zu regeln, werden den Materialien häufig Verbindungen zugegeben, die im IR absorbieren (WO 02/077081). Je nach Anwendung oder Anforderungsprofil werden den Materialien diese Verbindungen in unterschiedlichen Konzentrationen zugegeben. Hierdurch wird diesen Materialien eine permanente IR Absorption verliehen. Diese IR Absorption ist jedoch im Allgemeinen nicht schaltbar.

Zur sichtbaren bzw. unsichtbaren Markierung von Materialien werden häufig im sichtbaren Spektralbereich bzw. im IR absorbierende oder fluoreszierende Substanzen eingesetzt (WO 98/52950, WO 99/56125, WO 2007/099059). Die Markierung dieser Materialien ist jedoch permanent und lässt sich ohne die Entfernung der Markierstoffe nicht einfach an und abschalten.

Eine Aufgabe der vorliegenden Erfindung war es daher Verbindungen bereitzustellen deren Absorption im sichtbaren oder IR Wellenlängenbereich nicht permanent sondern schaltbar ist. Eine weitere Aufgabe der vorliegenden Erfindung war es Substanzen aufzufinden, deren regulierbare (schaltbare) Absorption eine Verwendung dieser Substanzen als Markierstoffe von Materialien zulässt. Eine andere Aufgabenstellung der Erfindung war es einfache Verfahren zur Verfügung zu stellen, die es ermöglichen die Absorption von elektromagnetischer Strahlung durch Materialien zu regeln.

Diese und andere Aufgaben werden, wie aus dem Offenbarungsgehalt der vorliegenden Erfindung ersichtlich, durch die verschiedenen Ausführungsformen der erfindungsgemäßen Verfahren zur Änderung der Absorption elektromagnetischer Strahlung durch chemische Verbindungen oder Materialien, die chemische Verbindungen enthalten, die im weiteren beschrieben sind, gelöst.

Gegenstand der Erfindung ist daher ein Verfahren zur Änderung der Absorption elektromagnetischer Strahlung durch eine oder mehre Verbindungen der allgemeinen Formeln (I) 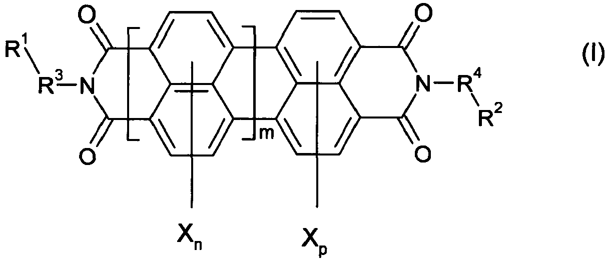 wobei diese Verbindungen mit elektromagnetischer Strahlung der Wellenlänge von 300 bis 750 nm bestrahlt werden und
durch die Bestrahlung mit elektromagnetischer Strahlung der Wellenlänge von 300 bis 750 nm eine bathochrome Verschiebung des Absorptionsspektrums der Verbindungen der allgemeinen Formel (I) resultiert
wobei die Symbole und Indizes folgende Bedeutung haben
- R¹, R²: unabhängig voneinander, gleich oder verschieden, Amino, C₁-C₈-Alkylamino, C₁-C₈-Dialkylamino, Arylamino, Diarylamino, N-Heterocyclyl, Triarylaminyl, Hydroxy, C₁-C₈-alkoxy, Aryloxy,
- R³, R⁴: unabhängig voneinander, gleich oder verschieden, Einfachbindung, C₁-C₈-Alkylen, C₃-C₆-Cycloalkylen, Arylen, C₈-C₁₄-Phenylalkylen,
- X: unabhängig voneinander, gleich oder verschieden, C₁-C₂₀-Alkoxy, C₁-C₂₀-Alkylthio, Aryloxy, Arylthio, Halogen, Cyan, CO₂R, SO₃R, SO₂R, oder eine Gruppe 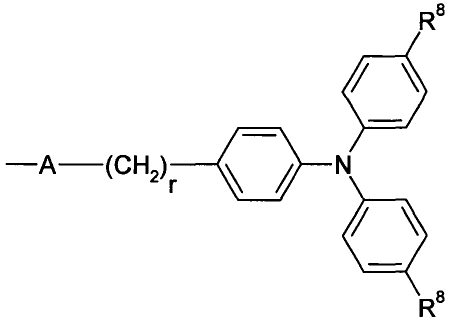 mit
- A: S, SO₂,
- r: 2, 3, 4, 5, 6,
- R⁸: C₁-C₂₀-Alkyl, C₁-C₂₀-Alkoxy,
und
falls m=1 können verschiedene X gemeinsam eine Thiogruppe darstellen,
- R: unabhängig voneinander, gleich oder verschieden, H, C₁-C₈-Alkyl, Aryl,
- n, p: 0, 1, 2, 3 oder 4,
- m: 0, 1, 2 oder 3,
und wobei
die Substituenten R, R¹, R², R³ oder R⁴ jeweils an beliebiger Position durch ein oder mehrere, gegebenenfalls durch Wasserstoff abgesättigte, Heteroatome unterbrochen sein können, wobei die Anzahl dieser Heteroatome nicht mehr als 4, bevorzugt nicht mehr als 3, ganz besonders bevorzugt nicht mehr als 2 und insbesondere nicht mehr als 1 beträgt, und/oder jeweils an beliebiger Position, allerdings nicht mehr als fünfmal, bevorzugt nicht mehr als viermal und besonders bevorzugt nicht mehr als dreimal, durch NR⁵R⁶, CONR⁵R⁶, COOM, COOR⁵, SO₃M, SO₃R⁵,
wobei,
- R⁵, R⁶: unabhängig voneinander, gleich oder verschieden, H, C₁-C₈-Alkyl, Aryl,
- M: H, Alkalimetall, NR⁷₄,
- R⁷: unabhängig voneinander H, C₁-C₈-Alkyl,
CN, NO₂, C₁-C₂₀-Alkyl, C₁-C₂₀-Alkoxy, Aryl, Aryloxy, Heterocyclen, Heteroatome oder Halogen substituiert sein können, wobei diese ebenfalls maximal zweimal, bevorzugt maximal einmal mit den genannten Gruppen substituiert sein können.

Ausdrücke der Form Cₐ-C_{b} bezeichnen im Rahmen dieser Erfindung chemische Verbindungen oder Substituenten mit einer bestimmten Anzahl von Kohlenstoffatomen. Die Anzahl an Kohlenstoffatomen kann aus dem gesamten Bereich von a bis b, einschließlich a und b gewählt werden, a ist mindestens 1 und b immer größer als a. Eine weitere Spezifizierung der chemischen Verbindungen oder der Substituenten erfolgt durch Ausdrücke der Form Cₐ-C_{b}-V. V steht hierbei für eine chemische Verbindungsklasse oder Substituentenklasse, beispielsweise für Alkylverbindungen oder Alkylsubstituenten.

Als Infrarotstrahlung (kurz IR-Strahlung) werden im Rahmen der vorliegenden Erfindung elektromagnetische Wellen im Spektralbereich zwischen sichtbarem Licht und den längerwelligen Mikrowellen bezeichnet. Dies entspricht einem Wellenlängenbereich von etwa 780 nm bis 1 mm. Bei kurzwelliger IR-Strahlung (ab 760 nm) spricht man oft von nahem Infrarot (near infrared, NIR), bei Wellenlängen von ca. 5-25 Mikrometer von mittlerem Infrarot (mid infrared, MIR). Extrem langweilige IR-Strahlung (25 µm-1 mm) bezeichnet man als fernes Infrarot (far infrared, FIR). Infrarotstrahlung ist ein Teil der Wärmestrahlung.

Als sichtbares Licht werden im Rahmen der vorliegenden Erfindung elektromagnetische Wellen im Spektralbereich von etwa 380 nm bis 780 nm bezeichnet.

Substanzen die elektromagnetische Strahlung im Wellenlängenbereich der IR-Strahlung absorbieren werden im Rahmen der vorliegenden Erfindung auch als IR-Absorber bezeichnet. Bevorzugt weisen IR-Absorber eine Absorption im Wellenlängenbereich von 760 bis 2000 nm, ganz bevorzugt von 780 bis 1500 nm und einen Extinktionskoeffizienten für IR-Strahlung von mindestens 100 l/(cm * mol) auf. Bevorzugt liegt der Extinktionskoeffizienten für IR-Strahlung über 1000 l/(cm * mol) und ganz bevorzugt über 104 l/(cm * mol).

Substanzen die sichtbares Licht absorbieren werden im Rahmen der vorliegenden Erfindung auch als farbig bezeichnet. Bevorzugt weisen farbige Substanzen einen Extinktionskoeffizienten für sichtbares Licht von mindestens 100 l/(cm * mol) auf. Bevorzugt liegt der Extinktionskoeffizienten für sichtbares Licht über 1000 l/(cm * mol) und ganz bevorzugt über 104 l/(cm * mol).

Halogen steht für Fluor, Chlor, Brom, oder Iod, vorzugsweise für Fluor, Chlor oder Brom, besonders bevorzugt für Chlor oder Brom.

Im Einzelnen haben die für die verschiedenen Substituenten angegebenen Sammelbegriffe folgende Bedeutung:
C₁-C₂₀-Alkyl: geradkettige oder verzweigte Kohlenwasserstoffreste mit bis zu 20 Kohlenstoffatomen, beispielsweise C₁-C₁₀-Alkyl oder C₁₁-C₂₀-Alkyl, bevorzugt C₁-C₈-Alkyl beispielsweise C₁-C₃-Alkyl, wie Methyl, Ethyl, Propyl, Isopropyl, oder C₄-C₆-Alkyl, n-Butyl, sec-Butyl, tert.-Butyl, 1,1-Dimethylethyl, Pentyl, 2-Methylbutyl, 1,1-Dimethylpropyl, 1,2-Dimethylpropyl, 2,2-Dimethylpropyl, 1-Ethylpropyl, Hexyl, 2-Methylpentyl, 3-Methyl-pentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 2-Ethylbutyl, 1,1,2-Trimethylpropyl, 1,2,2-Tri-methylpropyl, 1-Ethyl-1-methylpropyl, 1-Ethyl-2-methylpropyl, oder C₇-C₈-Alkyl, wie Heptyl, Octyl, 2-Ethyl-hexyl, 2,4,4-Trimethylpentyl, 1,1,3,3-Tetramethylbutyl.
Aryl: ein ein- bis dreikemiges aromatisches Ringsystem enthaltend 6 bis 14 Kohlenstoffringglieder, z. B. Phenyl, Naphthyl oder Anthracenyl, bevorzugt ein ein- bis zweikerniges, besonders bevorzugt ein einkerniges aromatisches Ringsystem.
Aryloxy ist ein ein- bis dreikerniges aromatisches Ringsystem (wie vorstehend genannt), welches über ein Sauerstoffatom (-O-) angebunden ist, bevorzugt ein ein- bis zweikerniges, besonders bevorzugt ein einkerniges aromatisches Ringsystem.
Arylthio ist ein ein- bis dreikerniges aromatisches Ringsystem (wie vorstehend genannt), welches über ein Sauerstoffatom (-S-) angebunden ist, bevorzugt ein ein- bis zweikerniges, besonders bevorzugt ein einkerniges aromatisches Ringsystem.
C₁-C₈-Alkylen: geradkettige oder verzweigte Kohlenwasserstoffreste mit 1 bis 8 Kohlenstoffatomen, beispielsweise C₁-C₆-Alkylen, insbesondere Methylen, Dimethylen, Trimethylen, Tetramethylen, Pentamethylen oder Hexamethylen.
C₈-C₁₄-Phenylalkylen: geradkettige oder verzweigte Kohlenwasserstoffreste mit 8 bis 14 Kohlenstoffatomen, wobei die Alkylenkette durch eine Phenylengruppe unterbrochen ist oder eine endständige Phenylengruppe enthält, bevorzugt C₉-C₁₀-Phenylakylen.
Heterocyclen: fünf- bis zwölfgliedrige, bevorzugt fünf- bis neungliedrige, besonders bevorzugt fünf- bis sechsgliedrige, Sauerstoff-, Stickstoff- und/oder Schwefelatome, gegebenenfalls mehrere Ringe aufweisende Ringsysteme wie Furyl, Thiophenyl, Pyrryl, Pyridyl, Indolyl, Benzoxazolyl, Dioxolyl, Dioxyl, Benzimidazolyl, Benzthiazolyl, Dimethylpyridyl, Methylchinolyl, Dimethylpyrryl, Methoxyfuryl, Dimethoxypyridyl, Difluorpyridyl, Methylthiophenyl, Isopropylthiophenyl oder tert.-Butylthiophenyl.
N-Heterocyclyl: Heterocyclus angebunden über ein Stickstoffatom, bevorzugt Pyrrolidino, Piperidino, N'-Alkylpiperazinyl, Morpholino, Pyrrolyl, Indolyl, Carbazolyl, Phenothiazinyl, ganz bevorzugt Pyrrolidino, Piperidino, Morpholino, Carbazolyl.
C₁-C₂₀-Alkoxy bedeutet eine geradkettige oder verzweigte Alkylgruppe mit 1 bis 20 Kohlenstoffatomen (wie vorstehend genannt), welche über ein Sauerstoffatom (-O-) an gebunden sind, beispielsweise C₁-C₁₀-Alkoxy oder C₁₁-C₂₀-Alkoxy, bevorzugt C₁-C₈Alkyloxy, insbesondere bevorzugt C₁-C₃-Alkoxy, wie beispielweise Methoxy, Ethoxy, Propoxy.
C₁-C₂₀-Alkylthio bedeutet eine geradkettige oder verzweigte Alkylgruppe mit 1 bis 20 Kohlenstoffatomen (wie vorstehend genannt), welche über ein Sauerstoffatom (-S-) an gebunden sind, beispielsweise C₁-C₁₀-Alkylthio oder C₁₁-C₂₀-Alkylthio, bevorzugt C₁-C₈-Alkylthio, insbesondere bevorzugt C₁-C₃-Alkylthio.
Heteroatome sind bevorzugt Sauerstoff, Stickstoff, Schwefel oder Phosphor.
Triarylaminyl ist bevorzugt N,N-Diarylanilin-4-yl.
C₁-C₈-Dialkylamino bedeutet eine Aminogruppe mit zwei Alkylsubstituenen, wobei jeder Alkylsubstituenten unabhängig vom anderen von 1 bis 8 Kohhlenstoffatome aufweisen kann. Bevorzugt sind die beiden Alkylsubstituenten gleich.

Für die Durchführung des erfindungsgemäßen Verfahrens können selbstverständlich ebenfalls Mischungen der Verbindungen der allgemeinen Formel (I) eingesetzt werden.

Bevorzugt haben die Symbole der allgemeinenen Formeln (I) folgende Bedeutungen:
- R¹, R²: unabhängig voneinander, gleich oder verschieden, Amino, C₁-C₈-Alkylamino, C₁-C₈-Dialkylamino, Arylamino, Diarylamino, N,N-Diarylanilin-4-yl, Carbazolyl,
- R³, R⁴: unabhängig voneinander, gleich oder verschieden, Einfachbindung, C₁-C₈-Alkylen,
- X: unabhängig voneinander, gleich oder verschieden, C₁-C₂₀-Alkoxy, Aryloxy, Halogen, Cyan, SO₂R, oder eine Gruppe 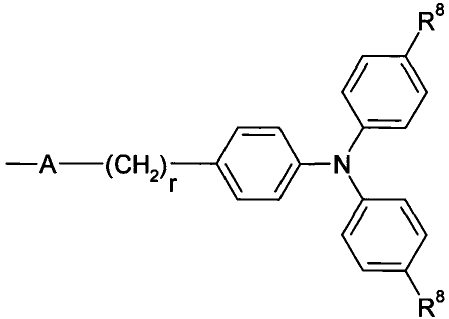 mit
A S, SO₂,
r 2, 3, 4, 5, 6,
R⁸ C₁-C₂₀-Alkyl, C₁-C₂₀-Alkoxy,
- m: 1 oder 2,
wobei die Substituenten R¹, R², R³ oder R⁴ jeweils an beliebiger Position wie oben erwähnt substituiert oder durch Heteroatome unterbrochen sein können und alle anderen Symbole und Indizes haben die gleiche Bedeutung wie anfangs erwähnt haben. Besonders bevorzugt sind hierbei die Substituenten R¹ und R² durch C₁-C₂₀-Alkyl- oder C₁-C₂₀-Alkoxygruppen substituiert.

Besonders bevorzugt haben die Symbole der allgemeinen Formeln (I) folgende Bedeutungen:
- R¹, R²: beide gleich Amino, C₁-C₈-Alkylamino, C₁-C₈-Dialkylamino, Arylamino, Diarylamino, N,N-Diarylanilin-4-yl, Carbazolyl,
- R³, R⁴: unabhängig voneinander, gleich oder verschieden, C₁-C₈-Alkylen,
- X: unabhängig voneinander, gleich oder verschieden, Halogen, Cyan, Alkoxy,
oder eine Gruppe 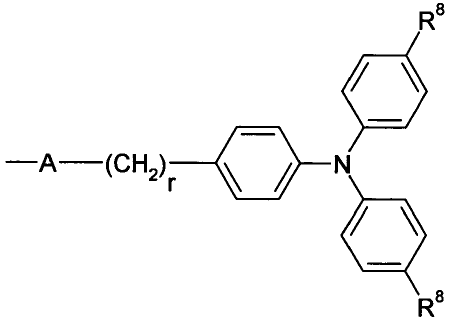 mit
A S, SO₂,
r 2, 3, 4, 5, 6,
R⁸ C₁-C₂₀-Alkyl, C₁-C₂₀-Alkoxy,
- n, p: 0, 1 oder 2,
- m: 1 oder 2,
wobei die Substituenten R¹, R², R³ oder R⁴ jeweils an beliebiger Position wie oben erwähnt substituiert oder durch Heteroatome unterbrochen sein können und alle anderen Symbole und Indizes die gleiche Bedeutung wie anfangs erwähnt haben. Besonders bevorzugt sind hierbei die Substituenten R¹ und R² durch C₁-C₂₀-Alkyl- oder C₁-C₂₀-Alkoxygruppen substituiert.

Allgemeine Verfahren zur Herstellung von Verbindungen der allgemeinen Formeln (I) sind dem Fachmann beispielsweise aus F. Würthner, Chem. Commun. 2004, S. 1564 - 1579, bekannt. Beispielsweise lassen sich die Verbindungen der allgemeinen Formeln (I) durch Umsetzung von Perylen-bis(dicarbonsäureanhydride) bzw. Perylenimid-dicarbonsäureanhydrid mit Aminen in einem Lösungsmittel bei in der Regel 80 - 160 °C erhalten, wobei beispielsweise bei einer Umsetzungen mit Arylaminen Lewissäuren wie z. B. Zinkacetat zugesetzt werden.

Erfindungsgemäß wird die Änderung der Absorption elektromagnetischer Strahlung durch eine Bestrahlung von Verbindungen der allgemeinen Formel (I) mit elektromagnetischer Strahlung der Wellenlänge von 300 bis 750 nm durchgeführt. Bevorzugt erfolgt die Bestrahlung in einem Wellenlängenbereich von 400 bis 700 nm. Eine mögliche Erklärung für die Änderung der Absorption ist, dass durch die Bestrahlung veränderte Verbindungen, beispielsweise Radikalanionen der Verbindungen der allgemeinen Formel (I) entstehen. Diese veränderten Verbindungen, die durch die oben beschriebene Bestrahlung aus den Verbindungen der allgemeinen Formel (I) entstehen, werden im Rahmen der vorliegenden Erfindung als "durch die Bestrahlung veränderte Verbindungen" oder als "Verbindungen mit veränderter Absorption von elektromagnetischer Strahlung" bezeichnet.

Die Bestrahlung erfolgt beispielsweise mit einer weißen Lichtquelle, bevorzugt durch Sonnenstrahlung oder ebenfalls bevorzugt mit einer Xenon-Lampe. Die Bestrahlung kann ebenfalls mit Hilfe von schmalbandiger elektromagnetischer Strahlung, bevorzugt mit Hilfe von Lasern oder Laserdioden erfolgen.

Die Bestrahlung der Verbindungen der allgemeinen Formeln (I) oder von Materialien, die in Kontakt mit diesen Verbindungen sind kann, abhängig beispielsweise vom gewünschten Einsatzzweck, einmalig, zeitlich wiederkehrend oder auch kontinuierlich erfolgen. Die Bestrahlungsdauer liegt, falls keine kontinuierliche Bestrahlung erfolgt, üblicherweise im Bereich von Millisekunden bis Stunden. Häufig werden Bestrahlungsdauern im Bereich von wenigen Sekunden bis zu einer Stunde eingesetzt. Die Bestrahlungsintensität ist häufig von untergeordneter Bedeutung, es muss nur sichergestellt werden, dass durch die Dauer und die Intensität eine für die jeweilige Verwendung ausreichende geänderte Absorption erreicht wird. Eine höhere Bestrahlungsintensität verkürzt in der Regel die hierfür nötige Bestrahlungsdauer. Eine zu hohe Bestrahlungsintensität kann jedoch zu einer Zerstörung der Verbindungen der allgemeinen Formeln (I) führen. Der Fachmann ist in der Lage durch Routineversuche geeignete Bestrahlungsintensitäten und -dauern für die jeweilige Verbindung zu ermitteln.

Die Änderung der Absorption elektromagnetischer Strahlung, die durch das erfindungsgemäße Verfahren hervorgerufen wird, bedeutet, dass die durch die Bestrahlung veränderten Verbindungen Absorptionsbanden für elektromagnetische Strahlung in Bereichen des elektromagnetischen Spektrums aufweisen, die vor der Bestrahlung nicht vorhanden waren. Bevorzugt bewirken diese Änderungen der Absorptionseigenschaften eine sichtbare Farbveränderung. Durch das erfindungsgemäße Verfahren erfolgt eine bathochrome Verschiebung des Absorptionsspektrums, d.h. dass die durch Bestrahlung veränderten Verbindungen in der Lage sind elektromagnetische Strahlung mit größerer Wellenlänge zu absorbieren, als die Verbindungen der allgemeinen Formel (I) vor der Bestrahlung. Beispielsweise erfolgt bevorzugt dadurch eine optisch wahrnehmbare Farbänderung mit diesen Verbindungen, insbesondere auch von einem farblosen zu einem farbigen Eindruck. Weiterhin entsteht bevorzugt durch die Bestrahlung eine vorher nicht vorhandene Absorption im IR-Bereich, d.h., dass die durch die Bestrahlung veränderten Verbindungen IR-Absorber sind.

Im Allgemeinen zeigen die Verbindungen der allgemeinen Formel (I) nach erfolgter einmaliger Bestrahlung die geänderte Absorption der elektromagnetischen Strahlung, abhängig von der Molekülstruktur beziehungsweise Umgebungseffekten, für einen an die Bestrahlung anschließenden Zeitdauer von einigen Millisekunden bis Tagen. Bevorzugt bleibt die geänderte Absorption für eine Zeitdauer im Bereich von einigen Sekunden bis mehreren Stunden erhalten.

Die durch die Bestrahlung hervorgerufenen Änderungen der Absorption können wie oben beschrieben im Allgemeinen wieder verschwinden, bleiben aber häufig auch für einen Zeitraum von mehreren Stunden oder Tagen erhalten.

Der Ausschluss von sauerstoffhaltigen Gasen, insbesondere von Luft verlängert den Zeitraum der hervorgerufenen Änderungen in der Absorption von elektromagnetischer Strahlung. Umgekehrt lässt sich mit Hilfe dieses Effektes die Gegenwart von Sauerstoff nachweisen, d.h. durch Bestrahlung können die Verbindungen der allgemeinen Formel (I) als Sauerstoffsonden oder-indikatoren eingesetzt werden. In Gegenwart von Sauerstoff wird häufig die Zeitdauer der hervorgerufenen Änderungen der Absorption deutlich verkürzt. Durch die Bestimmung der Verkürzung der Zeitdauer lässt sich der Gehalt an Sauerstoff bestimmen. Daher ist ein weiterer Gegenstand der Erfindung die Verwendung von Verbindungen der allgemeinen Formel (I) nach erfolgter Bestrahlung zum Nachweis von Sauerstoff.

Weiterhin finden Verbindungen der allgemeinen Formel (I) nach der erfindungsgemäßen Bestrahlung Verwendung als Radikalfänger, mit dem Vorteil, dass die Verwendung als Radikalfänger kontrolliert durch Bestrahlung schaltbar ist.

Weiterhin finden Verbindungen der allgemeinen Formel (I) nach der erfindungsgemäßen Bestrahlung Verwendung als Photoinitiatoren, beispielsweise für Polymerisationsreaktionen.

Die oben beschriebenen erfindungsgemäßen Verfahren finden ihre Verwendung auch in allen Feldern der Technik, in denen es nützlich ist durch Bestrahlung von Verbindungen der allgemeinen Formel (I) Verbindungen zu erhalten, deren Absorptionseigenschaften bzgl. elektromagnetischer Strahlung eine Veränderung aufweisen. Diese Eigenschaftsänderung lässt sich häufig als Schalter ausnutzen. Einem Material, das in Kontakt mit den Verbindungen der allgemeinen Formel (I) ist, werden durch diese Eigenschaftsänderung der Verbindungen häufig ebenfalls neue Eigenschaften verliehen. Die Menge an Verbindungen der allgemeinen Formel (I), die mit einem Material in Kontakt gebracht wird, insbesondere dem Material zugesetzt wird, ist von den Details der jeweiligen Anwendung abhängig und kann über einen weiten Bereich variieren.

In einer bevorzugten Ausführungsform werden die Verbindungen der allgemeinen Formeln (I) in einem Lösungsmittel gelöst und bestrahlt um bevorzugt als IR-Absorber eingesetzt zu werden oder eine Farbänderung hervorzurufen. Als geeignete Lösungsmittel kommen beispielsweise organische Lösungsmittel, beispielsweise Toluol, Xylol, NMP, DMF, Dimethylacetamid, Pyridin, Dichlormethan, Tetrahydrofuran und Essigester in Frage. Bevorzugt werden, NMP, Pyridin, DMF oder Dimethylacetamid eingesetzt. Selbstverständlich lassen sich auch Mischungen von Lösungsmitteln einsetzten.

Die Zeitdauer der erfindungsgemäßen Verwendbarkeit als IR-Absorber oder der Bereitstellung einer Farbänderung der Verbindungen der allgemeinen Formeln (I) ist abhängig von der jeweiligen Struktur des Moleküls und gegebenenfalls der Art des Materials mit der die Verbindung in Kontakt steht. Die Zeitdauer der Verwendbarkeit variiert in der Regel nach erfolgter Bestrahlung im Bereich von wenigen Millisekunden bis Stunden, häufig liegt die Zeitdauer der Verwendbarkeit im Bereich von wenigen Sekunden bis zu mehreren Stunden.

Erfolgt eine kontinuierliche oder zeitlich wiederkehrende Bestrahlung der Verbindungen der allgemeinen Formeln (I) oder der Materialien, die in Kontakt mit diesen Verbindungen sind, so kann auch eine kontinuierliche Verwendbarkeit als IR-Absorber oder die kontinuierliche Bereitstellung einer Farbänderung erreicht werden.

Ein weiterer Gegenstand der vorliegenden Erfindung ist ein Verfahren zur, insbesondere schaltbaren, Regulierung der Absorption elektromagnetischer Strahlung durch ein Material, wobei eine oder mehrere Verbindungen der allgemeinen Formel (I) in Kontakt mit diesem Material gebracht werden und diese Verbindungen mit elektromagnetischer Strahlung der Wellenlänge von 300 bis 750 nm bestrahlt werden und durch die Bestrahlung mit elektromagnetischer Strahlung der Wellenlänge von 300 bis 750 nm eine bathochrome Verschiebung des Absorptionsspektrums der Verbindungen der allgemeinen Formel (I) resultiert. Bevorzugt werden die Verbindungen der allgemeinen Formel (I) dem Material zugesetzt, d.h. sie sind im Material enthalten, insbesondere in homogener Verteilung. Ebenfalls bevorzugt befinden sich die Verbindungen der allgemeinen Formel (I) im Wesentlichen an der Oberfläche des Materials, beispielsweise in Form einer Schicht enthaltend die Verbindungen der allgemeinen Formel (I), insbesondere in Form einer Lackschicht.

Die Menge an Verbindungen der allgemeinen Formel (I) beträgt im Allgemeinen von 0,001 bis 6,0 g/m², bezogen auf die Oberfläche des Materials die bestrahlt wird. Bevorzugt werden 0,03 bis 3,0 g/m², besonders bevorzugt 0,05 bis 2,5 g/m² und insbesondere von 0,09 bis 1,0 g/m² eingesetzt.

Die Verbindungen werden im Allgemeinen in Mengen von 0,02 ppm bis 10000 ppm bezogen auf das Gewicht des Materials eingesetzt. Bevorzugt werden die Verbindungen in einer Menge von 1 ppm bis 5000 ppm, bevorzugt von 2 ppm bis 3000 ppm, insbesondere von 5 ppm bis 2000 ppm eingesetzt.

Bevorzugt ist im erfindungsgemäßen Verfahren das Material ein Fenster, beispielsweise ein Glasfenster oder ein Kunststofffenster. Das erfindungsgemäße Verfahren lässt sich für alle Arten von Fenster, beispielsweise in Gebäuden, Kraftfahrzeugen, Flugzeugen oder anderen Maschinen anwenden.

Bevorzugt wird mit Hilfe des erfindungsgemäßen Verfahrens die Absorption elektromagnetischer Strahlung durch ein Material im IR-Wellenlängenbereich reguliert.

Im Allgemeinen werden durch das erfindungsgemäße Verfahren zur Regulierung der IR-Absorption durch ein Material mindestens 5% der einfallenden IR-Strahlung absorbiert, bevorzugt mindestens 10%, besonders bevorzugt mindestens 20% und insbesondere mindestens 50%. In einer speziellen Ausführungsform werden mehr als 80% der einfallenden IR-Strahlung absorbiert, insbesondere mehr als 90%.

In einer spezifischen Ausführungsform des erfindungsgemäßen Verfahrens zur Regulierung der IR-Absorption befinden sich die Verbindungen der allgemeinen Formel (I) an der Oberfläche eines Fensters. Durch die Sonnenstrahlung erfolgt eine wie oben beschriebene Bestrahlung der Verbindungen der allgemeinen Formel (I) und es resultiert eine Regulierung der IR-Absorption an der Oberfläche eines Fensters.

In einer weiteren spezifischen Ausführungsform des erfindungsgemäßen Verfahrens zur Regulierung der IR-Absorption befinden sich die Verbindungen der allgemeinen Formel (I) eingearbeitet in einem Fenstermaterial. Durch die Sonnenstrahlung erfolgt eine wie oben beschriebene Bestrahlung der Verbindungen der allgemeinen Formel (I) und es resultiert eine Regulierung der IR-Absorption des Fensters.

Weiterhin bevorzugt wird mit Hilfe des erfindungsgemäßen Verfahrens die Absorption elektromagnetischer Strahlung durch ein Material im Wellenlängenbereich des sichtbaren Lichts reguliert.

In einer spezifischen Ausführungsform des erfindungsgemäßen Verfahrens zur Regulierung der Absorption des sichtbaren Lichts befinden sich die Verbindungen der allgemeinen Formel (I) an der Oberfläche eines Fensters. Durch die Sonnenstrahlung erfolgt eine wie oben beschriebene Bestrahlung der Verbindungen der allgemeinen Formel (I) und es resultiert eine Regulierung der Absorption des sichtbaren Lichts an der Oberfläche eines Fensters. Insbesondere resultiert hieraus eine Tönung des Fensters.

In einer weiteren spezifischen Ausführungsform des erfindungsgemäßen Verfahrens zur Regulierung der Absorption des sichtbaren Lichts befinden sich die Verbindungen der allgemeinen Formel (I) eingearbeitet in einem Fenstermaterial. Durch die Sonnenstrahlung erfolgt eine wie oben beschriebene Bestrahlung der Verbindungen der allgemeinen Formel (I) und es resultiert eine Regulierung der Absorption des sichtbaren Lichts im Fenster. Insbesondere resultiert hieraus eine Tönung des Fensters.

Ein weiterer Gegenstand der Erfindung ist ein Verfahren zur Regulierung der Transmission elektromagnetischer Strahlung, bevorzugt im IR oder im Wellenlängenbereich des sichtbaren Lichts, durch ein Material, insbesondere durch ein Fenster, wobei dieses Material sich in Kontakt mit Verbindungen der allgemeinen Formel (I) befindet. Nach der oben beschriebenen Bestrahlung fungieren diese Verbindungen als Absorber, insbesondere für IR-Strahlung oder sichtbares Licht und verringern daher die Transmission der entsprechenden Strahlung durch das Material.

Insbesondere kann eine Regulierung der Transmission des Infrarotanteils der Sonnenstrahlung durch ein Fenster erfolgen und damit die Erwärmung eines hinter dem Fenster liegenden Raums durch die Sonnenstrahlung verzögert oder vermieden werden (Wärememanagement).

Weiterhin lassen sich die erfindungsgemäßen Verfahren oder die durch die Bestrahlung veränderte Verbindungen daher beim Wärmemanagement oder zur Wärmedämmung einsetzen.

Ein weiterer Gegenstand der Erfindung ist daher ein Verfahren zum Wärmemanagement oder zur Wärmedämmung, wobei die Materialien zunächst mit Verbindungen der allgemeinen Formeln (I) in Kontakt gebracht werden. Zur Änderung der IRAbsorption der Materialien und damit zum Wärmemanagement wird im Material mit dem oben beschriebenen erfindungsgemäßen Verfahren eine Zunahme der Absorption im IR bewirkt.

Bevorzugt befinden sich die Verbindungen der allgemeinen Formeln (I) in Kontakt mit Fenstern, insbesondere Fensterscheiben. Durch das erfindungsgemäße Verfahren wird die IR-Transmission durch das Fenster reguliert und somit ein Wärmemanagement des hinter dem Fenster liegenden Raums ermöglicht. Hierbei kann es sich natürlich neben Fenstern in Gebäuden natürlich auch um Fenster von Automobilen, Flugzeugen oder Maschinen mit Fahrerkanzeln handeln.

Weiterhin kann bevorzugt eine Regulierung der Transmission des sichtbaren Anteils der Sonnenstrahlung durch ein Fenster erfolgen und damit die Helligkeit eines hinter dem Fenster liegenden Raums bei Sonneneinstrahlung geregelt werden (Helligkeitsmanagement).

Weiterhin lassen sich die erfindungsgemäßen Verfahren daher beim Helligkeitsmanagement analog wie oben für das Wärmemanagement beschrieben einsetzen.

Ein weiterer Gegenstand der vorliegenden Erfindung ist die Ausnutzung des erfindungsgemäßen Verfahrens für die Markierung von Materialien. Bevorzugt wird das erfindungsgemäße Verfahren beim Nachweis der Markierung von Papier, Mineralöl, Kunststoffen oder Metalloberflächen eingesetzt. Besonders bevorzugt werden die Verbindungen der allgemeinen Formel (I) als Markierstoffe für Mineralöle oder Papier eingesetzt. Ganz bevorzugt ist die Verwendung zur Markierung von Papier, insbesondere als Sicherheitsmerkmal für Dokumente, Wertpapiere oder Geldscheine.

Ein Vorteil der Verwendung als Markierstoff für Mineralöle ist, dass Mineralöle häufig in dem spektralen Bereich der nach Bestrahlung der Verbindungen (I) auftretenden veränderten Absorption, selbst keine Absorption aufweisen und ein ausgezeichnetes Signal/Rauschverhältnis für den Nachweis auch geringster Mengen des Markierstoffes erreicht wird. Dies gilt insbesondere für eine mit Hilfe des erfindungsgemäßen Verfahrens erzeugte Änderung der Absorption im IR.

Häufig absorbieren die Verbindungen der allgemeinen Formeln (I) selbst sichtbares Licht und können daher auch zur Anfärbung von Materialien, insbesondere von Papier und Kunststoffen eingesetzt werden. Diese Anfärbung kann durch das erfindungsgemäße Verfahren reduziert oder entfernt werden, unter der Bedingung, dass das Absorptionsspektrum der durch Bestrahlung veränderten Verbindung eine bathochrome Verschiebung in den IR Bereich erfahren hat.

Ein weiterer Gegenstand der Erfindung ist ein Verfahren zur Markierung von Materialien, wobei die Materialien mit Verbindungen der allgemeinen Formeln (I) in Kontakt gebracht werden. Zum Nachweis der Markierung werden im Material mit den oben beschriebenen erfindungsgemäßen Verfahren Veränderungen der Absorption erzeugt und detektiert. Die Detektion erfolgt in der Regel visuell (mit bloßem Auge) oder mit Hilfe eines (Absorptions)spektrometers.

Die erfindungsgemäße Markierung von Papier erfolgt beispielsweise durch das Aufbringen von Verbindungen der allgemeinen Formeln (I) auf die Oberfläche des zu markierenden Papiers, beispielsweise durch Besprühen, Tränken oder Auftropfen mit Lösungen enthaltend die Verbindungen der Formeln (I). Insbesondere lassen sich die Verbindungen der allgemeinen Formeln (I) in einer Mischung mit einer Druckfarbe auf das Papier auftragen. Die Menge an Verbindungen der allgemeinen Formeln (I) kann je nach Anwendung in einem breiten Bereich variieren. Bevorzugt werden weniger als 10 Gew.-% Verbindungen der allgemeinen Formeln (I) bezogen auf die Menge an Druckfarbe eingesetzt, ganz bevorzugt weniger als 5 Gew.-%. Der Nachweis der Markierung kann beispielsweise durch einen Farbwechsel visuell oder mit Hilfe eines (Absorptions)spektrometers vorgenommen werden.

Die erfindungsgemäße Markierung von Mineralöl erfolgt durch Zugabe von Verbindungen der allgemeinen Formeln (I) zum zu markierenden Mineralöl. Die Menge an Verbindungen der allgemeinen Formeln (I) kann je nach Anwendung in einem breiten Bereich variieren. Bevorzugt werden weniger als 5 ppm Verbindungen der allgemeinen Formeln (I) bezogen auf die Menge an Mineralöl eingesetzt, ganz bevorzugt weniger als 1 ppm. Der Nachweis der Markierung kann beispielsweise mit Hilfe eines (Absorptions)spektrometers vorgenommen werden.

Weiterhin lassen sich die erfindungsgemäßen Verfahren beim Laserschweißen einsetzen.

Ein weiterer Gegenstand der Erfindung ist daher ein Verfahren zum Laserschweißen von Materialien, wobei die Materialien zunächst mit Verbindungen der allgemeinen Formeln (I) in Kontakt gebracht werden. Zum Verschweißen wird im Material mit den oben beschriebenen erfindungsgemäßen Verfahren eine Zunahme in der IR-Absorption bewirkt.

Das Schweißen von Materialien, insbesondere von Kunststoffen erfolgt durch Absorption von Laserenergie im oder auf dem Kunststoffmaterial durch die zugesetzten lasersensitiven IR-Absorber, die durch Absorption der Laserenergie zu einer lokalen Erhitzung des Materials führen. Beim Laserscheißen von beispielsweise zwei Materialien wird im Fügebereich der zu verschweißenden Materialien durch Absorption der Laserenergie eine starke Erwärmung erzeugt, so dass die Materialien aufschmelzen und beide Materialien miteinander verschmelzen. Häufig ist es ausreichend, wenn nur eines der Materialien lasersensitive IR-Absorber im Material oder als Schicht an der Oberfläche enthält. Die Laserschweißbarkeit ist Abhängig von der Natur der Materialien, insbesondere Kunststoffe sowie von der Wellenlänge und der Strahlungsleistung des eingesetzten Lasers. Beispielsweise kommen für das erfindungsgemäße Verfahren zum Laserschweißen CO₂-, Excimer-, oder ND:YAG-Laser in Frage.

In der Regel beträgt der Gehalt an Verbindungen der allgemeinen Formeln (I) insgesamt zwischen von 0,0001 bis 1 Gew.-% bezogen auf das zu verschweißende Material. Bevorzugt beträgt der Gehalt von 0,001 bis 0,1 Gew.%. Insbesondere ergibt sich in diesem Bereich von 0,001 bis 0,1 Gew.-% eine ausreichende Verschweißbarkeit von Kunststoffen.

Die Verbindungen der allgemeinen Formeln (I) können mit Hilfe von dem Fachmann bekannten Verfahren, wie beispielsweise durch Extrusion, in praktisch alle Kunststoffe eingearbeitet werden, insbesondere um diesen Laserschweißbarkeit zu verleihen. Typisch sind Kunststoffmaterialien bei denen die Kunststoffmatrix auf Poly(meth)acrylat, Polyamid, Polyurethan, Polyolefinen, Styrolpolymeren und Styrolcopolymeren, Polycarbonat, Silikonen, Polyimiden, Polysulfon, Polyethersulfon, Polyketone, Polyetherketone, PEEK, Polyphenylensulfid, Polyester (wie PET, PEN, PBT), Polyethylenoxid, Polyurethan, Polyolefinen, Cycloolefincopolymeren oder fluorhaltigen Polymeren (wie PVDF, EFEP, PTFE) basiert. Ebenfalls ist eine Einarbeitung in Blends möglich, die als Komponenten oben genannte Kunststoffe beinhalten, oder in von diesen Klassen abgeleitete Polymere, die durch nachträgliche Reaktionen verändert wurden. Diese Materialien sind in großer Vielfalt bekannt und kommerziell erhältlich.

Ein weiterer Gegenstand der Erfindung sind neue spezielle Verbindungen der allgemeinen Formel (I) wie eingangs beschrieben, wobei
- R¹, R²: unabhängig voneinander, gleich oder verschieden, C₁-C₈-Alkylamino, C₁-C₈-Dialkylamino, Arylamino, Diarylamino, Triarylaminyl, Carbazolyl,
- R³, R⁴: unabhängig voneinander, gleich oder verschieden, C₁-C₈-Alkylen, Arylen,
- X: unabhängig voneinander, gleich oder verschieden, Halogen, Cyan, oder eine Gruppe 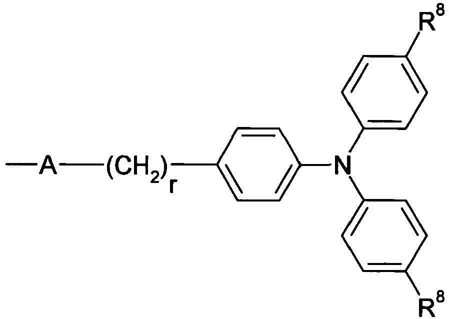 mit
A S, SO₂,
r 2, 3, 4, 5, 6,
R⁸ C₁-C₂₀-Alkyl, C₁-C₂₀-Alkoxy,
- n, p: 0, 1, oder 2,
- m: 0, oder 1,
und wobei die Substituenten R¹, R² jeweils an beliebiger Position, allerdings nicht mehr als fünfmal durch C₁-C₂₀-Alkyl, C₁-C₂₀-Alkoxy substituiert sein können und die aus diesen Verbindungen durch Bestrahlung mit Hilfe von elektromagnetischer Strahlung der Wellenlänge von 300 nm bis 750 nm erhaltenen durch Bestrahlung veränderten Verbindungen.

Bevorzugt sind spezielle Verbindungen der allgemeinen Formel (I) wie eingangs beschrieben, wobei
- R¹, R²: unabhängig voneinander, gleich oder verschieden, *-N(CH₃)₂, 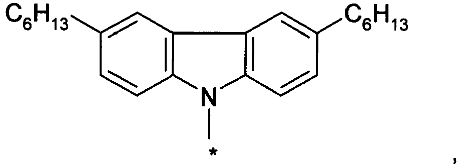 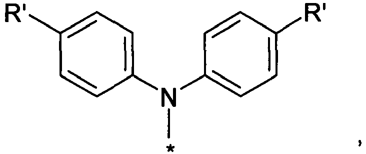
- R³, R⁴: unabhängig voneinander, gleich oder verschieden, *-CH₂-CH₂-*, *-CH₂-CH₂-CH₂-*, 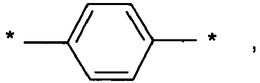
- X: unabhängig voneinander, gleich oder verschieden, Halogen, Cyan, oder eine Gruppe 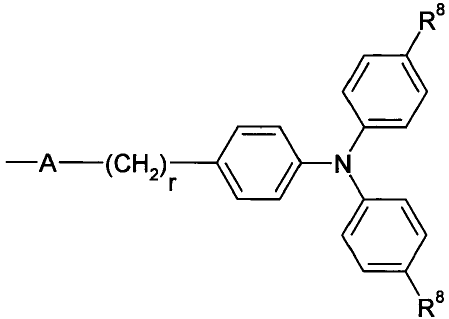 mit
A S, SO₂,
r 2, 3, 4, 5, 6,
R⁸ C₁-C₂₀-Alkyl, C₁-C₂₀-Alkoxy,
- n, p: 0, 1, oder 2,
- m: 0, oder 1,
- R': H, C₆-C₈-Alkyl, C₆-C₈-Alkoxy,
und wobei "*" die Anbindungsstelle der chemischen Bindung bezeichnet, und die aus diesen Verbindungen durch Bestrahlung mit Hilfe von elektromagnetischer Strahlung der Wellenlänge von 300 nm bis 750 nm erhaltenen durch Bestrahlung veränderten Verbindungen. Bevorzugt ist R' gegeben durch H oder

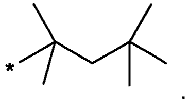

Ganz bevorzugt sind die Substituenten R¹ = R² und R³ = R⁴ und alle Substituenten X untereinander gleich.

Ganz besonders bevorzugte neue und erfinderische spezielle Verbindungen der allgemeinen Formel (I) wie eingangs beschrieben, sind gemeinsam mit neuen und erfinderischen Zwischenstufen zu ihrer Herstellung in den Beispielen angegeben.

Zwischenprodukte der allgemeinen Formeln (III), (IV) und (V), die bei der Herstellung der Verbindungen der allgemeinen Formeln (I) Verwendung finden können, sind:
mit 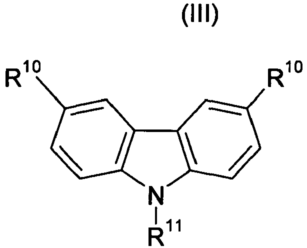
- R¹⁰: C₁-C₂₀-Alkyl, C₁-C₂₀-Alkoxy,
- R¹¹: -(CH₂)ᵣ-OH, 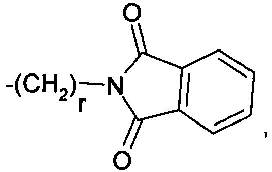 -(CH₂)ᵣ-NH₂,
-(CH₂)ᵣ-COOR²²,
-(CH₂)ᵣ-NH3⁺ Y⁻,
-(CH₂)ᵣ-SH,
mit r = 2, 3, 4, 5 oder 6 und Y- = einfach negativ geladenes Anionenequivalent als Gegenion, beispielsweise Cl⁻, Br, ½ SO₄²⁻, bevorzugt Halogenid. R²² C₁-C₂₀-Alkyl, bevorzugt Methyl oder Ethyl. 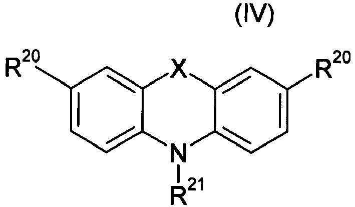 mit
- R²⁰: C₁-C₂₀-Alkyl, C₁-C₂₀-Alkoxy,
- R²¹: H, -(CH₂)ᵣ-OH, 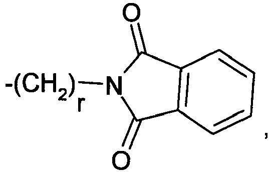 -(CH₂)ᵣ-NH2,
-(CH₂)ᵣ-NH₃⁺ Y⁻,
-(CH₂)ᵣ-SH,
mit r = 2, 3, 4, 5 oder 6 und X⁻ = einfach negativ geladenes Anionenequivalent als Gegenion, beispielsweise Cl⁻, Br, ½ SO₄²⁻, bevorzugt Halogenid,
- X: 2H, S. 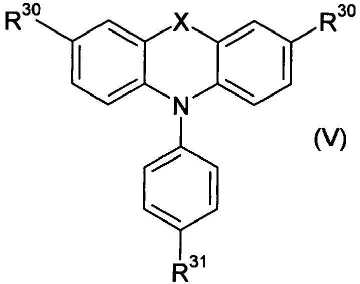 mit
R³⁰ C₁-C₂₀-Alkyl, C₁-C₂₀-Alkoxy,
R³¹ H, CHO, CH₂CN, CH=CH-CN, CH=CH-COOR³², (CH₂)ᵣ-NH2, (CH₂)ᵣ-COOR³², NH₂, NO₂, Halogen, (CH₂)ᵣ-SH, (CH₂)ᵣ-NHCO-t-Bu mit r = 2, 3, 4, 5 oder 6, X Einfachbindung, 2H, S,
R³² C₁-C₂₀-Alkyl,
unter der Maßgabe, dass R³⁰ nicht C₁-C₄-Alkyl oder C₁-C₄-Alkoxy und gleichzeitig, R³¹ nicht H oder CHO und gleichzeitig X nicht 2H.

Wobei 2H zwei einzelne Wasserstoffatome bedeuten, die jeweils einzeln an die beiden Ringe in Formel (IV) oder (V) angebunden sind.

Ein weiterer Gegenstand der Erfindung sind Materialien, beispielsweise Glas, Kunststoff, Textilien oder Metalle, die mit den oben beschrieben neuen speziellen Verbindungen der allgemeinen Formel (I) in Kontakt sind, insbesondere diese enthalten.

Für das in Kontakt bringen, insbesondere die Einarbeitung von Verbindungen der allgemeinen Formeln (I) können die dem Fachmann für das jeweilige Material bekannten Verfahren zum In-Kontakt-bringen von Materialien mit anderen Additiven eingesetzt werden. Bei Papier handelt es sich hierbei beispielsweise um die Massefärbung mit Farbstoffen oder das Aufbringen von Druckfarben. Bei Kunststoffen können die Verfahren zur Beschichtung von Kunststoffen oder die Verfahren zur Massefärbung von Kunststoffen, beispielsweise mit Hilfe eines Extruders verwendet werden. Auch die Techniken des Spin-Coatings oder des Aufdampfens von Stoffen auf Materialien sind hierbei einsetzbar.

Weiterhin ist es möglich die Verbindungen der allgemeinen Formeln (I) zunächst in einem Lösungsmittel zu lösen, die Materialien mit der Lösung in Kontakt zu bringen und das Lösungsmittel danach zu entfernen, beispielsweise durch einen Trockenprozeß.

Ein weitere Gegenstand der Erfindung sind daher Polymere, Lacke oder Gläser enthaltend Verbindungen der allgemeinen Formel (I).

Die vorliegende Erfindung erlaubt die Verwendung von Verbindungen in Anwendungen in denen eine gewünschte Absorption im IR oder im sichtbaren Bereich des Spektrums nicht permanent sondern schaltbar sein soll. Weiterhin stellt die Erfindung Verfahren zur zuverlässigen Markierung von Materialien zur Verfügung. Ebenso stellt die Erfindung einfache Verfahren zur Verfügung, die es ermöglichen die Absorption elektromagnetischer Strahlung durch Materialien zu regeln.

Die folgenden Beispiele sollen die Erfindung verdeutlichen, ohne sie jedoch einzuschränken.

### Beispiele:

### Beispiel 1: Herstellung von N,N'-Bis[2-(3,6-dihexyl-N-carbazolyl)-ethyl]-perylentetracarbonsäurediimid

### a) N-Methoxycarbonylmethyl-3,6-di-n-hexyl-carbazol

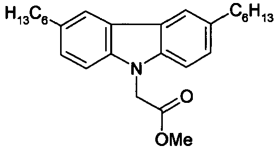

α) Eine Lösung von 10,07 g (30,0 mmol) 3,6-Di-n-hexylcarbazol (Herstellung nach J. J. Piet et al., Chem. Phys. Lett. 289 (1998) 13 - 18) in 50 ml wasserfreiem Dimethylformamid wurde unter Stickstoff mit 11,2 g (81,0 mmol) Kaliumcarbonat versetzt und auf 50 °C erhitzt. Nach 45 min Rühren wurden 14,19 g (90,0 mmol) 97 %iges Methylbromacetat innerhalb von 1 Stunde zugetropft. Anschließend wurde das Reaktionsgemisch 24 Stunden lang bei 50 °C gerührt. Das Reaktionsgemisch wurde auf 300 ml Wasser gegossen und mit 300 ml Xylol extrahiert. Die wässrige Phase wurde abgetrennt und dreimal mit jeweils 200 ml Xylol ausgeschüttelt. Die vereinigten organischen Phasen wurden zweimal mit jeweils 200 ml Wasser und einmal mit gesättiger Natriumchlorid-Lösung gewaschen, mit Natriumsulfat getrocknet und dann zur Trockne eingeengt. Es wurden 13,72 g Rohprodukt erhalten, das an Kieselgel (60 Å, 60 - 200 µm) mit Toluol/n-Heptan (2:1) als Laufmittel gereinigt wurde. Es wurden 8,03 g (66 % d. Th.) hellgelbes Öl erhalten.

Analysen ¹H-NMR:
¹H-NMR (CDCl₃, 400 MHz): δ = 7,88 (s; 2H, arom. H), 7,27 (d; 2H, arom. H), 7,20 (d; 2H, arom. H), 4,96 (s; 2H, N-CH₂-), 3,69 (s; 3H, O-CH₃), 2,77 (t; 4H, Ph-CH₂-), 1,69 (mc; 4H, -CH₂-), 1,25 - 1,42 (m; 12H, -CH₂-), 0,88 ppm (t; 6H, -CH₃)

β) Eine Lösung von 3,00 g (8,94 mmol) 3,6-Di-n-hexylcarbazol und 1,55 g (9,84 mmol) 97 %iges Methylbromacetat in 40 ml Methylisobutylketon wurden mit 7,15 g 50 % NaOH und 0,14 g Aliquat 175 bei Raumtemperatur 30 min lang gerührt. Anschließend wurde die Reaktionslösung mit 70 ml Wasser versetzt. Die wässrige Phase wurde abgetrennt, mit 30 ml Methylisobutylketon und dann mit 20 ml Essigester ausgeschüttelt. Die vereinigten organischen Phasen wurden zweimal mit jeweils 50 ml gesättigter Natriumchlorid-Lösung gewaschen, über Magnesiumsulfat getrocknet und bis zur Trockne eingeengt. Es wurden 3,6 g (94 % d. Th.) gelbliches Öl erhalten.
Die Verbindung ist laut ¹H-NMR identisch mit Beispiel 1aα).

### b) N-(2-Hydroxyethyl)-3,6-di-n-hexyl-carbazol

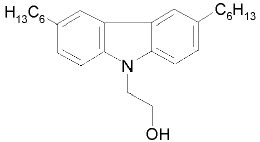

Zu einer auf 0 °C gekühlten Suspension von 0,88 g (22,4 mmol) 97 %igem Lithiumaluminiumhydrid in 30 ml Tetrahydrofuran (THF) wurde unter Argon eine Lösung von 8,00 g (19,6 mmol) N-Methoxycarbonylmethyl-3,6-di-n-hexyl-carbazol in 30 ml THF innerhalb von 15 min getropft, wobei die Temperatur bei 0 - 5 °C gehalten wurde. Man ließ die Reaktionsmischung auf Raumtemperatur erwärmen und rührte 30 min nach. Nach der tropfenweisen Zugabe von 20 ml Wasser/THF wurde die Lösung 15 min bei Raumtemperatur gerührt und dann mit 2 ml 10 % NaOH versetzt, wobei ein Niederschlag ausfiel, der abfiltriert wurde. Das Filtrat wurde mit 200 ml Diethylether/ Wasser (1:1) versetzt. Die wässrige Phase wurde abgetrennt und zweimal mit jeweils 100 ml Diethylether ausgeschüttelt. Die vereinigten organischen Phasen wurden zweimal mit jeweils 100 ml Wasser neutral und mit 100 ml gesättigter Natriumchlorid-Lösung gewaschen, über Natriumsulfat getrocknet und zur Trockne eingeengt. Es wurden 7,5 g (100 % d. Th.) farbloser Feststoff erhalten.

Analyse ¹H-NMR:
¹H-NMR (CDCl₃, 400 MHz): δ = 7,87 (s; 2H, arom. H), 7,33 (d; 2H, arom. H), 7,25 (d; 2H, arom. H), 4,41 (t; 2H, N-CH₂-CH₂-), 4,00 (t; 2H, N-CH₂-CH₂-), 2,77 (t; 4H, Ph-CH₂-), 1,70 (mc; 4H, -CH₂-), 1,23 - 1,42 (m; 12H, -CH₂-), 0,83 ppm (t; 6H, -CH₃)

### c) N-[2-(N-Phthalimido)-ethyl]-3,6-di-n-hexyl-carbazol

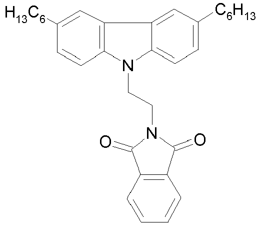

Zu einer Lösung von 7,50 g (19,8 mmol) N-(2-Hydroxyethyl)-3,6-di-n-hexyl-carbazol, 3,14 g (21,3 mmol) Phthalimid und 5,29 g (20,0 mmol) 99 %iges Triphenylphosphin in 200 ml THF wurden unter Stickstoff 3,94 g (22,1 mmol) 98 %iges Diethylazodicarboxylat (DEAD) bei Raumtemperatur getropft (exotherm). Nach 27 Stunden Rühren bei Raumtemperatur wurde das Reaktionsgemisch bis zur Trockne eingeengt und dann mit eiskaltem Methanol versetzt. Der Feststoff wurde abgesaugt. Es wurden 8,93 g (89 % d. Th.) gelber Feststoff erhalten, der bei 118 - 119 °C schmolz.

Analyse ¹H-NMR:
¹H-NMR (CDCl₃, 400 MHz): δ = 7,84 (s; 2H, arom. H), 7,79 (mc; 2H, arom. H), 7,69 (mc; 2H, arom. H), 7,39 (d; 2H, arom. H), 7,23 (d; 2H, arom. H), 4,52 (t; 2H, N-CH₂-), 4,08 (t; 2H, N-CH₂-), 2,75 (t; 4H, Ph-CH₂-), 1,67 (mc; 4H, -CH₂-), 1,23 - 1,42 (m; 12H, - CH₂-), 0,88 ppm (t; 6H, -CH₃)

### d) N-(2-Aminoethyl)-3,6-di-n-hexyl-carbazol

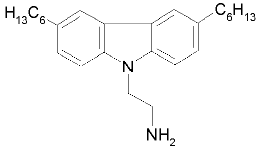

Eine Lösung von 8,90 g (17,5 mmol) N-[2-(N-Phthalimido)-ethyl]-3,6-di-n-hexylcarbazol in 150 ml THF und 150 ml Ethanol wurde mit 1,79 g (35,0 mmol) 98 %iges Hydrazin-Monohydrat versetzt und dann 20 Stunden lang zum Sieden unter Rückfluss erhitzt. Nach der Zugabe von 80 ml Wasser wurde das Reaktionsgemisch eingeengt, dann mit jeweils 160 ml Wasser und Diethylether versetzt und mit 20 % NaOH auf pH 13,6 gestellt. Die wässrige Phase wurde abgetrennt und zweimal mit jeweils 150 ml Diethylether extrahiert. Die vereinigten organischen Phasen wurden zweimal mit jeweils 150 ml Wasser gewaschen, über Natriumsulfat getrocknet. Nach dem Einengen des Filtrats wurden 5,25 g (79 % d. Th.) gelbbraunes Öl isoliert, das nach einiger Zeit kristallisierte und bei 56 °C schmolz.

Analyse ¹H-NMR:
¹H-NMR (CDCl₃, 400 MHz): δ = 7,88 (s; 2H, arom. H), 7,33 (d; 2H, arom. H), 7,26 (d; 2H, arom. H), 4,34 (t; 2H, N-CH₂-), 3,19 (t; 2H, -CH₂-NH₂), 2,77 (t; 4H, Ph-CH₂-), 1,70 (mc; 4H, -CH₂-), 1,23 - 1,43 (m; 12H, -CH₂-), 0,88 ppm (t; 6H, -CH₃)

### e) N,N'-Bis[2-(3,6-dihexyl-N-carbazolyl)-ethyl]-perylentetracarbonsäurediimid

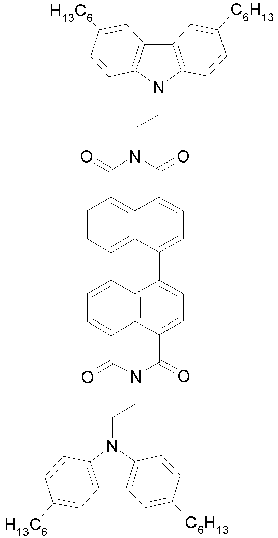

2,00 g (5,10 mmol) Perylentetracarbonsäuredianhydrid und 4,05 g (10,7 mmol) N-(2-Aminoethyl)-3,6-di-n-hexyl-carbazol wurden in 100 ml N-Methylpyrrolidinon (NMP) 6 Stunden lang bei 120 °C gerührt. Nach dem Abkühlen auf Raumtemperatur wurden 100 ml Methanol zugegeben. Der ausgefällte Feststoff wurde abgesaugt und getrocknet (5,53 g). Das Rohprodukt wurde in 270 ml Chlorbenzol umkristallisiert. Der Feststoff wurde abgesaugt, mit Chlorbenzol und Pentan gewaschen und getrocknet. Es wurden 4,65 g (82 % d. Th.) rote Mikrokristalle mit einem Schmp. von 314 - 315 °C erhalten.
UV/Vis (Chloroform): λₘₐₓ (ε) = 526 nm (84730)

| | |
|---|---|
| C₇₆H₈₀N₄O₄ (M = 1113,49 g/mol): | Ber. C 81,98 H 7,24 N 5,03 O 5,75 |
| | Gef. C 81,8 H 7,2 N 4,9 O 5,7 |

### Beispiel 2: Herstellung von N,N'-Bis[3-(3,6-di-n-hexyl-N-carbazolyl)-propyl]-perylentetracarbonsäurediimid

### a) N-[3-(N-Phthalimido)-propyl]-3,6-di-n-hexyl-carbazol

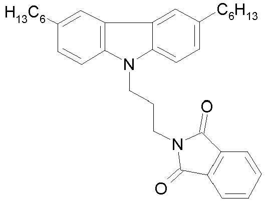

Zu einer Suspension von 1,28 g (32,0 mmol) 60 %iges Natriumhydrid in Mineralöl in 25 ml wasserfreiem THF wurde eine Lösung von 6,71 g (20,0 mmol) 3,6-Di-n-hexylcarbazol in 25 ml wasserfreiem THF innerhalb von 15 min getropft. Nach dem Zutropfen einer Lösung von 8,76 g (32,0 mmol) 98 %iges N-(3-Brompropyl)-phthalimid in 25 ml wasserfreiem THF innerhalb von 15 min wurde die Reaktionsmischung 21 Stunden lang zum Sieden unter Rückfluss erhitzt. Nach dem Abkühlen auf Raumtemperatur wurden 10 ml THF/Wasser (1 : 1) zugetropft, um überschüssiges Natriumhydrid zu vernichten. Die Reaktionsmischung wurde mit 300 ml Wasser und mit 50 ml gesättiger Kochsalzlösung versetzt und mit 300 ml Xylol extrahiert. Die wässrige Phase wurde abgetrennt und zweimal mit jeweils 200 ml Xylol ausgeschüttelt. Die vereinigten organischen Phasen wurden zweimal mit jeweils 200 ml Wasser und einmal mit 200 ml gesättiger Kochsalzlösung gewaschen, über Natriumsulfat getrocknet und nach Filtration bis zur Trockne eingeengt. Das Rohprodukt (12,46 g gelbes Öl) wurde an Kieselgel (60 Ä, 60 - 200 µm) mit n-Heptan /Essigester (10 : 1) als Laufmittel gereinigt. Es wurden 7,52 g (72 % d. Th.) dunkelgelbes Öl erhalten.

### b) N-(3-Aminopropyl)-3,6-di-n-hexyl-carbazol

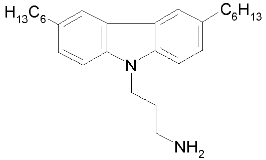

Eine Lösung von 6,91 g (13,2 mmol) N-[3-(N-Phthalimido)-propyl]-3,6-di-n-hexylcarbazol in 100 ml THF und 100 ml Ethanol wurde mit 1,35 g (26,4 mmol) 98 %iges Hydrazin-Monohydrat versetzt und dann 20,5 Stunden lang zum Sieden unter Rückfluss erhitzt. Nach der Zugabe von 120 ml Wasser wurde das Reaktionsgemisch eingeengt, dann mit 120 ml Wasser und 100 ml Diethylether versetzt und mit 20 % NaOH auf pH 13,5 gestellt. Die wässrige Phase wurde abgetrennt und zweimal mit jeweils 120 ml Diethylether extrahiert. Die vereinigten organischen Phasen wurden zweimal mit jeweils 100 ml Wasser gewaschen, über Natriumsulfat getrocknet. Nach dem Einengen des Filtrats wurden 4,24 g (82 % d. Th.) hellgelbes Öl isolert, das nach einiger Zeit kristallisierte.

Analyse ¹H-NMR:
¹H-NMR (CDCl₃, 400 MHz): δ = 7,85 (s; 2H, arom. H), 7,18 - 7,32 (m; 4H, arom. H), 4,29 (t; 2H, N-CH₂-), 2,77 (t; 4H, Ph-CH₂-), 2,67 (t; 2H, -CH₂NH₂), 1,95 (q; 2H, -CH₂-CH₂-NH₂), 1,70 (mc; 4H, -CH₂-), 1,23 - 1,43 (m; 12H, -CH₂-), 0,88 ppm (t; 6H, -CH₃)

### c) N,N'-Bis[3-(3,6-di-n-hexyl-N-carbazolyl)-propyl]-perylentetracarbonsäurediimid

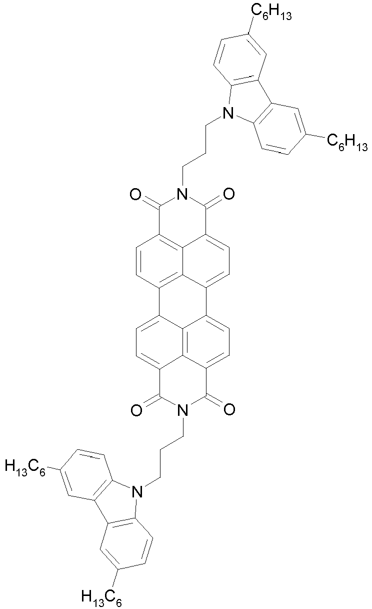

4,20 g (10,7 mmol) N-(3-Aminopropyl)-3,6-di-n-hexyl-carbazol und 2,00 g (5,10 mmol) Perylentetracarbonsäuredianhydrid wurden in 50 ml N-Methylpyrrolidinon (NMP) unter Rühren auf 120 °C erhitzt und 8,5 Stunden bei dieser Temperatur gehalten. Nach Abkühlung der Reaktionsmischung auf Raumtemperatur wurden 150 ml Methanol zugegeben, wobei ein Niederschlag ausfiel. Dieser wurde abgesaugt, zweimal mit Methanol gewaschen und getrocknet. 5,64 g Rohprodukt wurden über Blitz-Chromatographie an Kieselgel (60 Å, 60 - 200 µm) mit Methylenchlorid / n-Heptan / Aceton (10 : 3 : 1) als Laufmittel gereinigt. Es wurden 4,50 g (78 % d. Th.) dunkelroter Feststoff mit einem Schmp. von 218 - 220 °C erhalten.
UV/Vis (Methylenchlorid): λₘₐₓ (ε) = 524 nm (73040)

| | |
|---|---|
| C₇₈H₈₄N₄O₄ (M = 1141,56 g/mol): | Ber. C 82,07 H 7,42 N 4,91 O 5,61 |
| | Gef. C 82,0 H 7,4 N 4,8 O 5,8 |

### Beispiel 3: Herstellung von N,N'-Bis(3-dimethylaminopropyl)-1,6,7,12-tetrachlorperylentetracarbonsäurediimid

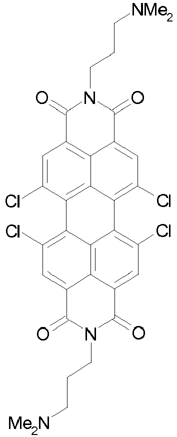

5,30 g (10,0 mmol) 1,6,7,12-Tetrachloperylentetracarbonsäuredianhydrid und 2,45 g (24,0 mmo) N,N-Dimethyl-1,3-propylendiamin wurden in 100 ml NMP unter Rühren und Stickstoff auf 80 °C erhitzt und 4 Stunden bei dieser Temperatur gehalten. Nach dem Abkühlen der Reaktionsmischung auf Raumtemperatur wurde diese filtriert. Der Rückstand wurde mit Methanol gewaschen und getrocknet. Das Rohprodukt (3,21 g) wurde zweimal aus o-Dichlorbenzol umkristallisiert. Es wurden 1,09 g (16 % d. Th.) orangefarbener Feststoff erhalten, der sich ab 246 °C zersetzte.
UV/Vis (Methylenchlorid): λₘₐₓ (ε) = 518 nm (39990)

| | |
|---|---|
| C₃₄H₂₈Cl₄N₄O₄ (M = 698,44 g/mol): | Ber. C 58,47 H 4,04 Cl 20,30 N 8,02 O 9,16 |
| | Gef. C 58,1 H 4,1 Cl 20,7 N 8,0 |

### Beispiel 4: Herstellung von N,N'-Bis[2-(3,6-dihexyl-N-carbazolyl)-ethyl]-1,6,7,12-tetrachlor-perylentetracarbonsäurediimid

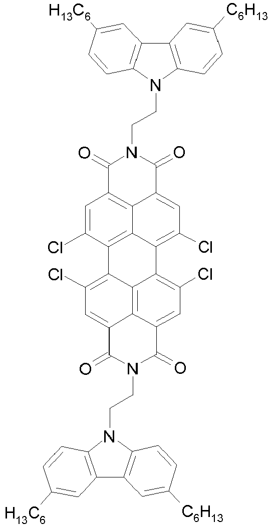

Die Herstellung erfolgte entsprechend der in Beispiel 1 mit 2,00 g (3,77 mmol) 1,6,7,12-Tetrachlorperylentetracarbonsäuredianhydrid. Es wurden 1,37 g (29 % d. Th.) dunkelbraunroter Feststoff mit einem Schmp. von 277 °C erhalten.
UV/Vis (Methylenchlorid): λₘₐₓ (ε) = 520 nm (39620)

| | |
|---|---|
| C₇₆H₇₆Cl₄N₄O₄ (M = 1251,29 g/mol): | Ber. C 72,95 H 6,12 Cl 11,33 N 4,48 O 5,11 |
| | Gef. C 73,3 H 6,3 Cl 11,4 N 3,8 O 4,9 |

### Beispiel 5: Herstellung von N,N'-Bis[3-(3,6-di-n-hexyl-N-carbazolyl)-propyl]-1,6,7,12-tetrachlor-perylentetracarbonsäurediimid

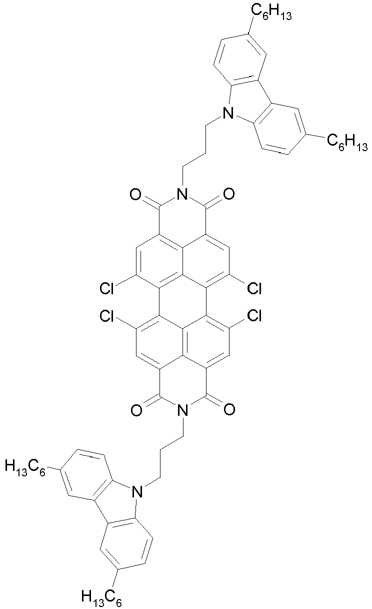

Die Herstellung erfolgte entsprechend der in Beispiel 2 mit 2,50 g (4,72 mmol) 1,6,7,12-Tetrachlorperylentetracarbonsäuredianhydrid. Es wurden 2,54 g (56 % d. Th.) rotbrauner Feststoff erhalten, der bei 107 °C zusammensinterte.
UV/Vis (Methylenchlorid): λₘₐₓ (ε) = 518 nm (37610)

| | |
|---|---|
| C₇₈H₈₀Cl₄N₄O₄ (M = 1279,34 g/mol): | Ber. C 73,23 H 6,30 Cl 11,08 N 4,38 O 5,00 |
| | Gef. C 72,9 H 6,5 Cl 11,1 N 4,3 O 5,2 |

### Beispiel 6: Herstellung von N,N'-Bis[4-(N,N-diphenylamino)-phenyl]-1,6,7,12-tetrachlorperylentetracarbonsäurediimid

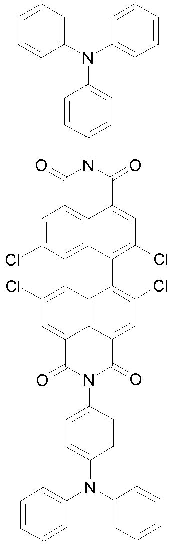

3,18 g (6,00 mmol) 1,6,7,12-Tetrachlorperylentetracarbonsäuredianhydrid, 3,90 g (15,0 mmol) N,N-Diphenyl-p-phenylendiamin und 0,15 g (0,075 mmol) Zinkacetat wurden in 60 ml Chlorbenzol gegeben und 8 Stunden lang zum Sieden unter Rückfluss erhitzt. Nach dem Abkühlen auf Raumtemperatur wurde der Niederschlag abgesaugt, mit Methanol gewaschen und getrocknet (3,50 g). Das Rohprodukt wurde in einer größeren Menge Chlorbenzol in der Hitze gelöst. Die Lösung wurde heiß filtriert. Das Filtrat wurde eingeengt und bei Raumtemperatur mit Diethylether überschichtet. Nach dreitägigem Stehen im Kühlschrank wurde der auskristallisierte Feststoff abgesaugt, gewaschen und getrocknet. Es wurden 0,55 g (9 % d. Th.) rotbrauner Feststoff mit einem Schmp. von > 410 °C erhalten.
UV/Vis (Methylenchlorid): λₘₐₓ = 518 nm
MALDI-TOF-MS: m/e = 1012,3 (ber. 1012,1)

### Beispiel 7: Herstellung von N,N'-Bis{4-[N,N-bis-(1,1,3,3-tetramethyl-butyl-4-phenyl)-amino]-phenyl]-1,6,7,12-tetrachlor-perylentetracarbonsäurediimid

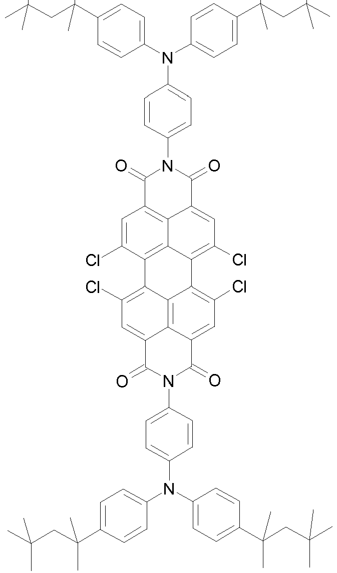

3,18 g (6,00 mmol) 1,6,7,12-Tetrachlorperylentetracarbonsäuredianhydrid, 7,27 g (15,0 mmol) N,N-Bis[4-(1,1,3,3-tetramethyl-butyl)-phenyl]-p-phenylendiamin und 0,15 g (0,075 mmol) Zinkacetat wurden in 60 ml Chlorbenzol gegeben und 5 Stunden lang zum Sieden unter Rückfluss erhitzt. Nach dem Abkühlen auf Raumtemperatur wurde der Niederschlag abgesaugt, mit Methanol gewaschen und getrocknet (8,05 g). Das Rohprodukt wurde an Kieselgel (60 Å, 60 - 200 µm) mit Toluol / n-Heptan (3,5 : 1 bis 2 : 1) chromatographisch gereinigt. Es wurden 2,05 g (54 % d. Th.) rosafarbener Feststoff mit einem Schmp. von 374 - 375 °C erhalten.
UV/Vis (Methylenchlorid): λₘₐₓ (ε) = 520 nm (48910)

| | |
|---|---|
| C₉₂H₉₆Cl₄N₄O₄ (M = 1463,63 g/mol): | Ber. C 75,45 H 6,47 Cl 9,79 N 3,87 O 4,42 |
| | Gef. C 75,7 H 6,8 Cl 9,3 N 3,8 |

### Beispiel 8: Herstellung von N,N'-Bis[3-(3,6-di-n-hexyl-N-carbazolyl)-propyl]-1,7-dibromperylentetracarbonsäurediimid

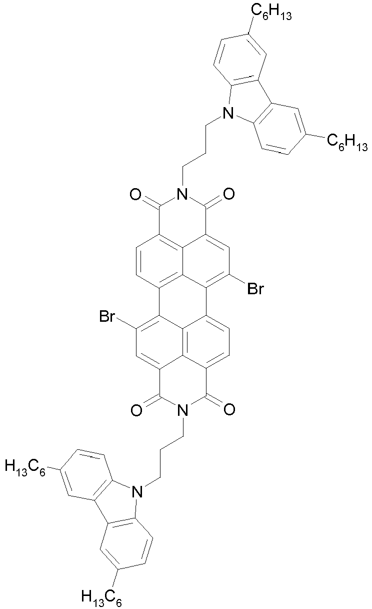

6,00 g (10,9 mmol) Dibromperylentetracarbonsäuredianhydrid und 9,00 g (22,9 mmol) N-(3-Aminopropyl)-3,6-dihexylcarbazol wurden in 100 ml Chlorbenzol gelöst. Die Lösung wurde auf 80 °C erhitzt und 5,5 Stunden bei dieser Temperatur gehalten. Nach dem Abkühlen auf Raumtemperatur wurde die Lösung bis zur Trockne eingeengt und dann mit 70 ml Methanol versetzt. Nach dreistündigem Rühren wurde der Feststoff abgesaugt, mit Methanol gewaschen und getrocknet (13,0 g). Das Rohprodukt wurde an Kieselgel (60 Å, 60 - 200 µm) mit Methylenchlorid als Laufmittel gereinigt. Es wurden 9,2 g (65 % d. Th.) roter Feststoff erhalten. Laut 1 H-NMR enthielt die Substanz zu 18 % das isomere N,N'-Bis[3-(3,6-di-n-hexyl-N-carbazolyl)-propyl]-1,6-dibromperylentetracarbonsäurediimid.
UV/Vis (Methylenchlorid): λₘₐₓ (ε) = 526 nm (48440)

| | |
|---|---|
| C₇₈H₈₂Br₂N₄O₄ (M = 1299,36 g/mol): | Ber. C 72,10 H 6,36 Br 12,30 N 4,31 O 4,93 |
| | Gef. C 72,0 H 6,5 Br 12,4 N 4,3 O 5,0 |

### Beispiel 9: Herstellung von N,N'-Bis{4-[N,N-bis-(1,1,3,3-tetramethylbutyl-4-phenyl)-amino]-phenyl}-1,7-dibrom-perylentetracarbonsäurediimid

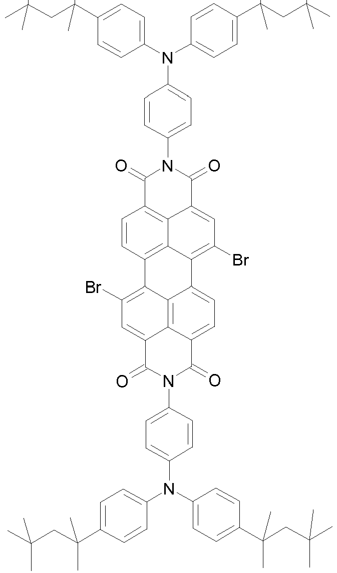

Die Herstellung erfolgte entsprechend der in Beispiel 7 mit 3,30 g (6,0 mmol) Dibromperylentetracarbonsäuredianhydrid anstelle von 1,6,7,12-Tetrachlorperylentetracarbonsäuredianhydrid. Es wurden 2,08 g (23 % d. Th.) rotbrauner Feststoff erhalten. Laut ¹H-NMR enthielt die Substanz geringe Mengen des 1,6-Dibrom-Isomers.
UV/Vis (Methylenchlorid): λₘₐₓ (ε) = 526 nm (60510)

| | |
|---|---|
| C₉₂H₉₈Br₂N₄O₄ (M = 1483,64 g/mol): | Ber. C 74,48 H 6,66 Br 10,77 N 3,78 O 4,31 |
| | Gef. C 74,5 H 6,8 Br 11,4 N 3,7 O 4,0 |

### Beispiel 10: Herstellung von N,N'-Bis{3-[4-(N,N-di-n-hexylphenyl-amino)-phenyl]-propyl}-1,6,7,12-tetrachlorperylentetracarbonsäurediimid

### a) N,N-(Di-4-n-hexylphenyl)-anilin

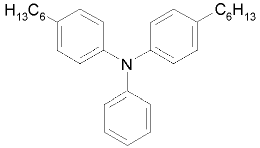

Eine Mischung von 203 g (0,845 mol) 4-Brom-n-hexylbenzol (hergestellt nach M. P. Aldred et al., J. Mater. Chem. 2005, 15, 3208 - 3213), 30,2 g (0,325 mol) Anilin, 105 g (1,09 mol) Natrium-tert.-butylat, 11 g (0,012 mol) Dipalladium-tris(dibenzylidenaceton) und 8,4 g (0,015 mol) 1,1'-Bis(diphenylphosphino)ferrocen in 1000 ml Toluol wurden auf 90 °C erhitzt und 16 Stunden bei dieser Temperatur gehalten. Nach dem Abkühlen auf Raumtemperatur wurde Methylenchlorid zugegeben. Die Mischung wurde filtriert. Das Filtrat wurde bis zur Trockne eingeengt. Der Rückstand wurde in Methylenchlorid aufgenommen und an Kieselgel mit Petrolether als Laufmittel chromatographisch gereinigt. Es wurden 120 g (90 % d. Th.) des Triarylamins erhalten.

Analyse ¹H-NMR:
¹H-NMR (400 MHz, CDCl₃): δ = 7,20 (mc, 2H, arom. H), 7,00 (mc; 11 H, arom. H), 2,55 (t; 4H, Ph-CH₂-), 1,60 (mc; 4H, Ph-CH₂-CH₂-), 1,27 - 1,37 (m; 12H, -CH₂-), 0,83 - 0,91 ppm (t; 6H, -CH₃).

### b) 4-[N,N-(Di-4-n-hexylphenyl)-amino]-benzaldehyd

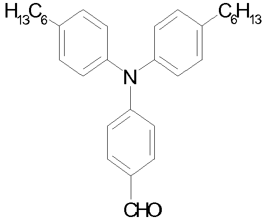

Zu einer Lösung von 234 g (0,567 mol) N,N-(Di-4-n-hexylphenyl)-anilin in 940 ml DMF wurden 181 g (1,19 mol) Phosphoroxychlorid bei 0 °C getropft. Anschließend wurde die Reaktionsmischung auf 95 °C erhitzt und 24 Stunden bei dieser Temperatur gerührt. Die Reaktionsmischung wurde in Eiswasser gegossen, mit NaOH neutraiisiert und mit Methylenchlorid extrahiert. Die organische Phase wurde abgetrennt, mit Wasser gewaschen und über Natriumsulfat getrocknet. Das nach Einengen zur Trockne erhaltene Rohprodukt wurde an Kieselgel chromatographisch gereinigt. Es wurden 170 g (67 % d. Th.) des gewünschten Aldehyds erhalten.

Analyse ¹H-NMR:
¹H-NMR (400 MHz, CDCl₃): δ = 9,77 (s; 1 H, CHO), 7,64 (mc; 2H, arom. H), 7,14 (mc; 4H, arom. H), 7,08 (mc; 4H, arom. H), 6,94 (mc; 2H, arom. H), 2,59 (t; 4H, Ph-CH₂-), 1,62 (m; 4H, Ph-CH₂-CH₂-), 1,31 - 1,35 (m; 12H, -CH₂-), 0,89 ppm (t; 6H, -CH₃).

### c) 4-[N,N-(Di-4-n-hexylphenyl)-amino]-zimtsäurenitril

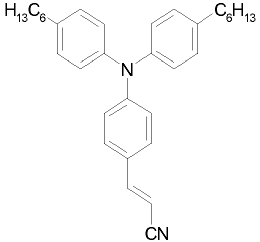

Zu einer Lösung von 73,5 g (0,415 mol) Cyanomethanphosphonsäuredimethylester in 2500 ml wasserfreiem THF wurden 170 ml (2,5 mol/l) n-Butyllithium bei -78 °C unter Stickstoff gegeben. Nach 2stündigem Rühren bei -78 °C wurde eine Lösung von 170,6 g (0,386 mol) 4-[N,N-(Di-4-n-hexylphenyl)-amino]-benzaldehyd in 1200 ml wasserfreiem THF zugegeben. Anschließend ließ man die Reaktionsmischung unter Rühren innerhalb von 3 Stunden auf Raumtemperatur kommen. 1000 ml Wasser wurden zugefügt. Die Mischung wurde mit Essigsäureethylester extrahiert. Die vereinigten organischen Phasen wurden getrocknet, filtriert und bis zur Trockne eingeengt. Das Rohprodukt wurde an Kieselgel chromatographisch gereinigt. Es wurden 172 g (96 % d. Th.) des gewünschten Nitrils erhalten.

Analyse ¹H-NMR:
¹H-NMR (400 MHz, CDCl₃): δ = 7,22 - 7,25 (m; 3H, arom. H, =CH-Ph), 7,09 - 7,11 (m; 4H, arom. H), 7,02 - 7,04 (m; 4H, arom. H), 6,91 (d; 2H, arom. H), 5,62 (d; 1 H, NC-CH=), 2,57 (t; 4H, Ph-CH₂-), 1,59 - 1,62 (m; 4H, Ph-CH₂-CH₂-), 1,32 - 1,36 (m; 12H, - CH₂-), 0,87 ppm (t; 6H, -CH₃).

### d) 4-(3-Aminopropyl)-N,N-di-(4-n-hexylphenyl)-anilin

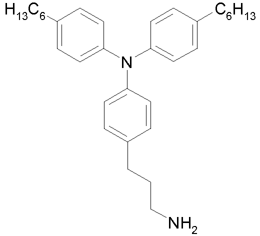

Eine Lösung von 47 g (0,195 mol) 4-[N,N-(Di-4-n-hexylphenyl)-amino]-zimtsäurenitril in 2000 ml n-Propanol wurde 30 min lang unter Rückfluss zum Sieden erhitzt. Anschließend wurden 120 g (5,2 mol) Natrium portionsweise zugegeben. Die Reaktionsmischung wurde eine Stunde lang gerührt und dann auf Raumtemperatur abgekühlt. 500 ml Wasser wurden zugegeben. Die Reaktionsmischung wurde mit Essigester extrahiert. Die organische Phase wurde getrocknet, filtriert und bis zur Trockne eingeengt.

Das Rohprodukt wurde an Kieselgel chromatographisch gereinigt. Es wurden 43,7 g (92 % d. Th.) Amin erhalten.

Analyse ¹H-NMR:
¹H-NMR (400 MHz, CDCl₃): δ = 7,01 - 7,03 (m; 6H, arom. H), 6,95 - 6,97 (m; 6H, arom.
H), 2,74 (t; 2H, -CH₂-NH₂), 2,59 (mc; 2H, -CH₂-CH₂-CH₂-NH₂), 2,53 (mc; 4H, Ph-CH₂-), 1,77 (mc; 2H, -CH₂CH₂-NH₂), 1,57- 1,61 (m; 4H, Ph-CH₂-CH₂-), 1,30 - 1,32 (m; 12H,-CH₂-), 0,88 ppm (t; 6H, -CH₃).

### e) N,N'-Bis{3-[4-(N,N-di-n-hexylphenyl-amino)-phenyl]-propyl}-1,6,7,12-tetrachlorperylentetracarbonsäurediimid

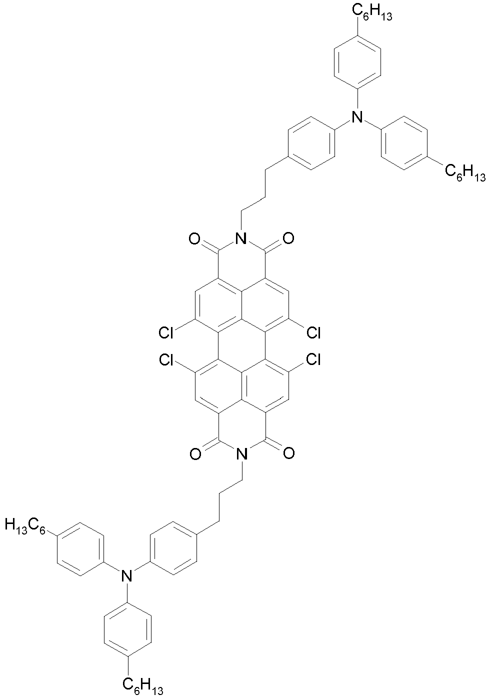

1,06 g (2,00 mmol) 1,6,7,12-Tetrachlorperylentetracarbonsäurediimid und 1,18 g (5,40 mmol) 4-(3-Aminopropyl)-N,N-di-(4-n-hexylphenyl)-anilin wurden in 20 ml Chlorbenzol unter Stickstoff 12 Stunden lang bei 80 °C gerührt. Nach Abkühlen der Reaktionsmischung auf Raumtemperatur wurde diese bis zur Trockne unter vermindertem Druck eingeengt. Das so erhaltene Öl wurde an Kieselgel mit Methylenchlorid / n-Heptan (5 : 1) chromatographisch gereinigt. Nach Entfernen des Lösungsmittels wurden 1,28 g (45 % d. Th.) rotbrauner Feststoff mit einem Schmp. von 146 °C erhalten.
UV/Vis (Methylenchlorid): λₘₐₓ (ε) = 518 nm (42070)

| | |
|---|---|
| C₉₀H₉₂CL₄N₄O₄ (M = 1435,57 g/mol): | Ber. C 75,30 H 6,46 Cl 9,88 N 3,90 |
| | Gef. C 75,2 H 6,5 Cl 9,8 N 3,9 |

### Beispiel 11: Herstellung von N,N'-Bis{3-[4-(N,N-di-n-hexylphenyl-amino)-phenyl]-propyl}-1,7-dibromperylentetracarbonsäurediimid

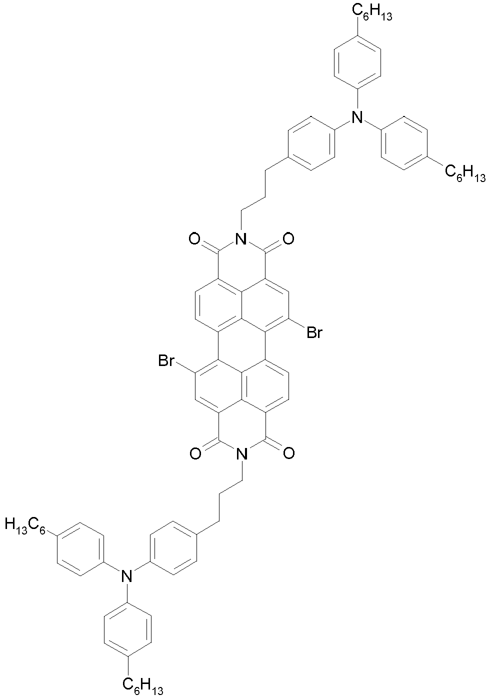

Die Herstellung erfolgte entsprechend der von Beispiel 10e mit 3,00 g (5,45 mmol) Dibromperylentetracarbonsäuredianhydrid anstelle von 1,6,7,12-Tetrachlorperylentetracarbonsäuredianhydrid. Es wurden 1,28 g (25 % d. Th.) rotbrauner Feststoff erhalten.
UV/Vis (Methylenchlorid): λₘₐₓ (ε) = 524 nm (50430)

| | |
|---|---|
| C₉₀H₉₄Br₂N₄O₄ (M = 1455,59 g/mol): | Ber. C 74,27 H 6,51 Br 10,98 N 3,85 O 4,40 |
| | Gef. C 74,8 H 6,9 Br 10,5 N 3,7 O 4,1 |

### Beispiel 12: Herstellung von N,N'-Bis{3-[4-(N,N-di-n-hexyloxyphenyl-amino)-phenyl]-propyl}-1,7-dibromperylentetracarbonsäurediimid

### a) N,N-(Di-4-n-hexyloxyphenyl)-anilin

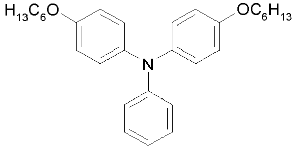

Die Herstellung erfolgte entsprechend der von Beispiel 10a) mit 128 g (0,50 mol) 4-Brom-n-hexyloxybenzol (hergestellt nach S. Sharma et al., Liquid Cryst. 2003, 30, 451 - 461). Es wurden 81 g (81 % d. Th.) Feststoff erhalten.
Analyse ¹H-NMR:
¹H-NMR (400 MHz, CDCl₃): δ = 7,08 (mc, 2H, arom. H), 6,95 (mc; 4H, arom. H), 6,85 (mc; 2H, arom. H), 6,75 (mc; 5H, arom. H), 3,85 (t; 4H, O-CH₂-), 1,70 (mc; 4H, O-CH₂-CH₂-), 1,37 (mc; 4H, -CH₂-), 1,27 (mc; 8H, -CH₂-), 0,85 ppm (t; 6H, -CH₃).

### b) 4-[N,N-(Di-4-n-hexyloxyphenyl)-amino]-benzaldehyd

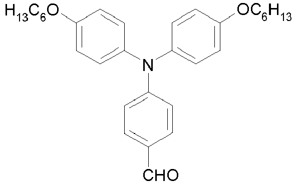

Die Herstellung erfolgte entsprechend der in Beispiel 10b). Aus 130 g (0,29 mol) N,N-(Di-4-n-hexyloxyphenyl)-anilin wurden 111 g (81 % d. Th.) Aldehyd erhalten.

Analyse ¹H-NMR:
¹H-NMR (400 MHz, CDCl₃): δ = 9,75 (s; 1 H, CHO), 7,62 (mc; 2H, arom. H), 7,11 (mc; 4H, arom. H), 6,86 (mc; 6H, arom. H), 3,95 (t; 4H, O-CH₂-), 1,79 (mc; 4H, O-CH₂-CH₂-), 1,47 (mc; 4H, -CH₂-), 1,35 (mc; 8H, -CH₂-), 0,93 ppm (t; 6H, -CH₃).

### c) 4-[N,N-(Di-4-n-hexyloxyphenyl)-amino]-zimtsäurenitril

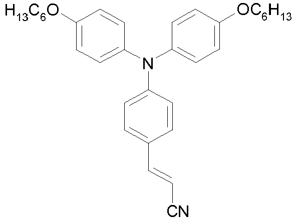

Die Herstellung erfolgte entsprechend der in Beispiel 10c). Aus 69 g (0,145 mol) 4-[N,N-(Di-4-n-hexyloxyphenyl)-amino]-benzaldehyd wurden 64 g (89 % d. Th.) Nitril erhalten.

Analyse ¹H-NMR:
¹H-NMR (400 MHz, CDCl₃): δ = 7,19 - 7,27 (m; 3H, arom. H, =CH-Ph), 7,07 (mc; 4H, arom. H), 6,80 - 6,86 (m; 6H, arom. H), 5,61 (d; 1 H, NC-CH=), 2,57 (t; 4H, Ph-CH₂-), 1,59 - 1,62 (m; 4H, O-CH₂-CH₂-), 1,32 - 1,36 (m; 12H, -CH₂-), 0,87 ppm (t; 6H,-CH₃).

### d) 4-(3-Aminopropyl)-N,N-di-(4-n-hexyloxyphenyl)-anilin

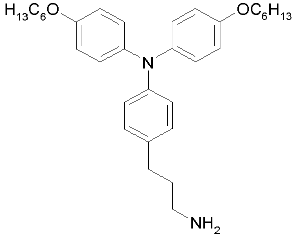

Die Herstellung erfolgte entsprechend der in Beispiel 10d). Aus 47 g (0,195 mol) 4-[N,N-(Di-4-n-hexyloxyphenyl)-amino]-zimtsäurenitril wurden 43,7 g (92 % d. Th.) öliges Amin erhalten.

Analyse ¹H-NMR:
¹H-NMR (400 MHz, CDCl₃): δ = 6,97 - 7,01 (m; 6H, arom. H), 6,85 - 6,88 (m; 2H, arom. H), 6,76 - 6,80 (m; 4H, arom. H), 3,91 (t; 4H, O-CH₂-), 2,74 (t; 2H, -CH₂-NH₂), 2,57 (t; 2H, -CH₂-Ph), 1,72 - 1,79 (m; 6H, -CH₂-), 1,55 (mc; 2H, -CH₂-), 1,44 (mc; 4H, -CH₂-), 1,35 (mc; 8H, -CH₂-), 0,89 ppm (t; 6H, -CH₃).

### e) N,N'-Bis{3-[4-(N,N-di-n-hexyloxyphenyl-amino)-phenyl]-propyl}-1,7-dibromperylentetracarbonsäurediimid

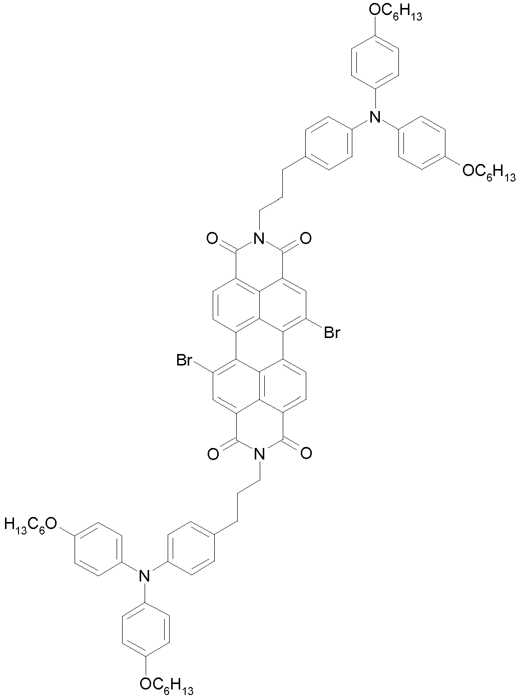

Die Herstellung erfolgte entsprechend der in Beispiel 10e) mit 3,00 g (5,45 mmol) Dibromperylentetracarbonsäuredianhydrid. Es wurden 3,68 g (44 % d. Th.) rote Mikrokristalle erhalten, die bei 84 - 85 °C schmolzen.
UV/Vis (Methylenchlorid): λₘₐₓ (ε) = 524 nm (52790)

| | |
|---|---|
| C₉₀H₉₄Br₂N₄O₈ (M = 1519,58 g/mol): | Ber. C 71,14 H 6,24 Br 10,52 N 3,69 O 8,42 |
| | Gef. C 71,0 H 6,4 Br 10,8 N 3,8 O 8,6 |

### Beispiel 13: Herstellung von N,N'-Bis[3-(3,6-di-n-hexyl-N-carbazolyl)-propyl]-1,7-dicyanoperylentetracarbonsäurediimid

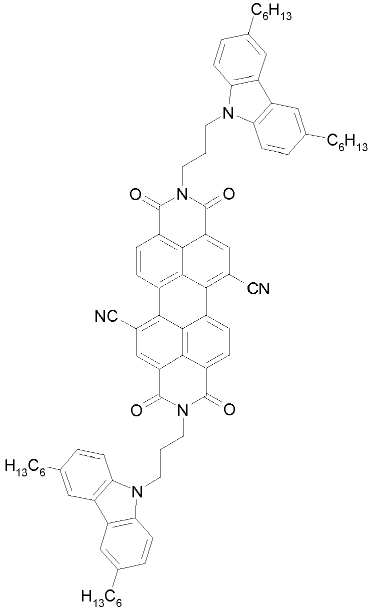

a) 2,50 g (1,92 mmol) N,N'-Bis[3-(3,6-di-n-hexyl-N-carbazolyl)-propyl]-1,7-dibromperylentetracarbonsäurediimid, 0,28 g (0,76 mmol) Kaliumhexacyanoferrat(II), 0,070 g (0,385 mmol) 98 %iges Kupferiodid und 0,48 g (3,85 mmol) 1-Butylimidazol wurden in 25 ml 1,3,5-Trimethylbenzol gegeben. Die Suspension wurde 6 Stunden lang unter Rückfluss zum Sieden erhitzt. Nach Abkühlen der Reaktionsmischung auf Raumtemperatur wurden 80 ml Methanol zugegeben. Der Niederschlag wurde abgesaugt, mit Methanol gewaschen und getrocknet (2,6 g). Das Rohprodukt wurde in 80 ml Methylenchlorid gelöst und an Kieselgel (60 Å, 60 - 200 µm) chromatographisch gereinigt. Es wurden 1,72 g (19 % d. Th.) schwarzer Feststoff erhalten.
b) Eine Lösung von 1,50 g (1,15 mmol) N,N'-Bis[3-(3,6-di-n-hexyl-N-carbazolyl)-propyl]-1,7-dibromperylentetracarbonsäurediimid in 15 ml N-Methylpyrrolidinon wurde mit 0,31 g (3,5 mmol) Kupfercyanid versetzt und auf 150 °C erhitzt. Nach einstündigem Rühren bei dieser Temperatur ließ man die Reaktionsmischung auf Raumtemperatur abkühlen. Nach der Zugabe von 50 ml Methanol wurde die Reaktionsmischung kurz gerührt und dann filtriert. Der Rückstand wurde mit 8 ml gesättigter Natriumhydrogencarbonatlösung aufgeschlämmt, abgesaugt, mit Wasser und dann mit Methanol gewaschen und getrocknet (1,3 g). Das Rohprodukt wurde in 80 ml Methylenchlorid gelöst und an Aluminiumoxid (neutral, 58 Å) mit Methylenchlorid / n-Heptan (5:1) chromatographisch gereinigt. Es wurden 0,65 g (47 % d. Th.) schwarzer Feststoff erhalten, der bei 242 - 244 °C schmolz. Laut ¹H-NMR enthielt die Substanz als Nebenprodukt das 1,6-Dicyano-Isomer.
UV/Vis (Methylenchlorid): λₘₐₓ (ε) = 524 nm (53700)

| | |
|---|---|
| C₈₀H₈₂N₆O₄ (M = 1191,58 g/mol): | Ber. C 80,64 H 6,94 N 7,05 |
| | Gef. C 80,8 H 6,8 N 6,9 |

Beispiel 14: Herstellung von N,N'-Bis[4-(N,N-di-t-octylphenyl-amino)-phenyl]-1,7-dicyano-perylentetracarbonsäurediimid

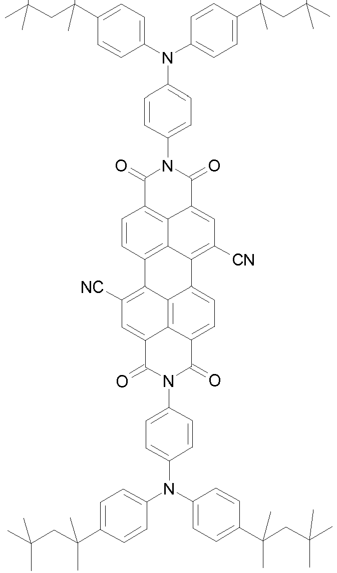

Die Herstellung erfolgte entsprechend der in Beispiel 13b) aus 1,50 g (1,01 mmol) N,N'-Bis{4-[N,N-bis-(1,1,3,3-tetramethylbutyl-4-phenyl)-amino]-phenyl}-1,7-dibromperylentetracarbonsäurediimid. Nach Umkristallisation in Nitroethan wurden 0,38 g (27 % d. Th.) dunkelrote Mikrokristalle erhalten, die bei 333 - 334 °C schmolzen.
UV/Vis (Methylenchlorid): λₘₐₓ (ε) = 524 nm (71050)

| | |
|---|---|
| C₉₄H₉₈N₆O₄ (M = 1375,87 g/mol): | Ber. C 82,06 H 7,18 N 6,11 O 4,65 |
| | Gef. C 81,4 H 7,2 N 5,9 O 4,7 |

### Beispiel 15: Herstellung von N,N'-Bis{3-[4-(N,N-di-n-hexyloxyphenyl-amino)-phenyl]-propyl}-1,7-dicyanoperylentetracarbonsäurediimid

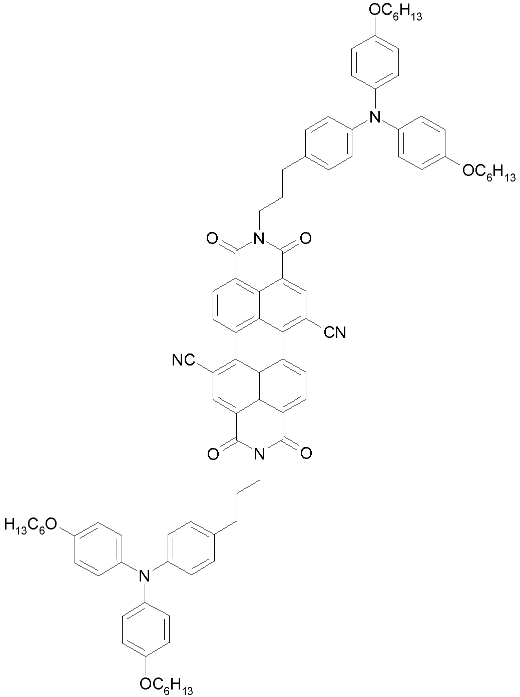

Die Herstellung erfolgte entsprechend der in Beispiel 13b) aus 2,00 g (1,32 mmol) N,N'-Bis{3-[4-(N,N-di-n-hexyloxyphenyl-amino)-phenyl]-propyl}-1,7-dibromperylentetracarbonsäurediimid. Es wurden 1,06 g (57 % d. Th.) dunkelrote Mikrokristalle erhalten, die bei 207 °C schmolzen.
UV/Vis (Methylenchlorid): λₘₐₓ (ε) = 524 nm (60680)

| | |
|---|---|
| C₉₂H₉₄N₆O₈ (M = 1411,81 g/mol): | Ber. C 78,27 H 6,71 N 5,95 O 9,07 |
| | Gef. C 78,3 H 7,0 N 5,8 O 9,0 |

### Beispiel 16: Herstellung von N,N'-Bis[2-(3,6-di-n-hexyl-N-carbzolyl)-ethyl]-naphthalintetracarbonsäurediimid

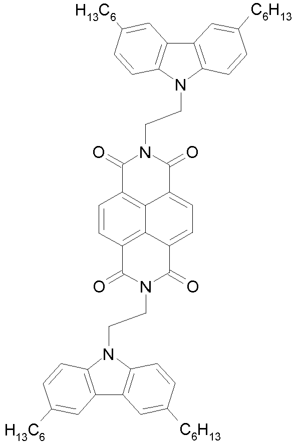

Eine Lösung von 1,50 g (5,31 mmol) 95 %iges 1,4,5,8-Naphthalintetracarbonsäuredianhydrid und 4,22 g (11,2 mmol) N-(2-Aminoethyl)-3,6-di-n-hexylcarbazol (siehe Beispiel 1d)) in 50 ml NMP wurde 2,5 Stunden lang bei 120 °C erhitzt. Nach dem Abkühlen auf Raumtemperatur wurde die Reaktionslösung mit 100 ml Methanol versetzt. Der Niederschlag wurde abgesaugt, gewaschen und getrocknet (5,29 g). Das Rohprodukt wurde in Toluol umkristallisiert. Es wurden 4,41 g (84 % d. Th.) brauner Feststoff erhalten, der bei 252 °C schmolz.
UV/Vis (Methylenchlorid): λₘₐₓ (ε) = 378 nm (30360)

| | |
|---|---|
| C₆₆H₇₆N₄O₄ (M = 989,37 g/mol): | Ber. C 80,13 H 7,74 N 5,66 O 6,47 |
| | Gef. C 80,2 H 7,6 N 5,7 O 6,4 |

### Beispiel 17: Herstellung von N,N'-Bis[3-(3,6-di-n-hexyl-N-carbzolyl)-propyl]-naphthalintetracarbonsäurediimid

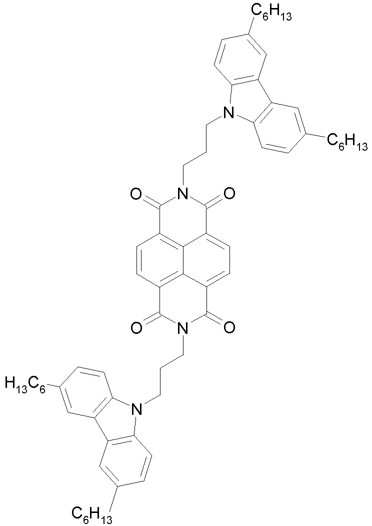

Die Herstellung erfolgte entsprechend der in Beispiel 17 mit N-(3-Aminopropyl)-3,6-di-n-hexylcarbazol. Aus 1,50 g (5,31 mmol) 1,4,5,8-Naphthalintetracarbonsäuredianhydrid wurden 4,53 g (80 % d. Th.) blassvioletter Feststoff erhalten, der bei 176 - 178 °C schmolz.
UV/Vis (Methylenchlorid): λₘₐₓ (ε) = 380 nm (26510)

| | |
|---|---|
| C₆₈H₈₀N₄O₄ (M = 1017,42 g/mol): | Ber. C 80,28 H 7,93 N 5,51 O 6,29 |
| | Gef. C 80,4 H 7,9 N 5,5 O 6,4 |

### Beispiel 18: Herstellung von N,N'-Bis{3-[4-(N,N-di-n-hexylphenyl-amino)-phenyl]-propyl}-naphthalintetracarbonsäurediimid

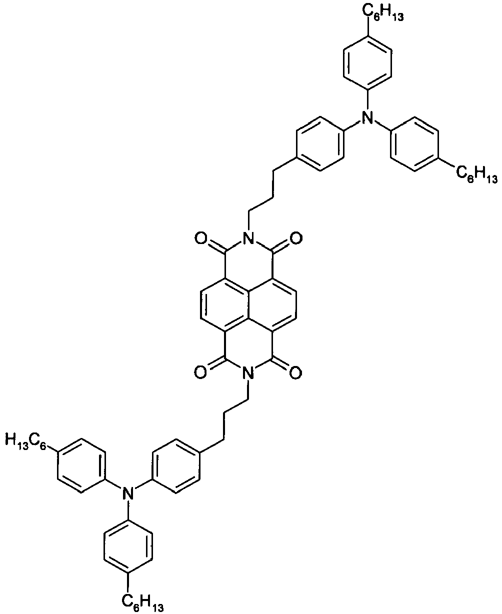

Eine Lösung von 1,58 g (5,59 mmol) 95 %iges 1,4,5,8-Naphthalintetracarbonsäuredianhydrid und 5,53 g (11,8 mmol) 4-(3-Aminopropyl)-N,N-di-(4-n-hexylphenyl)-anilin (siehe Beispiel 10d)) in 50 ml Toluol wurden 5 Stunden lang bei 80 °C erhitzt. Nach dem Abkühlen auf Raumtemperatur wurde das Reaktionsgemisch bis zur Trockne eingeengt (dunkelblaues Öl). Das Rohprodukt wurde an Kieselgel (60 Å, 60 - 200 µm) mit Methylenchlorid / n-Heptan (4:1) als Laufmittel chromatographisch gereinigt. Es wurden 5,15 g (75 % d. Th.) blauer harziger Feststoff erhalten, der bei 50 - 54 °C schmolz.
UV/Vis (Methylenchlorid): λₘₐₓ (ε) = 380 nm (28280)

| | |
|---|---|
| C₈₀H₉₂N₄O₄ (M = 1173,65 g/mol): | Ber. C 81,87 H 7,90 N 4,77 O 5,45 |
| | Gef. C 81,8 H 8,0 N 4,7 O 5,5 |

Aus n-Hexan kristallisierten blaustichige farblose Nadeln, die bei 104 - 105 °C schmolzen. Das ¹H-NMR-Spektrum dieser Substanz war mit dem des blauen harzigen Feststoffs identisch.

### Beispiel 21: Herstellung von 3-(3,6-Dimethyl-N-carbazolyl)-propyl-ammoniumchlorid

### a) N-[3-(N-Phthalimido)-propyl]-3,6-dimethyl-carbazol

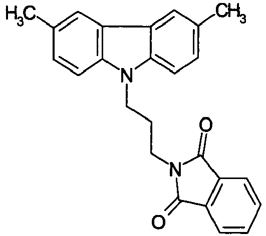

Die Herstellung erfolgte entsprechend der in Beispiel 2a). Aus 9,76 g (50,0 mmol) 3,6-Dimethylcarbazol wurden 18,32 g (96 % d. Th.) gelbe Mikrokristalle hergestellt, die bei 134 - 135 °C schmolzen.

### b) 3-(3,6-Dimethyl-N-carbazolyl)-propyl-ammoniumchlorid

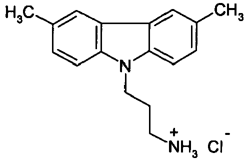

Zu einer Lösung von 15,00 g (39,2 mmol) N-[3-(N-Phthalimido)-propyl]-3,6-dimethylcarbazol in jeweils 250 ml THF und Ethanol wurden 3,93 g (78,4 mmol) 98 %iges Hydrazin-Monohydrat gegeben. Die Lösung wurde 20 Stunden lang zum Sieden unter Rückfluss erhitzt, wobei ein Niederschlag ausfiel. Nach Abkühlen auf Raumtemperatur wurden 600 ml Wasser zugegeben. Nach Entfernung der organischen Lösungsmittel wurden 400 ml Diethylether zugegeben und mit 62 ml 20 % NaOH auf pH 13,5 gestellt. Die wässrige Phase wurde abgetrennt und noch zweimal mit jeweils 150 ml Diethylether extrahiert. Die vereinigten organischen Phasen wurden über Natriumsulfat getrocknet, filtriert und bis zu einem Öl eingeengt, das in 100 ml Methylenchlorid gelöst und dann mit 15 ml konz. HCl versetzt wurde, wobei ein Niederschlag ausfiel. Dieser wurde abgesaugt, mit Methylenchlorid und n-Pentan gewaschen und getrocknet. Es wurden 7,08 (63 % d. Th.) farblose Kristalle erhalten, die bei 247 °C schmolzen.

Analyse ¹H-NMR:
¹H-NMR (D₆-DMSO, 400 MHz): δ = 8,12 (s; 3H), 7,90 (s; 2H), 7,55 (d; 2H), 7,27 (d; 2H), 4,48 (t; 2H), 2,79 (t; 2H), 2,07 ppm (q; 2H)

### Beispiel 22: Herstellung von N-[2-(4-Diphenylamino-phenyl)-ethyl]-pivalinsäureamid

### a) 4-(N,N-Diphenylamino)-phenyl-acetonitril

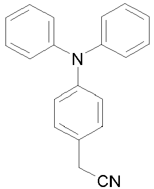

Eine Lösung von 68,3 g (0,35 mol) (p-Toluolsulfonyl)methylisocyanid (TOSMIC) in 300 ml Dimethoxyethan wurde zu einer gerührten Suspension von 78 g (0,70 mol) Kaliumtert.-butylat in 300 ml Dimethoxyethan bei -30 °C unter Stickstoff getropft. Nach Kühlung der Lösung auf -55 °C wurde eine Lösung von 92 g (0,34 mol) 4-(N,N-Diphenylamino)-benzaldehyd in 100 ml Dimethoxyethan getropft. Nach 1 Stunde Rühren bei -55 °C wurde Methanol zugegeben. Die Reaktionslösung wurde 30 min lang zum Sieden unter Rückfluss erhitzt. Nach Einengen der Lösung bis zur Trockne wurde der Rückstand mit 600 ml Wasser und 30 ml Essigsäure aufgenommen. Die wässrige Lösung wurde mit Methylenchlorid extrahiert. Die vereinigten organischen Phasen wurden mit einer gesättigten Natriumcarbonat-Lösung gewaschen, über Magnesiumsulfat getrocknet und bis zur Trockne eingeengt. Es wurden 75 g (79 % d. Th.) des gewünschten Nitrils erhalten.

Analyse ¹H-NMR:
¹H-NMR (CDCl₃, 400 MHz): δ = 7,26 (mc; 5H, arom. H), 7,11 (mc; 2H, arom. H), 7,01 (mc; 7H, arom. H), 3,68 ppm (s; 2H, Ph-CH₂-CN)

### b) N-[2-(4-Diphenylamino-phenyl)-ethyl]-pivalinsäureamid

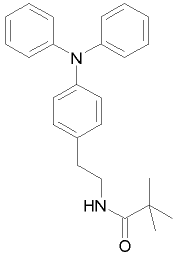

Zu einer auf 0 °C gekühlten Lösung von 150 g (0,528 mol) 4-(N,N-Diphenylamino)-phenyl-acetonitril in 500 ml wasserfreiem Methanol wurden unter Rühren 153 g (0,70 mol) Pyrokohlensäure-di-tert.-butylester und 1,25 g (53 mmol) Nickelchlorid gegeben. 136,8 g (3,70 mol) Natriumborhydrid wurde portionsweise innerhalb von 2 Stunden zugefügt. Nach 30 min Rühren wurde das Lösungsmittel bei vermindertem Druck entfernt. Der rötliche Rückstand wurde in Essigester gelöst und mit gesättigter Natriumhydrogencarbonatlösung ausgeschüttelt. Die organische Phase wurde abgetrennt, über Magnesiumsulfat getrocknet, filtriert und bis zur Trockne eingeengt. Es wurden 100 g (49 % d. Th.) des geschützten Amins erhalten.

Analyse ¹H-NMR:
¹H-NMR (CDCl₃, 400 MHz): δ = 7,17 (mc; 4H, arom. H), 6,99 (mc; 6H, arom. H), 6,90 (mc; 4H, arom. H), 3,29 (m; 2H), 2,66 (m; 2H), 1,35 (s; 9H, -CH₃)

### Beispiel 23: Herstellung von 4-(3-Aminopropyl)-N,N-di-(4-t-octylphenyl)-anilin

### a) N,N-(Di-4-t-octylphenyl)-anilin

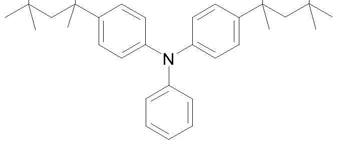

Eine Mischung aus 198,6 g (0,505 mol) 4,4'-Di-tert.-octylphenyl-amin, 102,5 g (0,653 mol), 100 g (1,04 mol) Natrium-tert.-butylat, 10 g (0,011 mol) Dipalladium-tris(dibenzylidenaceton) und 10 g (0,018 mol) 1,1'-Bis(diphenylphosphino)ferrocen in 1600 ml Toluol wurden unter Rühren 16 Stunden lang bei 90 °C erhitzt. Nach dem Abkühlen auf Raumtemperatur wurde Methylenchlorid zugegeben. Die Lösung wurde filtriert und bei vermindertem Druck bis zur Trockne eingeengt. Der Rückstand wurde mit Methylenchlorid aufgenommen und an Kieselgel mit Petrolether als Laufmittel chromatographisch gereinigt. Es wurden 210 g (88 % d. Th.) des gewünschten Triarylamins erhalten.

Analyse ¹H-NMR:
¹H-NMR (CDCl₃, 400 MHz): δ = 7,22 (mc; 6H, arom. H), 7,03 (mc; 2H, arom. H), 6,98 (mc; 4H, arom. H), 6,95 (mc; 1 H, arom. H), 1,71 (s; 4H, -CH₂-), 1,36 (s; 12H, Ph-C(CH₃)₂-), 0,76 ppm (s; 18H, -C(CH₃)₃)

### b) 4-[N,N-(Di-4-t-octylphenyl)-amino]-benzaldehyd

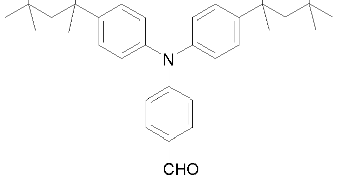

Die Herstellung erfolgte entsprechend der in Beispiel 10b). Aus 290 g (0,618 mol) N,N-(Di-4-t-octylphenyl)-anilin wurden 250 g (83 % d. Th.) Aldehyd erhalten.

Analyse ¹H-NMR:
¹H-NMR (CDCl₃, 400 MHz): δ = 9,78 (s; 1 H, -CHO), 7,65 (d; 2H, arom. H), 7,33 (d; 4H, arom. H), 7,06 (d; 4H, arom. H), 6,95 (d; 2H, arom. H), 1,73 (s; 4H, -CH₂-), 1,36 (s; 12H, Ph-C(CH₃)₂-), 0,75 ppm (s; 18H, -C(CH₃)₃)

### c) 4-[N,N-(Di-4-t-octylphenyl)-amino]-zimtsäurenitril

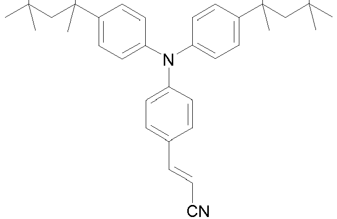

Die Herstellung erfolgte entsprechend der in Beispiel 10c). Aus 52,7 g (0,298 mol) 4-[N,N-(Di-4-t-octylphenyl)-amino]-benzaldehyd wurden 140 g (96 % d. Th.) Nitril erhalten.

Analyse ¹H-NMR:
¹H-NMR (CDCl₃, 400 MHz): δ = 7,23 - 7,31 (m; 7H, arom. H, -CH=CH-CN), 7,03 (mc; 4H, arom. H), 6,93 (mc; 2H, arom. H), 5,64 (d; 1 H, -CH=CH-CN), 1,73 (s; 4H, -CH₂-), 1,36 (s; 12H, Ph-C(CH₃)₂-), 0,75 ppm (s; 18H, -C(CH₃)₃)

### d) 4-(3-Aminopropyl)-N,N-di-(4-t-octylphenyl)-anilin

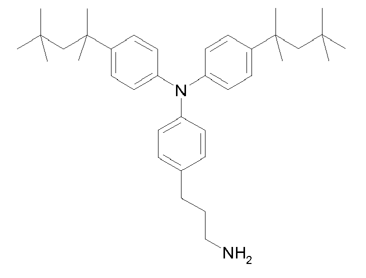

Die Herstellung erfolgte entsprechend der in Beispiel 10d). Aus 130 g (0,250 mol) 4-[N,N-(Di-4-t-octylphenyl)-amino]-zimtsäurenitril wurden 93 g (70 % d. Th.) Amin erhalten.

Analyse ¹H-NMR:
¹H-NMR (CDCl₃, 400 MHz): δ = 7,20 (mc; 4H, arom. H), 7,03 (mc; 2H, arom. H), 6,95 (mc; 6H, arom. H), 2,77 (t; 2H, -CH₂NH₂), 2,60 (t; 2H, Ph-CH₂-), 2,02 (s, breit; 2H, -NH₂), 1,75 - 1,83 (m; 2H, -CH₂-CH₂-CH₂-), 1,69 (s; 4H, -CH₂-), 1,34 (s; 12H, Ph-C(CH₃)₂-), 0,75 ppm (s; 18H, -C(CH₃)₃)

### Beispiel 24: Herstellung von 3-[N,N-(Di-4-hexylphenyl)-4-anilino]-propanthiol

### a) 4-[N,N-(Di-4-hexylphenyl)-amino]-zimtsäureethylester

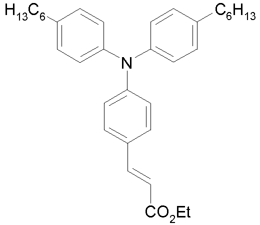

Zu einer Lösung von 46,5 g (0,207 mol) Diethoxyphosphoryl-essigsäurediethylester in 2500 ml wasserfreiem THF wurden 85 ml (2,5 mol/l) n-Butyllithium bei -78 °C unter Stickstoff gegeben. Nach zweistündigem Rühren bei -78 °C wurde eine Lösung von 85,0 g (0,193 mol) 4-[N,N-(Di-4-n-hexylphenyl)-amino]-benzaldehyd in 800 ml wasserfreiem THF zugegeben. Anschließend ließ man die Reaktionsmischung unter Rühren innerhalb von 3 Stunden auf Raumtemperatur kommen. 800 ml Wasser wurden zugefügt. Die Mischung wurde mit Essigsäureethylester extrahiert. Die vereinigten organischen Phasen wurden getrocknet, filtriert und bis zur Trockne eingeengt. Das Rohprodukt wurde an Kieselgel chromatographisch gereinigt. Es wurden 80 g (81 % d. Th.) des gewünschten Zimtsäureesters erhalten.
¹H-NMR (400 MHz, CDCl₃): δ = 7,53 (mc, 1 H), 7,26 (mc, 1 H), 7,26 (mc, 2H), 6,95 - 7,03 (m, 8H), 6,86 (m, 2H), 6,16 - 6,20 (m, 1 H), 4,12 - 4,20 (m, 2H), 2,48 - 2,52 (t, 4H), 1,50 - 1,57 (m, 4H), 1,19 - 1,30 (m, 15H), 0,83 ppm (t, 6H).

### b) 3-[N,N-(Di-4-hexylphenyl)-4-anilino]-propionsäureethylester

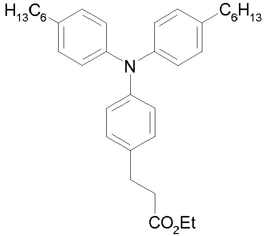

Eine Lösung von 82 g (0,16 mol) 4-[N,N-(Di-4-hexylphenyl)-amino]-zimtsäureethylester in 400 ml Tetrahydrofuran wurde in einer Wasserstoff-Atmosphäre in Gegenwart von 18 g Palladium-Katalysator (10 Gew.-% auf aktiviertem Kohlenstoff) bei Raumtemperatur gerührt. Nach 24 Stunden wurde die Reaktionsmischung durch eine Schicht Celite filtriert, um den Katalysator zu entfernen. Einengen des Filtrats bis zur Trockne lieferte 78 g (95 % d. Th.) des Esters.
¹H-NMR (400 MHz, CDCl₃): δ = 6,88 - 7,00 (m, 12H), 4,04 - 4,09 (m, 2H), 2,79 - 2,83 (m, 2H), 2,45-2,55 (m, 6H), 1,49 - 1,56 (m, 4H), 1,15 - 1,29 (m, 15H), 0,83 ppm (mc, 6H).

### c) 3-[N,N-(Di-4-hexylphenyl)-4-anilino]-propanol

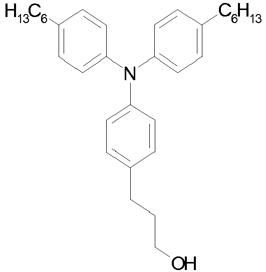

Zu einer Lösung von 10,5 g (0,276 mol) Lithiumaluminiumhydrid in 1000 ml Tetrahydrofuran wurden bei Raumtemperatur unter Stickstoff 70,8 g (0,138 mol) 3-[N,N-(Di-4-hexylphenyl)-4-anilino]-propionsäureethylester gegeben. Nach Beendigung der Zugabe wurde die Lösung auf 60 °C erwärmt und 24 Stunden bei dieser Temperatur gerührt. Nach dem Abkühlen auf Raumtemperatur wurde soviel Wasser zugesetzt, bis kein Wasserstoff mehr festgestellt wurde. Nach Filtration und Trocknen mit Natriumsulfat wurde die Lösung bis zur Trockne eingeengt. Es wurden 61 g (93 % d. Th.) des gewünschten Alkohols erhalten.
¹H-NMR (400 MHz, CDCl₃): δ = 6,88 - 7,01 (m, 12H), 3,60 - 3,69 (m, 4H), 5,58 - 5,60 (t, 2H), 2,57 - 2,61 (t, 2H), 2,51 - 2,55 (t, 4H), 1,76 - 1,85 (m, 4H), 1,42 - 1,56 (m, 4H), 1,15 - 1,29 (m, 15H), 0,82 - 0,84 ppm (t, 6H).

### d) 4-(3-Brompropyl)-N,N-(di-4-hexylphenyl)-anilin

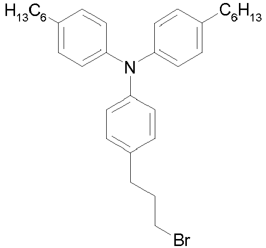

60 g (0,125 mol) 3-[N,N-(Di-4-hexylphenyl)-4-anilino]-propanol wurden in 600 ml 40 % HBr gelöst. Die Lösung wurde auf 80 °C erhitzt und 24 Stunden lang bei dieser Temperatur gerührt. Nach dem Abkühlen auf Raumtemperatur wurde die Lösung mit Methylenchlorid extrahiert. Die vereinigten organischen Phasen wurden über Natriumsulfat getrocknet, filtriert und bis zur Trockne eingeengt. Es wurden 62 g (94 % d. Th.) des Amins erhalten.
¹H-NMR (400 MHz, CDCl₃): δ = 6,88 - 7,01 (m, 12H), 3,32 - 3,36 (t, 2H), 2,62 - 2,66 (t, 2H), 2,45-2,49 (t, 4H), 2,04-2,11 (m, 2H), 1,48- 1,56 (m, 4H), 1,23 - 1,31 (m, 12H), 0,82 - 0,84 ppm (m, 6H).

### e) 3-[N,N-(Di-4-hexylphenyl)-4-anilino]-propanthiol

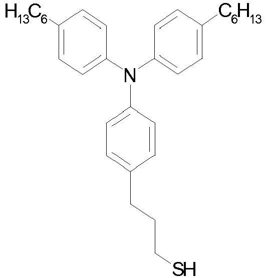

Eine Mischung von 53,3 g (0,10 mol) 4-(3-Brompropyl)-N,N-(di-4-hexylphenyl)-anilin und 9,6 g (0,10 mol) Thioharnstoff wurden in 300 ml Ethanol 48 Stunden unter Rückfluss zum Sieden erhitzt. Nach der Zugabe von 50 ml 40 % NaOH wurde die Reaktionsmischung 4 Stunden bei 60 °C gerührt. Nach dem Abkühlen wurde diese mit Methylenchlorid extrahiert. Die vereinigten organischen Phasen wurden getrocknet und dann bis zur Trockne eingeengt. Es wurden 36,5 g (75 % d. Th.) des gewünschten Thiols erhalten.
¹H-NMR (400 MHz, CDCl₃): δ = 6,91 - 7,01 (m, 12H), 2,63 - 2,71 (m, 3H), 2,50 - 2,54 (m, 5H), 1,88 -2,00 (m, 2H), 1,54 - 1,61 (m, 4H), 1,25 - 1,36 (m,12H), 0,82 - 0,84 ppm (m, 6H).

### Beispiel 25: Herstellung von 3-[N,N-(Di-4-hexyloxyphenyl)-4-anilino]-propanthiol a) 4-[N,N-(Di-4-hexyloxyphenyl)-amino]-zimtsäureethylester

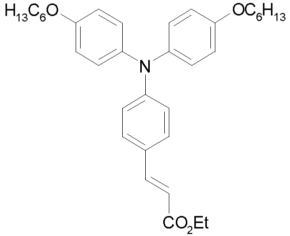

Die Herstellung erfolgte entsprechend der von 4-[N,N-(Di-4-hexylphenyl)-amino]-zimtsäureethylester (Beispiel 24a). Aus 140 g (0,296 mol) 4-[N,N-(Di-4-n-hexyloxyphenyl)-amino]-benzaldehyd (Beispiel 12b) wurden 130 g (81 % d. Th.) des Zimtsäureesters erhalten.
¹H-NMR (400 MHz, CDCl₃): δ = 7,59 (mc, 1 H), 7,30 (mc, 2H), 7,04 - 7,08 (m, 4H), 6,82 - 6,86 (m, 6H), 6,23 (mc, 1 H), 4,21 - 4,26 (m, 2H), 3,93 (t, 4H), 1,74 - 1,81 (m, 4H), 1,30 - 1,50 (m, 15H), 0,93 ppm (t, 6H).

### b) 3-[N,N-(Di-4-hexyloxyphenyl)-4-anilino]-propionsäureethylester

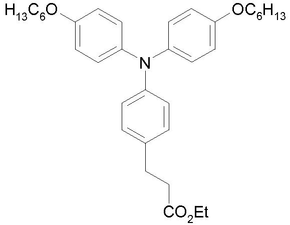

Die Herstellung erfolgte entsprechend der von 3-[N,N-(Di-4-hexylphenyl)-4-anilino]-propionsäureethylester (Beispiel 24b). Aus 182 g (0,335 mol) 4-[N,N-(Di-4-hexyloxyphenyl)-amino]-zimtsäureethylester (Beispiel 25a) wurden 178 g (97 % d. Th.) des Propionsäureesters erhalten.
¹H-NMR (400 MHz, CDCl₃): δ = 6,78 - 7,01 (m, 12H), 4,11 - 4,16 (m, 2H), 3,92 (t, 4H), 2,85 - 2,89 (m, 2H), 2,57 - 2,59 (m, 2H), 1,73 -1,80 (m, 4H), 1,23 - 1,50 (m, 15H), 0,92 ppm (t, 6H).

### c) 3-[N,N-(Di-4-hexyloxyphenyl)-4-anilino]-propanol

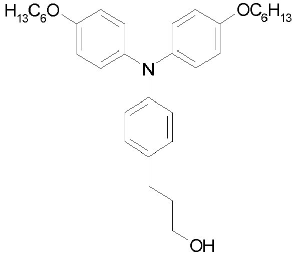

Die Herstellung erfolgte entsprechend der von 3-[N,N-(Di-4-hexylphenyl)-4-anilino]-propanol (Beispiel 24c). Aus 178 g (0,327 mol) 3-[N,N-(Di-4-hexyloxyphenyl)-4-anilino]-propionsäureethylester (Beispiel 25b) wurden 161 g (98 % d. Th.) des Alkohols erhalten.
¹H-NMR (400 MHz, CDCl₃): δ = 7,01 (mc, 6H), 6,87 (mc, 2H), 6,70 (mc, 4H), 3,92 (t, 4H), 3,67 (mc, 2H), 2,62 (mc, 2H), 1,88 (mc, 2H), 1,78 (mc, 4H), 1,46 (mc, 4H), 1,34 (mc, 8H), 0,90 ppm (t, 6H).

### d) 4-(3-Brompropyl)-N,N-(di-4-hexyloxyphenyl)-anilin

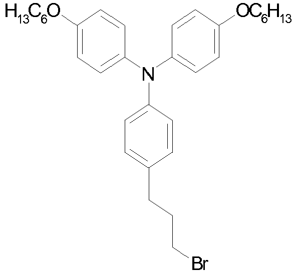

Die Herstellung erfolgte entsprechend der von 4-(3-Brompropyl)-N,N-(di-4-hexyl-phenyl)-anilin (Beispiel 24d). Aus 60 g (0,125 mol) 3-[N,N-(Di-4-hexyloxyphenyl)-4-anilino]-propanol (Beispiel 25c) wurden 62 g (94 % d. Th.) des Amins erhalten. ¹H-NMR (400 MHz, CDCl₃): δ = 7,01 (mc, 6H), 6,89 (mc, 2H), 6,70 (mc, 4H), 3,93 (t, 4H), 3,42 (t, 2H), 2,70 (t, 2H), 2,16 (mc, 2H), 1,77 (mc, 4H), 1,47 (mc, 4H), 1,35 (mc, 8H), 0,92 ppm (t, 6H).

### e) 3-[N,N-(Di-4-hexyloxyphenyl)-4-anilino]-propanthiol

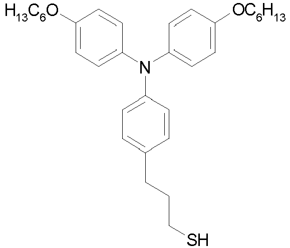

Die Herstellung erfolgte entsprechend der von 3-[N,N-(Di-4-hexylphenyl)-4-anilino]-propanthiol (Beispiel 24e). Aus 164 g (0,29 mol) 4-(3-Brompropyl)-N,N-(di-4-hexyloxyphenyl)-anilin (Beispiel 25d) wurden 102 g (68 % d. Th.) des Thiols erhalten. ¹H-NMR (400 MHz, CDCl₃): δ = 7,02 (mc, 6H), 6,88 (mc, 2H), 6,81 (mc, 4H), 3,92 (t, 4H), 2,67 (mc, 2H), 2,56 (mc, 2H), 1,90 (mc, 2H), 1,78 (mc, 4H), 1,47 (mc,4H), 1,35 (mc, 8H), 0,92 ppm (t, 6H).

### Beispiel 26: Herstellung von 4-(3-Aminopropyl)-N,N-di-(4-tolyl)-anilin

### a) 4-[N,N-(Di-4-tolyl)-amino]-zimtsäureethylester

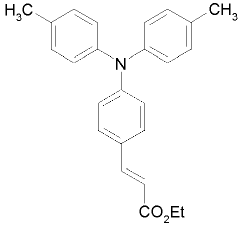

Die Herstellung erfolgte entsprechend der von 4-[N,N-(Di-4-n-hexylphenyl)-amino]-zimtsäurenitril (Beispiel 10c). Aus 104,3 g (0,347 mol) 4-[N,N-(Di-4-tolyl)-amino]-benzaldehyd wurden 80 g (71 %) des Zimtsäureesters erhalten.
¹H-NMR (400 MHz, CDCl₃): δ = 7,24 (d, 1 H), 7,21 (mc, 2H), 7,09 (mc, 4H), 7,01 (mc, 4H), 6,90 (mc, 2H), 5,61 (d, 1 H), 2,32 ppm (s, 6H).

### b) 4-(3-Aminopropyl)-N,N-di-(4-tolyl)-anilin

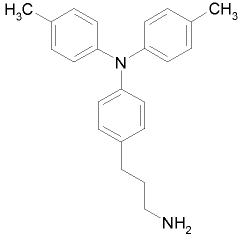

Die Herstellung erfolgte entsprechend der von 4-(3-Aminopropyl)-N,N-di-(4-n-hexylphenyl)-anilin (Beispiel 10d). Aus 100 g (0,309 mol) 4-[N,N-(Di-4-tolyl)-amino]-zimtsäureethylester (Beispiel 26a) wurden 78,7 g (81 %) des Amins erhalten. ¹H-NMR (400 MHz, CDCl₃): δ = 7,01 (mc, 6H), 6,96 (mc, 6H), 2,92 (s, 2H), 2,75 (t, 2H), 2,58 (mc, 2H), 2,27 (s, 6H), 1,79 ppm (m, 2H).

### Beispiel 27: Herstellung von 4-(3-Aminopropyl)-N,N-di-(4-methoxyphenyl)-anilin

### a) 4-[N,N-(Di-4-methoxyphenyl)-amino]-zimtsäurenitril

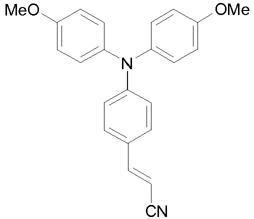

Die Herstellung erfolgte entsprechend der von 4-[N,N-(Di-4-n-hexylphenyl)-amino]-zimtsäurenitril (Beispiel 10c). Aus 170,6 g (0,386 mol) 4-[N,N-(Di-4-methoxyphenyl)-amino]-benzaldehyd wurden 150 g (84 %) des Zimtsäurenitrils erhalten.
¹H-NMR (400 MHz, CDCl₃): δ = 7,20 - 7,25 (m; 3H, arom. H, =CH-Ph), 7,07 - 7,10 (m; 4H, arom. H), 6,87 (m; 4H, arom. H), 6,80 - 6,85 (m; 2H, arom. H), 5,60 (d; 1 H, NC-CH=), 3,81 ppm (s, 6H, -OCH₃).

### b) 4-(3-Aminopropyl)-N,N-di-(4-methoxyphenyl)-anilin

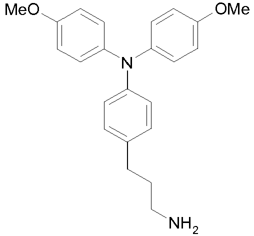

Die Herstellung erfolgte entsprechend der von 4-(3-Aminopropyl)-N,N-di-(4-n-hexylphenyl)-anilin (Beispiel 10d). Aus 70 g (0,196 mol) 4-[N,N-(Di-4-methoxyphenyl)-amino]-zimtsäurenitril (Beispiel 27a) wurden 43,7 g (60 %) des Amins erhalten. ¹H-NMR (400 MHz, CDCl₃): δ = 6,95 - 7,00 (m, 6H), 6,86 (mc, 2H), 6,75 - 6,84 (m, 6H), 3,76 (s, 6H), 2,81 (t, 2H), 2,6 - 2,8 (breit, 2H), 2,58 (mc, 2H), 1,84 ppm (mc, 2H).

### Beispiel 28: Herstellung von N,N-(Di-4-hexyloxyphenyl)-p-phenylendiamin

### a) Di-(4-hexyloxyphenyl)-amin

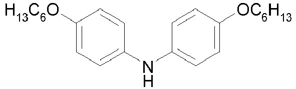

Eine Lösung von 118 g (0,61 mol) 4-Hexyloxyanilin, 130 g (0,51 mol) 4-Bromhexyloxybenzol, 146,9 (1,52 mol) Natrium-tert.-butylat, 13,96 g (0,015 mol) Di-palladium-tris(dibenzylidenaceton) und 8,46 g (0,015 mol) 1,1'-Bis(diphenylphosphino)-ferrocen in 780 ml Toluol wurden 16 Stunden bei 90 °C erhitzt. Nach dem Abkühlen auf Raumtemperatur wurde die Lösung mit Methylenchlorid versetzt und anschließend filtriert. Das Filtrat wurde bei vermindertem Druck eingeengt, in Methylenchlorid aufgenommen und an Kieselgel mit Petrolether als Laufmittel chromatographiert. Es wurden 120 g (64 % d. Th.) Amin erhalten.
¹H-NMR (400 MHz, D₆-DMSO): δ = 7,44 (s, 1 H), 6,86 (mc, 4H), 6,76 (mc, 4H), 3,83 (t, 4H), 1,63 (mc, 4H), 1,37 (mc, 4H), 1,26 (mc, 8H), 0,85 ppm (t, 6H).

### b) N,N-(Di-4-hexyloxyphenyl)-4-nitro-anilin

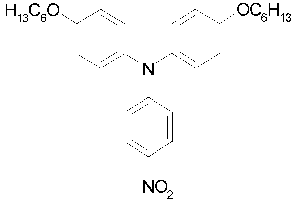

Zu einer Lösung von 177,7 g (0,48 mol) Di-(4-hexyloxyphenyl)-amin und 161,3 g (1,44 mol) Kalium-tert.-butylat in 1300 ml Dimethylformamid wurden bei 0 °C 169,5 g (1,20 mol) 1-Fluor-4-nitrobenzol getropft. Die Reaktionsmischung wurde auf Raumtemperatur erwärmt und über Nacht gerührt. Nachdem das Lösungsmittel bei vermindertem Druck entfernt worden war, wurde der Rückstand mit Methylenchlorid aufgenommen und an Kieselgel mit Petrolether als Laufmittel chromatographiert. Es wurden 150 g (64 % d. Th.) Amin erhalten.
¹H-NMR (400 MHz, CDCl₃): δ = 7,91 (mc, 2H), 7,04 (mc, 4H), 6,82 (mc, 4H), 6,70 (mc, 2H), 3,88 (t, 4H), 1,71 (mc, 4H), 1,39 (mc, 4H), 1,29 (mc, 8H), 0,84 ppm (t, 6H).

### c) N,N-(Di-4-hexyloxyphenyl)-p-phenylendiamin

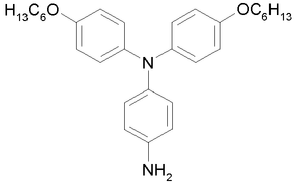

Zu einer Lösung von 150 g (0,306 mol) N,N-(Di-4-hexyloxyphenyl)-4-nitro-anilin in 750 ml Tetrahydrofuran wurde Palladium/Kohle (10 Gew.-%) gegeben. Die Reaktionsmischung wurde in einer Wasserstoff-Atmosphäre über Nacht gerührt. Anschließend wurde das Lösungsmittel bei vermindertem Druck entfernt. Es wurden 130 g (92 % d. Th.) Amin erhalten.
¹H-NMR (400 MHz, D₆-DMSO): δ = 6,75 (mc, 8H), 6,69 (mc, 2H), 6,50 (mc, 2H), 4,89 (s, 2H), 3,85 (t, 4H), 1,63 - 1,66 (m, 4H), 1,34 - 1,39 (m, 4H), 1,17 - 1,29 (m, 8H), 0,85 ppm (t, 6H).

### Beispiel 29: Herstellung von N,N'-Bis[4-(N,N-di-t-octylphenyl-amino)-phenyl]-perylen-bis(dicarboximid)

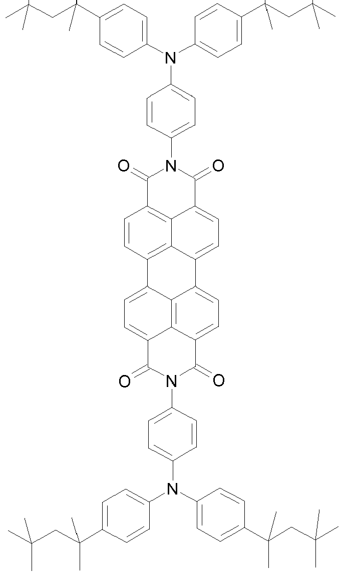

1,18 g (3,00 mmol) Perylentetracarbonsäuredianhydrid, 3,64 g (7,50 mmol) N,N-Bis[4-(1,1,3,3-tetramethyl-butyl)-phenyl]-p-phenylendiamin und 0,075 g (0,038 mmol) Zinkacetat wurden in 30 ml N-Methylpyrrolidinon gegeben und 48 Stunden bei 140 °C erhitzt. Nach dem Abkühlen auf Raumtemperatur wurden 60 ml Methanol zugegeben. Der Niederschlag wurde abgesaugt, mit Methanol und Wasser gewaschen und getrocknet (3,87 g). Das Rohprodukt wurde an Kieselgel (60 Å, 60 - 200 µm) mit Toluol / Essigester (15 : 2) chromatographisch gereinigt. Anschließend wurde die Substanz in 150 ml tert.-Amylalkohol umkristallisiert. Es wurden 1,00 g (33 % d. Th.) Feststoff mit einem Schmp. von 364 °C erhalten.
UV/Vis (Methylenchlorid): λₘₐₓ (ε) = 526 nm (98190)

| | |
|---|---|
| C₉₂H₁₀₀N₄O₄ (M = 1325,65 g/mol): | Ber. C 83,34 H 7,60 N 4,23 O 4,83 |
| | Gef. C 82,8 H 7,8 N 4,2 O 5,2 |

Beispiel 30: Herstellung von N,N'-Bis{3-[4-(N,N-di-n-hexyloxyphenyl-amino)-phenyl]-propyl}-perylen-bis(dicarboximid)

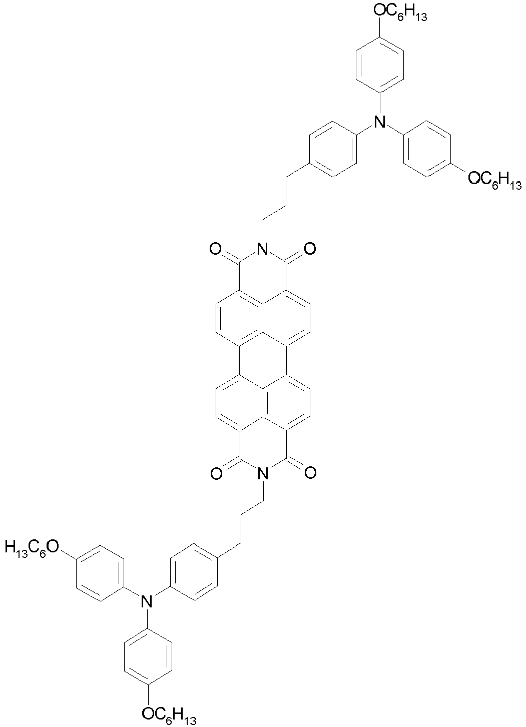

1,18 g (3,00 mmol) Perylentetracarbonsäuredianhydrid und 3,17 g (6,30 mmol) 4-(3-Aminopropyl)-N,N-di-(4-n-hexyloxyphenyl)-anilin wurden in 30 ml N-Methylpyrrolidinon suspendiert bzw. gelöst und 5 Stunden bei 120 °C erhitzt. Nach dem Abkühlen auf Raumtemperatur wurden 60 ml Methanol zugegeben. Der Niederschlag wurde abgesaugt, mit Methanol und Wasser gewaschen und getrocknet (4,00 g). Das Rohprodukt wurde an Kieselgel (60 Å, 60 - 200 µm) mit Cyclohexan-Essigester-n-Propanol (14 : 3 : 3) chromatographisch gereinigt. Es wurden 2,11 g (53 % d. Th.) dunkelroter Feststoff mit einem Schmp. von 221 - 223 °C erhalten.
UV/Vis (Methylenchlorid): λₘₐₓ (ε) = 524 nm (74930)

| | |
|---|---|
| C₉₀H₉₆N₄O₈ (M = 1361,79 g/mol): | Ber. C 79,38 H 7,11 N 4,11 O 9,40 |
| | Gef. C 79,4 H 7,3 N 4,0 O 9,2 |

### Beispiel 31: Herstellung von N,N'-Bis{3-[4-(N,N-di-n-hexylphenyl-amino)-phenyl]-propyl}-1,7-dicyano-perylen-bis(dicarboximid)

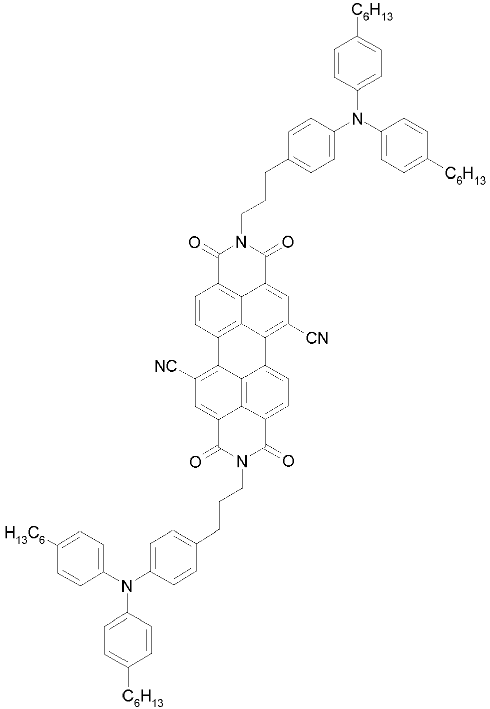

Eine Lösung von 2,00 g (1,37 mmol) N,N'-Bis{3-[4-(N,N-di-n-hexylphenyl-amino)-phenyl]-propyl}-1,7-dibrom-perylen-bis(dicarboximid) in 50 ml N-Methylpyrrolidinon wurde mit 0,37 g (4,1 mmol) Kupfercyanid versetzt und auf 150 °C erhitzt. Nach zweistündigem Rühren bei dieser Temperatur ließ man die Reaktionsmischung auf Raumtemperatur abkühlen. Nach der Zugabe von 50 ml Methanol wurde die Reaktionsmischung kurz gerührt und dann filtriert. Der Rückstand wurde mit 30 ml Methanol gewaschen, dann mit 10 ml gesättigter Natriumhydrogencarbonatlösung aufgeschlämmt, abgesaugt, mit Wasser und dann mit Methanol gewaschen und getrocknet (1,81 g). Das Rohprodukt wurde in Methylenchlorid gelöst, auf 10 g Kieselgel (60 Å, 60 - 200 µm) aufgezogen und an Kieselgel mit Toluol-Essigester chromatographisch gereinigt. Es wurden 1,69 g dunkelrotes Harz erhalten, das in 20 ml Nitroethan unter Rückfluss-temperatur gelöst wurde und beim Abkühlen auskristallisierte. Der Feststoff wurde abgesaugt und aus 100 ml Methylethylketon umkristallisiert. Es wurden 0,71 g (38 % d. Th.) dunkelrote Mikrokristalle erhalten, die bei 204 - 205 °C schmolzen. Laut ¹H-NMR enthielt die Substanz als Nebenprodukt das 1,6-Dicyano-Isomer.
UV/Vis (Methylenchlorid): λₘₐₓ (ε) = 524 nm (61700)

| | |
|---|---|
| C₉₂H₉₄N₆O₄ (M = 1347,81 g/mol): | Ber. C 81,99 H 7,03 N 6,24 O 4,75 |
| | Gef. C 81,9 H 6,9 N 6,1 O 4,8 |

### Beispiel 32: Herstellung von N,N'-Bis{3-[4-(N,N-di-n-hexyloxyphenyl-amino)-phenyl]-propyl}-1,7-dicyano-perylen-bis(dicarboximid)

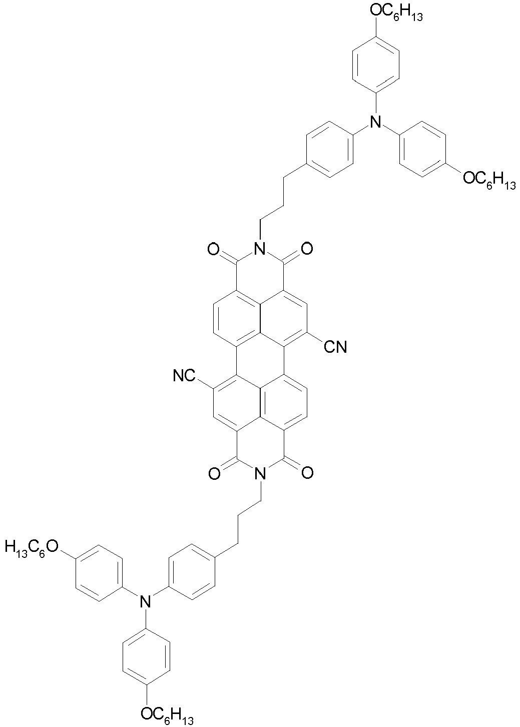

Eine Lösung von 2,00 g (1,32 mmol) N,N'-Bis{3-[4-(N,N-di-n-hexyloxyphenyl-amino)-phenyl]-propyl}-1,7-dibrom-perylen-bis(dicarboximid) in 20 ml N-Methylpyrrolidinon wurde mit 0,35 g (4,0 mmol) Kupfercyanid versetzt und auf 150 °C erhitzt. Nach zweistündigem Rühren bei dieser Temperatur ließ man die Reaktionsmischung auf Raumtemperatur abkühlen. Nach der Zugabe von 50 ml Methanol wurde die Reaktionsmischung kurz gerührt und dann filtriert. Der Rückstand wurde mit 20 ml gesättigter Natriumhydrogencarbonatlösung aufgeschlämmt, abgesaugt, mit Wasser und dann mit Methanol gewaschen und getrocknet (1,78 g). Das Rohprodukt wurde an Aluminium-oxid (aktiviert, neutral, Brockmann Grade I, 58 Å) mit Methylenchlorid-n-Heptan 5 : 1 chromatographiert. Das vorgereinigte Produkt wurde an Kieselgel (60 Å, 60 -200 µm) mit Toluol-n-Heptan-Aceton 6 : 1 : 0,1 chromatographisch gereinigt. Es wurden 1,06 g (57 % d. Th.) dunkelrote Mikrokristalle erhalten, die bei 206 - 207 °C schmolzen. Laut ¹H-NMR enthielt die Substanz als Nebenprodukt das 1,6-Dicyano-Isomer.
UV/Vis (Methylenchlorid): λₘₐₓ (ε) = 524 nm (60680)

| | |
|---|---|
| C₉₂H₉₄N₆O₈ (M = 1411,81 g/mol): | Ber. C 78,27 H 6,71 N 5,95 O 9,07 |
| | Gef. C 78,3 H 7,0 N 5,8 O 9,0 |

### Beispiel 33: Herstellung von N,N'-Bis(2-dibutylamino-ethyl)-1,7-dibrom-perylen-3,4:9,10-bis(dicarboximid)

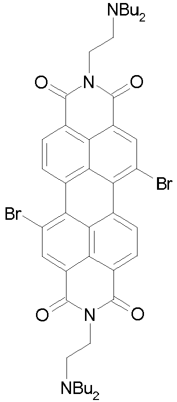

Eine Suspension von 3,60 g (6,54 mmol) Dibromperylen-3,4:9,10-bis(dicarbonsäureanhydrid) (ca. 80 % 1,7-Dibrom- und ca. 20 % 1,6-Dibrom-Isomer) und 2,40 g (13,9 mmol) 2-Dibutylamino-ethylamin in 60 ml Chlorbenzol wurde auf 80 °C erhitzt und 4 Stunden bei dieser Temperatur gerührt. Nach dem Abkühlen auf Raumtemperatur wurde Feststoff abgesaugt, mit 50 ml Methanol aufgenommen und 3 Stunden gerührt. Der Feststoff wurde abgesaugt, mit 30 ml Methanol gewaschen und getrocknet. Das Rohprodukt (4,98 g) wurde in Dimethylformamid umkristallisiert. Der Niederschlag wurde abfiltriert, mit Methanol gewaschen und getrocknet. Es wurden 4,55 g (81 % d. Th.) rotes Pulver erhalten, das bei 194 °C schmolz.
UV/Vis (Methylenchlorid): λₘₐₓ (ε) = 524 nm (49320)

| | |
|---|---|
| C₄₄H₅₀Br₂N₄O₄ (M = 858,72 g/mol): | Ber. C 61,54 H 5,87 Br 18,61 N 6,52 O 7,45 |
| | Gef. C 61,2 H 5,9 Br 18,6 N 6,5 O 7,8 |

### Beispiel 34: Herstellung von N,N'-Bis(2-dibutylamino-propyl)-1,7-dibrom-perylen-3,4:9,10-bis(dicarboximid)

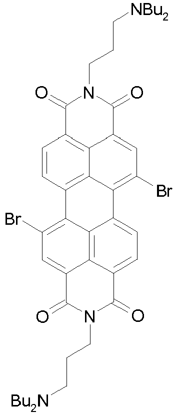

Eine Suspension von 3,50 g (6,36 mmol) Dibromperylen-3,4:9,10-bis(dicarbonsäureanhydrid) (ca. 80 % 1,7-Dibrom- und ca. 20 % 1,6-Dibrom-Isomer) und 2,49 g (13,4 mmol) 3-Dibutylamino-propylamin in 60 ml Chlorbenzol wurde auf 80 °C erhitzt und 5,5 Stunden bei dieser Temperatur gerührt. Nach dem Abkühlen auf Raumtemperatur wurde Feststoff abgesaugt, mit 40 ml Methanol aufgenommen und 3 Stunden gerührt. Der Feststoff wurde abgesaugt, mit 30 ml Methanol gewaschen und getrocknet. Das Rohprodukt (4,86 g) wurde in Dimethylformamid umkristallisiert. Der Niederschlag wurde abfiltriert, mit Methanol gewaschen und getrocknet. Es wurden 4,33 g (77 % d. Th.) rotes Pulver erhalten, das bei 179 - 180 °C schmolz.
UV/Vis (Methylenchlorid): λₘₐₓ (ε) = 524 nm (50250)

| | |
|---|---|
| C₄₆H₅₄Br₂N₄O₄ (M = 886,78 g/mol): | Ber. C 62,31 H 6,14 Br 18,02 N 6,32 O 7,22 |
| | Gef. C 62,1 H 6,1 Br 18,1 N 6,3 O 7,4 |

### Beispiel 35: Herstellung von N,N'-Bis(2-dibutylamino-propyl)-1,7-diphenoxy-perylen-3,4:9,10-bis(dicarboximid)

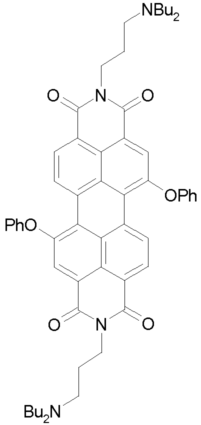

2,00 g (2,26 mmol) N,N'-Bis(2-dibutylamino-propyl)-1,7-dibrom-perylen-3,4:9,10-bis(dicarboximid), 0,43 g (4,5 mmol) Phenol und 0,62 g (4,5 mmol) Kaliumcarbonat wurden in 40 ml 1-Methyl-2-pyrrolidon auf 100 - 105 °C erhitzt und 6 Stunden bei dieser Temperatur gerührt. Nach dem Abkühlen wurden 50 ml Methanol zugegeben und 30 min nachgerührt. Der Niederschlag wurde abgesaugt, mit Methanol gewaschen und getrocknet. Das Rohprodukt (1,84 g) wurde an Kieselgel (60 Å, 60 - 200 µm) mit Methylenchlorid-Methanol-Aceton 6 : 1 : 0,02 chromatographisch gereinigt. Es wurden 1,34 g (65 % d. Th.) dunkelroter Feststoff erhalten.
UV/Vis (Methylenchlorid): λₘₐₓ (ε) = 536 nm (49160)

| | |
|---|---|
| C₅₈H₆₄N₄O₆ (M = 913,18 g/mol): | Ber. C 76,29 H 7,06 N 6,14 O 10,51 |
| | Gef. C 75,7 H 7,1 N 6,3 O 10,9 |

### Beispiel 36: Herstellung von N,N'-Bis(2,6-diisopropylphenyl)-1,7-bis{3-[4-(bis-{4-n-hexylphenyl}-amino)-phenyl]-propanthio}-perylen-3,4:9,10-bis(dicarboximid)

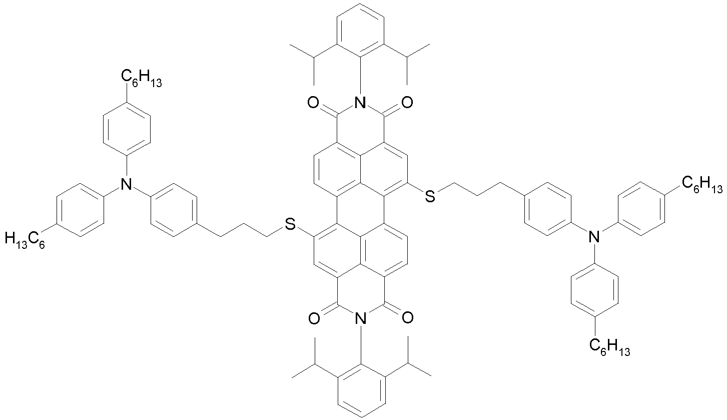

2,00 g (2,30 mmol) N,N'-Bis(2,6-diisopropylphenyl)-1,7(6)-dibromperylen-3,4:9,10-bis(dicarboximid), 2,58 g (5,30 mmol) 3-[N,N-(Di-4-hexylphenyl)-4-anilino]-propanthiol und 0,74 g (5,3 mmol) Kaliumcarbonat wurden in 30 ml N-Methyl-2-pyrrolidon 7,5 Stunden bei 80 °C gerührt. Nach dem Abkühlen auf Raumtemperatur wurden 50 ml Methanol und Wasser zugegeben. Der Niederschlag wurde abfiltriert, Methanol und Wasser gewaschen und getrocknet. Das Rohprodukt (2,95 g) wurde an Kieselgel (60 Å, 60 - 200 µm) chromatographisch gereinigt. Die 1. Fraktion (1,34 g) wurde in 120 ml Isopropanol umkristallisiert. Es wurden 0,53 g (14 % d. Th.) dunkelrote Mikrokristalle erhalten.
UV/Vis (Methylenchlorid): λₘₐₓ (ε) = 570 nm (31460)

| | |
|---|---|
| C₁₁₄H₁₃₈N₄O₄Sₛ (M = 1682,44 g/mol): | Ber. C 81,39 H 7,67 N 3,33 O 3,80 S 3,81 |
| | Gef. C 81,5 H 7,6 N 3,3 O 3,8 S 3,7 |

### Beispiel 37: Herstellung von N,N'-Bis(2,6-diisopropylphenyl)-1,7-bis{3-[4-(bis-{4-n-hexylphenyl}-amino)-phenyl]-propansulfonyl}-perylen-3,4:9,10-bis(dicarboximid)

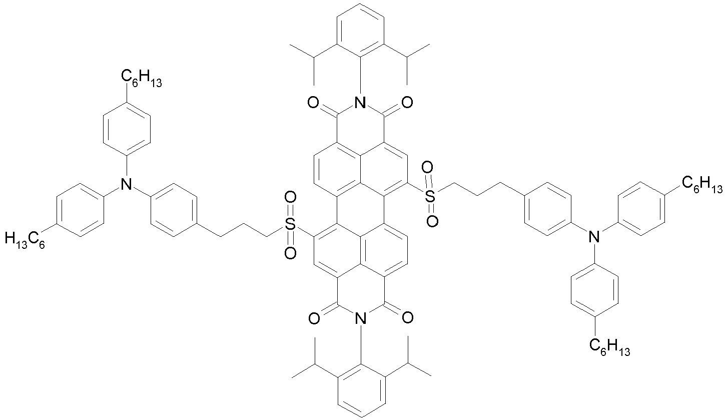

Zu einer Lösung von 1,00 g (0,59 mmol) N,N'-Bis(2,6-diisopropylphenyl)-1,7-bis{3-[4-(bis-{4-n-hexylphenyl}-amino)-phenyl]-propanthio}-perylen-3,4:9,10-bis(dicarboximid) in 20 ml Methylenchlorid wurden bei 0 °C 0,93 g (4,2 mmol) 77 %ige 3-Chlorperbenzoesäure getropft. Nach einstündigem Rühren bei 0 °C ließ man die Lösung auf Raumtemperatur erwärmen und 16 Stunden nachrühren. Nach der Zugabe von 0,18 g (1,19 mmol) Natriumiodid wurde die Lösung 5 Stunden bei Raumtemperatur gerührt. Die Reaktionslösung wurde mit Wasser versetzt. Die organische Phase wurde abgetrennt, mit Magnesiumsulfat getrocknet und bis zur Trockne eingeengt. Das Rohprodukt (1,67 g) wurde an Kieselgel (60 Å, 60 - 200 µm) mit Methylenchlorid-Isopropanol 20 : 1 chromatographisch gereinigt. Es wurden 0,50 g (49 % d. Th.) dunkelroter Feststoff erhalten.
UV/Vis (Methylenchlorid): λₘₐₓ (ε) = 526 nm (32320)

| | |
|---|---|
| C₁₁₄H₁₂₈N₄O₈Sₛ (M = 1746,44 g/mol): | Ber. C 78,40 H 7,39 N 3,21 O 7,33 S 3,67 |
| | Gef. C 78,3 H 7,6 N 3,2 O 6,6 S 3,4 |

### Beispiel 38: Herstellung von N,N'-Bis{3-[4-(N,N-di-n-hexyloxyphenyl-amino)-phenyl]-propyl}-naphthalin-1,8:4,5-bis(dicarboximid)

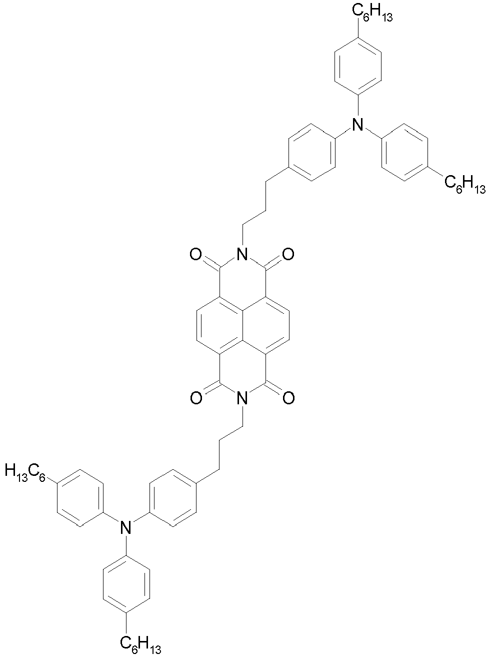

Eine Lösung von 1,05 g (3,73 mmol) 95 %iges 1,4,5,8-Naphthalintetracarbonsäure-dianhydrid und 4,14 g (7,83 mmol) 4-(3-Aminopropyl)-N,N-di-(4-n-hexyloxyphenyl)-anilin (siehe Beispiel 12d)) in 50 ml Toluol wurden 10 Stunden bei 80 °C gerührt. Nach dem Abkühlen auf Raumtemperatur wurde das Reaktionsgemisch bis zur Trockne eingeengt (grünblaues Öl). Das Rohprodukt (6,5 g) wurde zweimal an Kieselgel (60 Å, 60 - 200 µm) mit Methylenchlorid / n-Heptan (5 : 1) als Laufmittel chromatographisch gereinigt. Es wurden 2,1 g (45 % d. Th.) hellblauer Feststoff erhalten, der bei 155 °C schmolz.
UV/Vis (Methylenchlorid): λₘₐₓ (ε) = 380 nm (29030)

| | |
|---|---|
| C₈₀H₉₂N₄O₆ (M = 1237,65 g/mol): | Ber. C 77,64 H 7,49 N 4,53 O 10,34 |
| | Gef. C 77,7 H 7,4 N 4,6 O 10,7 |

### Beispiel 39 (Herstellung vgl. Bonnet et al., Synthetic Metals 156 (2006) 1292 - 1298):

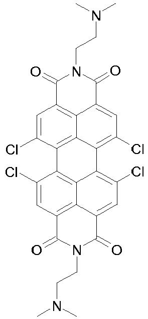

N,N'-Bis(3-dimethylaminoethyl)-1,6,7,12-tetrachlorperylentetracarbonsäurediimid wurde in NMP in einer Konzentration von 2,97*10⁻³ mol/l gelöst. Der molare Extintktionskoeffizient der Verbindung in NMP betrug 36930 (l/mol*cm). Ein Teil dieser Lösung wurde über einen Lichtleiter mit einer 75 Watt Xe-Lampe so belichtet, dass das gesamte Volumen der Lösung von dem Lichtstrahl erfasst wurde. Gleichzeitig mit der Belichtung wurde mit einem UV-VIS Spektrometer im Transmissionsmodus die spektrale Veränderung gemessen.

Es entsteht eine Bande bei 756 nm, deren Intensität innerhalb von 6 Sekunden Belichtungszeit um 2,7 Extinktionseinheiten zunimmt.

| Zeit (Sec) | **Zunahme bei 756nm** |
|---|---|
| 0 | 0 |
| 2 | 1.0555538 |
| 4 | 2.7220225 |
| 6 | 2.7336044 |

### Beispiel 40:

N,N'-Bis(3-dimethylaminoethyl)-1,6,7,12-tetrachlorperylentetracarbonsäurediimid wurde mit einer Schichtdicke von 130 nm auf Glas aufgedampft. Die Farbstoffschicht wurde über einen Lichtleiter mit einer 75 Watt Xe-Lampe belichtet. Bei Belichtung entsteht eine Bande bei 765 nm.

| Zeit (Min) | **765 nm** |
|---|---|
| **0** | 0 |
| **2** | 0.0387932 |

Die bei Belichtung entstehende Absorption bei 765 nm bildet sich nach 15 Minuten Dunkellagerung auf den Startwert zurück.

| **Dunkellagerung (Min)** | **765 nm** |
|---|---|
| 0 Min dunkel | 0.0387932 |
| 5 Min dunkel | 0.0186532 |
| 10 Min dunkel | 0.0106751 |
| 15 Min dunkel | 0.0065114 |

N,N'-Bis[3-(3,6-di-n-hexyl-N-carbazolyl)-propyl]-perylentetracarbonsäurediimid wurde in NMP mit einer Konzentration von 8,77*10⁻⁴ mol/l gelöst. Der molare Extintktionskoeffizient der Verbindung in NMP beträgt 74100 (l/mol*cm). Ein Teil dieser Lösung wurde in einer 1 mm Küvette mit einer 75 Watt Xe-Lampe so belichtet, dass das gesamte Volumen der Lösung von dem auftreffenden Lichtbündel erfasst wurde. Gleichzeitig mit der Belichtung wurde mit einem UV-VIS Spektrometer im Transmissionsmodus die spektrale Veränderung über eine Ulbrichtkugel gemessen.

Es entsteht eine Bande bei 706 nm die innerhalb von 2 Minuten Belichtung auf >80% ihres Wertes bei unendlicher Belichtungszeit ansteigt und nach anschließender Lagerung im Dunkeln um die Hälfte zurückgeht (Erholung).

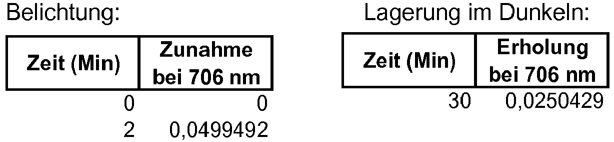

### Beispiel 42:

Durchführung einer Papierfärbung mit N,N'-Bis(3-dimethylaminoethyl)-1,6,7,12-tetrachlorperylentetracarbonsäurediimid:
Die Papiermasse (holzfreier Cellulosepulp) wurde eingewogen. Die feuchte Papiermasse, entsprechend 1,7 g Trockenmasse, wurde in 65 ml Wasser gegeben und die Masse 10-15 min gerührt. 0,1g N,N'-Bis(3-dimethylaminoethyl)-1,6,7,12-tetrachlorperylentetracarbonsäurediimid wurden in Wasser und 2 ml Essigsäure gelöst und auf 100 ml aufgefüllt. 10ml dieser Lösung wurden zur vorbereiteten Papiermasse gegeben, und der Ansatz auf 1 Liter mit Wasser aufgefüllt. Der Ansatz wurde über einen Filter abgesaugt. Die Papiermasse wurde vom Filter gelöst und zwischen zwei Filterpapieren 5 Min. bei 90 °C getrocknet.

Die getrocknete Probe wird mit einer 75 Watt Xenonlampe über einen Y-Lichtleiter belichtet und die entstehende UV-Absorption gleichzeitig mit der Belichtung mit einem UV-VIS Spektrometer gemessen.

Die bei Belichtung entstehende Absorption bei 768 nm bildet sich nach 30 Minuten Dunkellagerung um 86% (des nach zwei Minuten Belichtung entstandenen Wertes) zurück.

| Zeit (Min) | **768 nm** | | **768 nm** |
|---|---|---|---|
| **0** | 0 | **Nach 30 Min** | 0.0538442 |
| **2** | 0.3915774 | **Dunkellagerung** | |

### Beispiel 43:

### Durchführung von "Tropfversuchen" und anschließende Belichtung auf Papier

0,1g N,N'-Bis(3-dimethylaminoethyl)-1,6,7,12-tetrachlorperylentetracarbonsäurediimid wurden in Wasser und 2 ml Essigsäure gelöst und auf 100 ml aufgefüllt. Mit einer Pipette wurden 2 ml Lösung aufgenommen. Diese Lösung wurde auf ein Blatt Papier aufgetropft und bei 90°C getrocknet.

Die getrocknete Probe wurde mit einer 75 Watt Xenonlampe über einen Y-Lichtleiter belichtet und die entstehende UV-Absorption gleichzeitig mit der Belichtung mit einem UV-VIS Spektrometer gemessen.

Die bei Belichtung entstehende Absorption bei 768 nm bildet sich nach 30 Minuten Dunkellagerung auf ca. 11 % des Ausgangswertes zurück.

| | **E max bei 770 nm** |
|---|---|
| Start Belichtung | **0** |
| 38 Sec. Belichtung | **0.4982** |
| 30 Min dunkel | **0.0526** |

## Patentansprüche

1. Verfahren zur Anderung der Absorption elektromagnetischer Strahlung durch eine oder mehrere Verbindungen der allgemeinen Formeln (I) 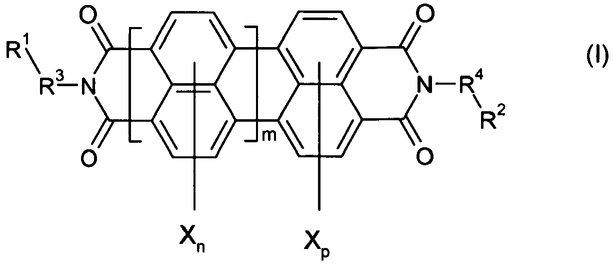 **dadurch gekennzeichnet, dass**
diese Verbindungen mit elektromagnetischer Strahlung
der Wellenlänge von 300 bis 750 nm bestrahlt werden und
durch die Bestrahlung mit elektromagnetischer Strahlung der Wellenlänge von 300 bis 750 nm eine bathochrome Verschiebung des Absorptionsspektrums der Verbindungen der allgemeinen Formel (I) resultiert
wobei die Symbole und Indizes folgende Bedeutung haben
R¹, R² unabhängig voneinander, gleich oder verschieden, Amino, C₁-C₈-Alkylamino, C₁-C₈-Dialkylamino, Arylamino, Diarylamino, N-Heterocyclyl, Triarylaminyl, Hydroxy, C₁-C₈-Alkoxy, Aryloxy,
R³, R⁴ unabhängig voneinander, gleich oder verschieden, Einfachbindung, C₁-C₈-Alkylen, C₃-C₆-Cycloalkylen, Arylen, C₈-C₁₄-Phenylalkylen,
X unabhängig voneinander, gleich oder verschieden, C₁-C₂₀-Alkoxy, C₁-C₂₀-Alkylthio, Aryloxy, Arylthio, Halogen, Cyan, CO₂R, SO₃R, SO₂R, oder eine Gruppe 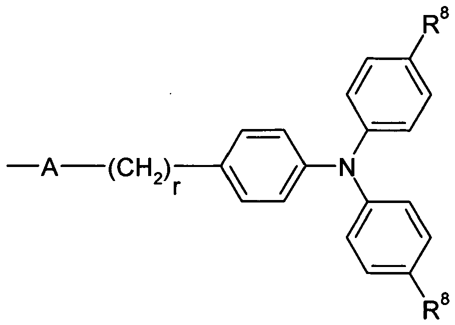 mit
A S, SO₂,
r 2, 3, 4, 5, 6,
R⁸ C₁-C₂₀-Alkyl, C₁-C₂₀-Alkoxy,
und
falls m=1 können verschiedene X gemeinsam eine Thiogruppe darstellen,
R unabhängig voneinander, gleich oder verschieden, H, C₁-C₈-Alkyl, Aryl,
n, p 0, 1, 2, 3 oder 4,
m 0, 1, 2 oder 3,
und wobei die Substituenten R, R¹, R², R³ oder R⁴ jeweils an beliebiger Position durch ein oder mehrere, gegebenenfalls durch H abgesättigte, Heteroatome unterbrochen sein können, wobei die Anzahl dieser Heteroatome nicht mehr als vier beträgt, und/oder jeweils an beliebiger Position, allerdings nicht mehr als fünfmal durch NR⁵R⁶, CONR⁵R⁶, COOM, COOR⁵, SO₃M, SO₃R⁵, wobei,
R⁵, R⁶ unabhängig voneinander, gleich oder verschieden, H, C₁-C₈-Alkyl, Aryl,
M H, Alkalimetall, NR⁷₄,
R⁷ unabhängig voneinander H, C₁-C₈-Alkyl,
CN, NO₂, C₁-C₂₀-Alkyl, C₁-C₂₀-Alkoxy, Aryl, Aryloxy, Heterocyclen, Heteroatome oder Halogen substituiert sein können, wobei diese ebenfalls maximal zweimal mit den genannten Gruppen substituiert sein können.

2. Verfahren nach Anspruch 1, wobei die Symbole und Indizes folgende Bedeutung haben,
R¹, R² unabhängig voneinander, gleich oder verschieden, Amino, C₁-C₈-Alkylamino, C₁-C₈-Dialkylamino, Arylamino, Diarylamino, N-Heterocyclyl, N,N-Diarylanilin-4-yl,
R³, R⁴ unabhängig voneinander, gleich oder verschieden, Einfachbindung, C₁-C₈-Alkylen,
X unabhängig voneinander, gleich oder verschieden, C₁-C₂₀-Alkoxy, Aryloxy, Halogen, Cyan, SO₂R, oder eine Gruppe 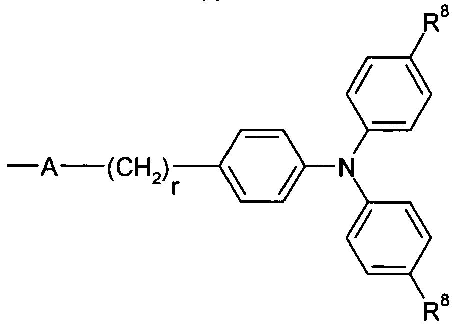 mit
A S, SO₂,
r 2, 3, 4, 5, 6,
R⁸ C₁-C₂₀-Alkyl, C₁-C₂₀-Alkoxy,
m 1 oder 2,
und wobei die Substituenten R¹, R², R³ oder R⁴ jeweils an beliebiger Position gemäß Anspruch 1 substituiert oder durch Heteroatome unterbrochen sein können
und alle anderen Symbole und Indizes die Bedeutung gemäß Anspruch 1 haben.

3. Verfahren nach Anspruch 1, wobei die Symbole und Indizes folgende Bedeutung haben,
R¹, R² beide gleich Amino, C₁-C₈-Alkylamino, C₁-C₈-Dialkylamino, Arylamino, Diarylamino, N-Heterocyclyl, N,N-Diarylanilin-4-yl,
R3, R⁴ unabhängig voneinander, gleich oder verschieden, Einfachbindung, C₁-C₈-Alkylen,
X unabhängig voneinander, gleich oder verschieden, Halogen, Cyan, Aryloxy,
oder eine Gruppe 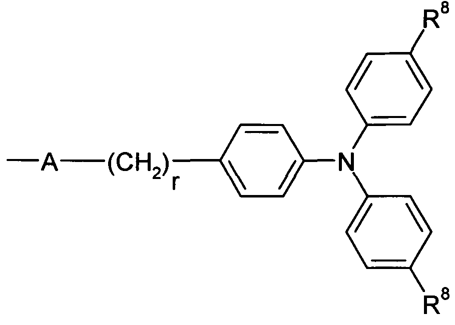 mit
A S, SO₂,
r 2, 3, 4, 5, 6,
R⁸ C₁-C₂₀-Alkyl, C₁-C₂₀-Alkoxy,
m 1 oder 2,
und wobei die Substituenten R¹, R², R³ oder R⁴ jeweils an beliebiger Position gemäß Anspruch 1 substituiert oder durch Heteroatome unterbrochen sein können
und alle anderen Symbole und Indizes die Bedeutung gemäß Anspruch 1 haben.

4. Verfahren nach den Ansprüchen 1 bis 3, **dadurch gekennzeichnet, dass** durch die Bestrahlung mit elektromagnetischer Strahlung der Wellenlänge von 300 bis 750 nm eine visuell wahrnehmbare Farbänderung durch die Verbindungen der allgemeinen Formel (I) resultiert.

5. Verfahren nach den Ansprüchen 1 bis 3, **dadurch gekennzeichnet, dass** durch die Bestrahlung durch elektromagnetische Strahlung der Wellenlänge von 300 bis 750 nm eine Absorption durch die Verbindungen der allgemeinen Formel (I) im IR resultiert.

6. Verwendung des Verfahrens gemäß Anspruch 1 zum Nachweis der Markierung von Materialien.

7. Verwendung nach Anspruch 6, zum Nachweis der Markierung von Papier.

8. Verwendung nach Anspruch 6, zum Nachweis der Markierung von Mineralöl.

9. Verwendung des Verfahrens gemäß Anspruch 1 zum Hervorrufen einer Farbänderung.

10. Verwendung nach Anspruch 9, zum Hervorrufen einer Farbänderung von oder auf Papier.

11. Verwendung des Verfahrens gemäß Anspruch 1 zum Laserschweißen.

12. Verwendung des Verfahrens gemäß Anspruch 1 im Wärmemanagement.

13. Verwendung des Verfahrens gemäß Anspruch 1 zur Bereitstellung von Photoinitiatoren.

14. Verwendung des Verfahrens gemäß Anspruch 1 zur Bereitstellung von Radikalfängern.

15. Verwendung des Verfahrens gemäß Anspruch 1 zum Nachweis von Sauerstoff.

16. Verfahren zur Regulierung der Absorption oder Transmission elektromagnetischer Strahlung durch ein Material, **dadurch gekennzeichnet, dass** eine oder mehrere Verbindungen der allgemeinen Formeln (I) gemäß Anspruch 1 in Kontakt mit diesem Material gebracht werden und diese Verbindungen mit elektromagnetischer Strahlung der Wellenlänge von 300 bis 750 nm bestrahlt werden
und
durch die Bestrahlung mit elektromagnetischer Strahlung der Wellenlänge von 300 bis 750 nm eine bathochrome Verschiebung des Absorptionsspektrums der Verbindungen der allgemeinen Formel (I) resultiert.

17. Verfahren nach Anspruch 16, **dadurch gekennzeichnet, dass** eine Regulierung der Absorption von IR-Strahlung durch das Material erfolgt.

18. Verfahren nach Anspruch 17, wobei das Material zum Wärmemanagement oder zur Wärmedämmung eingesetzt wird.

19. Verfahren nach Anspruch 16, **dadurch gekennzeichnet, dass** eine Regulierung der Absorption von sichtbarem Licht durch das Material erfolgt.

20. Verfahren nach Anspruch 19, wobei das Material zum Helligkeitsmanagement eingesetzt wird.

21. Verfahren nach den Ansprüchen 16 bis 20, wobei das Material ein Fenster ist.

22. Verbindungen der allgemeinen Formel (I) gemäß Anspruch 1, wobei
R¹, R² unabhängig voneinander, gleich oder verschieden, C₁-C₈-Alkylamino, C₁-C₈-Dialkylamino, Arylamino, Diarylamino, N-Heterocyclyl, Triarylaminyl,
R³, R⁴ unabhängig voneinander, gleich oder verschieden, C₁-C₈-Alkylen, Arylen,
X unabhängig voneinander, gleich oder verschieden, Halogen, Cyan, oder eine Gruppe 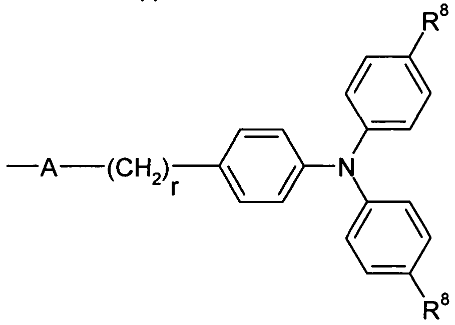 mit
A S, SO₂,
r 2, 3, 4, 5, 6,
R⁸ C₁-C₂₀-Alkyl, C₁-C₂₀-Alkoxy,
n, p 0, 1, oder 2,
m 0, oder 1,
und wobei die Substituenten R¹, R² jeweils an beliebiger Position, allerdings nicht mehr als fünfmal durch C₁-C₂₀-Alkyl, C₁-C₂₀-Alkoxy substituiert sein können.

23. Materialien in Kontakt mit Verbindungen gemäß Anspruch 22.

24. Polymere, Lacke oder Gläser enthaltend Verbindungen gemäß Anspruch 22.

## Claims

1. A process for altering the absorption of electromagnetic radiation by means of one or more compounds of the general formulae (I) 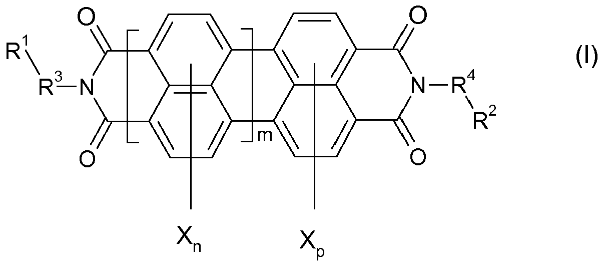 which comprises
irradiating these compounds with electromagnetic radiation of wavelength from 300 to 750 nm and
the irradiation with electromagnetic radiation of wavelength from 300 to 750 nm resulting in a bathochromic shift in the absorption spectrum of the compounds of the general formula (I)
where the symbols and indices are each defined as follows
R¹, R² are the same or different and are each independently amino, C₁-C₈-alkylamino, C₁-C₈-dialkylamino, arylamino, diarylamino, N-heterocyclyl, triarylaminyl, hydroxyl, C₁-C₈-alkoxy, aryloxy,
R³, R⁴ are the same or different and are each independently a single bond, C₁-C₈-alkylene, C₃-C₆-cycloalkylene, arylene, C₈-C₁₄-phenylalkylene,
X are the same or different and are each independently C₁-C₂₀-alkoxy, C₁-C₂₀-alkylthio, aryloxy, arylthio, halogen, cyano, CO₂R, SO₃R, SO₂R, or a group 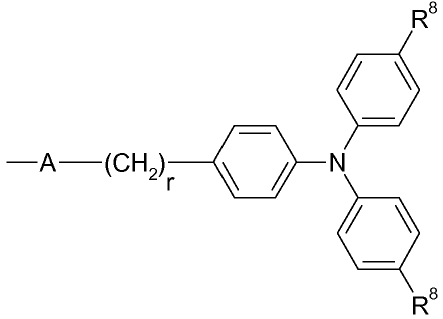 where
A is S, SO₂,
r is 2, 3, 4, 5, 6,
R⁸ is C₁-C₂₀-alkyl, C₁-C₂₀-alkoxy, and
if m=1, different X together may be a thio group,
R are the same or different and are each independently H, C₁-C₈-alkyl, aryl,
n, p is 0, 1, 2, 3 or 4,
m is 0, 1, 2 or 3,
and where the substituents R, R¹, R², R³ or R⁴ may each be interrupted at any position by one or more heteroatoms with valences saturated if appropriate by H, where the number of these heteroatoms is not more than four, and/or may be substituted in each case at any position, but not more than five times, by NR⁵R⁶, CONR⁵R⁶, COOM, COOR⁵, SO₃M, SO₃R⁵, where
R⁵, R⁶ are the same or different and are each independently H, C₁-C₈-alkyl, aryl,
M is H, alkali metal, NR⁷₄,
R⁷ is independently H, C₁-C₈-alkyl,
CN, NO₂, C₁-C₂₀-alkyl, C₁-C₂₀-alkoxy, aryl, aryloxy, heterocycles, heteroatoms
or halogen, where these may likewise be substituted not more than twice by the groups mentioned.

2. The process according to claim 1, where the symbols and indices are each defined as follows:
R¹, R² are the same or different and are each independently amino,
C₁-C₈-alkylamino, C₁-C₈-dialkylamino, arylamino,
diarylamino, N-heterocyclyl, N,Ndiarylanilin-4-yl,
R³, R⁴ are the same or different and are each independently a single bond, C₁-C₈-alkylene,
X are the same or different and are each independently
C₁-C₂₀-alkoxy, aryloxy, halogen, cyano, SO₂R,
or a group 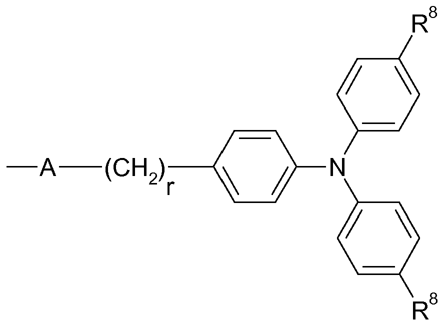 where
A is S, SO₂,
r is 2, 3, 4, 5, 6,
R⁸ is C₁-C₂₀-alkyl, C₁-C₂₀-alkoxy,
m is 1 or 2,
and where the substituents R¹, R², R³ and R⁴ may each be substituted at any position according to claim 1 or interrupted by heteroatoms,
and all other symbols and indices are as defined in claim 1.

3. The process according to claim 1, where the symbols and indices are each defined as follows:
R¹, R² are both amino, C₁-C₈-alkylamino, C₁-C₈-dialkylamino, arylamino, diarylamino, N-heterocyclyl, N,N-diarylanilin-4-yl,
R³, R⁴ are the same or different and are each independently a single bond, C₁-C₈-alkylene,
X are the same or different and are each independently halogen, cyano, aryloxy, or a group 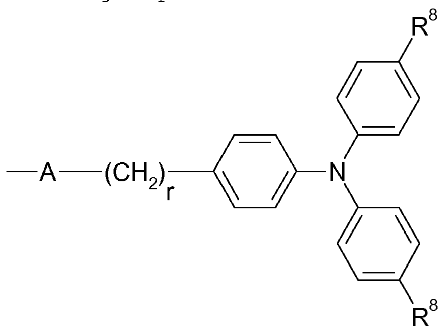 where
A is S, SO₂,
r is 2, 3, 4, 5, 6,
R⁸ is C₁-C₂₀₋alkyl, C₁-C₂₀-alkoxy,
m is 1 or 2,
and where the substituents R¹, R², R³ and R⁴ may each be substituted at any position according to claim 1 or interrupted by heteroatoms,
and all other symbols and indices are as defined in claim 1.

4. The process according to claims 1 to 3, wherein the irradiation with electromagnetic radiation of wavelength from 300 to 750 nm results in a visually perceptible color change by the compounds of the general formula (I).

5. The process according to claims 1 to 3, wherein the irradiation with electromagnetic radiation of wavelength from 300 to 750 nm results in an absorption by the compounds of the general formula (I) in the IR.

6. The use of the process according to claim 1 for detecting the marking of materials.

7. The use according to claim 6 for detecting the marking of paper.

8. The use according to claim 6 for detecting the marking of mineral oil.

9. The use of the process according to claim 1 for causing a color change.

10. The use according to claim 9 for causing a color change of or on paper.

11. The use of the process according to claim 1 for laser welding.

12. The use of the process according to claim 1 in heat management.

13. The use of the process according to claim 1 for providing photoinitiators.

14. The use of the process according to claim 1 for providing free-radical scavengers.

15. The use of the process according to claim 1 for detection of oxygen.

16. A process for regulating the absorption or transmission of electromagnetic radiation by a material, which comprises contacting one or more compounds of the general formula (I) according to claim 1 with this material and irradiating these compounds with electromagnetic radiation of wavelength from 300 to 750 nm and the irradiation with electromagnetic radiation of wavelength from 300 to 750 nm resulting in a bathochromic shift in the absorption spectrum of the compounds of the general formula (I).

17. The process according to claim 16, wherein the absorption of IR radiation is regulated by the material.

18. The process according to claim 17, wherein the material is used for heat management or for thermal insulation.

19. The process according to claim 16, wherein the absorption of visible light by the material is regulated.

20. The process according to claim 19, wherein the material is used for brightness management.

21. The process according to claims 16 to 20, wherein the material is a window.

22. A compound of the general formula (I) according to claim 1, wherein
R¹, R² are the same or different and are each independently C₁-C₈-alkylamino, C₁-C₈-dialkylamino, arylamino, diarylamino, N-heterocyclyl, triarylaminyl,
R³, R⁴ are the same or different and are each independently C₁-C₈-alkylene, arylene,
X are the same or different and are each independently halogen, cyano, or a group 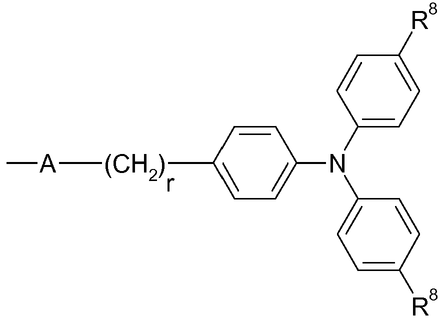 where
A is S, SO₂,
r is 2, 3, 4, 5, 6,
R⁸ is C₁-C₂₀-alkyl, C₁-C₂₀-alkoxy,
n, p are 0, 1 or 2,
m is 0 or 1,
and where the substituents R¹, R² may each be substituted at any position, but not more than five times, by C₁-C₂₀-alkyl, C₁-C₂₀-alkoxy.

23. A material in contact with compounds according to claim 22.

24. A polymer, coating or glass comprising compounds according to claim 22.

## Revendications

1. Procédé pour la modification de l'absorption du rayonnement électromagnétique par un ou plusieurs composés de formule générale (I) 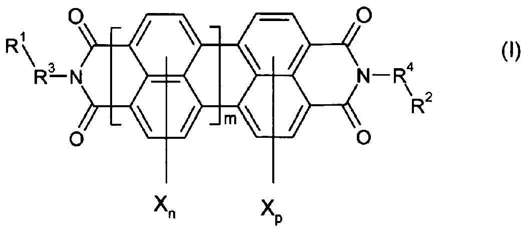 **caractérisé en ce que** ces composés sont irradiés par un rayonnement électromagnétique d'une longueur d'onde de 300 à 750 nm et l'irradiation par un rayonnement électromagnétique d'une longueur d'onde de 300 à 750 nm résulte en un déplacement bathochrome du spectre d'absorption des composés de formule générale (I), les symboles et les indices ayant la signification suivante
R¹, R² indépendamment l'un de l'autre, en étant identiques ou différents, amino, C₁-C₈-alkylamino, C₁-C₈-dialkylamino, arylamino, diarylamino, N-hétérocyclyle, triarylaminyle, hydroxy, C₁-C₈-alcoxy, aryloxy,
R³, R⁴ indépendamment l'un de l'autre, en étant identiques ou différents, une simple liaison, C₁-C₈-alkylène, C₃-C₆-cycloalkylène, arylène, C₈-C₁₄-phénylalkylène,
X indépendamment l'un de l'autre, en étant identiques ou différents, C₁-C₂₀-alcoxy, C₁-C₂₀-alkylthio, aryloxy, arylthio, halogène, cyano, CO₂R, SO₃R, SO₂R, ou un groupe 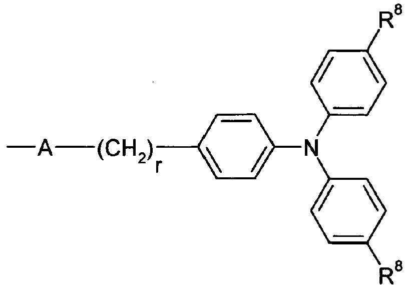 où
A signifie S, SO₂,
r vaut 2, 3, 4, 5, 6,
R⁸ représente C₁-C₂₀-alkyle, C₁-C₂₀-alcoxy, et si m = 1, différents X peuvent représenter, ensemble, un groupe thio,
R représentent, indépendamment l'un de l'autre, en étant identiques ou différents, H, C₁-C₈-alkyle, aryle
n, p valent 0, 1, 2, 3 ou 4,
m vaut 0, 1, 2 ou 3,
les substituants R, R¹, R², R³ ou R⁴ pouvant être interrompus, à chaque fois en une position quelconque, par un ou plusieurs hétéroatomes, le cas échéant saturés par H, le nombre de ces hétéroatomes n'étant pas supérieur à quatre, et/ou pouvant être substitués, à chaque fois en une position quelconque, mais pas plus de cinq fois, par NR⁵R⁶, CONR⁵R⁶, COOM, COOR⁵, SO₃M, SO₃R⁵, où
R⁵, R⁶ représentent, indépendamment l'un de l'autre, en étant identiques ou différents, H, C₁-C₈-alkyle, aryle
M signifie H, métal alcalin, NR⁷₄,
R⁷ indépendamment l'un de l'autre, signifient H, C₁-C₈-alkyle,
CN, NO₂, C₁-C₂₀-alkyle, C₁-C₂₀-alcoxy, aryle, aryloxy, des hétérocycles, des hétéroatomes ou des halogènes, ceux-ci pouvant également être substitués au maximum deux fois par les groupes mentionnés.

2. Procédé selon la revendication 1, les symboles et les indices présentant les significations suivantes,
R¹, R² indépendamment l'un de l'autre, en étant identiques ou différents, amino, C₁-C₈-alkylamino, C₁-C₈-dialkylamino, arylamino, diarylamino, N-hétérocyclyle, N,N-diazylanilin-4-yle,
R³, R⁴ indépendamment l'un de l'autre, en étant identiques ou différents, une simple liaison, C₁-C₈-alkylène,
X indépendamment l'un de l'autre, en étant identiques ou différents, C₁-C₂₀-alcoxy, aryloxy, halogène, cyano, SO₂R, ou un groupe 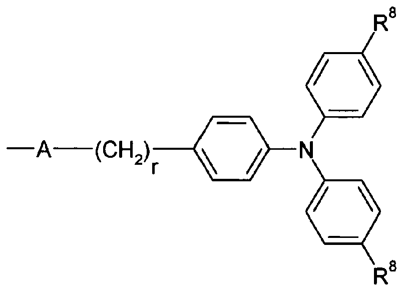 où
A signifie S, SO₂,
r vaut 2, 3, 4, 5, 6,
R⁸ signifie C₁-C₂₀-alkyle, C₁-C₂₀-alcoxy,
m vaut 1 ou 2,
et les substituants R¹, R², R³ ou R⁴ pouvant à chaque fois être substitués ou être interrompus par des hétéroatomes à chaque fois en une position quelconque selon la revendication 1 et tous les autres symboles et indices ayant la signification selon la revendication 1.

3. Procédé selon la revendication 1, les symboles et les indices présentant les significations suivantes,
R¹, R² tous les deux amino, C₁-C₈-alkylamino, C₁-C₈-dialkylamino, arylamino, diarylamino, N-hétérocyclyle, N,N-diazylanilin-4-yle,
R³, R⁴ indépendamment l'un de l'autre, en étant identiques ou différents, C₁-C₈-alkylène,
X indépendamment l'un de l'autre, en étant identiques ou différents, halogène, cyano, aryloxy, ou un groupe 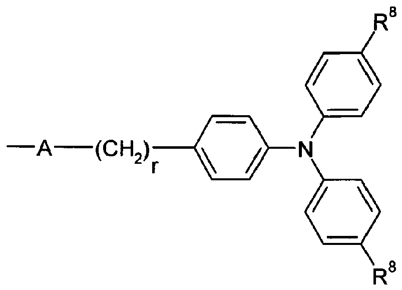 où
A signifie S, SO₂,
r vaut 2, 3, 4, 5, 6,
R⁸ signifie C₁-C₂₀-alkyle, C₁-C₂₀-alcoxy,
m vaut 1 ou 2,
et les substituants R¹, R², R³ ou R⁴ pouvant à chaque fois être substitués ou être interrompus à chaque fois par des hétéroatomes en une position quelconque selon la revendication 1 et tous les autres symboles et indices ayant la signification selon la revendication 1.

4. Procédé selon les revendications 1 à 3, **caractérisé en ce que** l'irradiation par un rayonnement électromagnétique d'une longueur d'onde de 300 à 750 nm résulte en une modification de la couleur perceptible visuellement par les composés de formule générale (I).

5. Procédé selon les revendications 1 à 3, **caractérisé en ce que** l'irradiation par un rayonnement électromagnétique d'une longueur d'onde de 300 à 750 nm résulte en une absorption par les composés de formule générale (I) dans l'infrarouge.

6. Utilisation du procédé selon la revendication 1 pour la détection du marquage de matériaux.

7. Utilisation selon la revendication 6 pour la détection du marquage de papier.

8. Utilisation selon la revendication 6 pour la détection du marquage d'huile minérale.

9. Utilisation du procédé selon la revendication 1 pour provoquer une modification de couleur.

10. Utilisation selon la revendication 9 pour provoquer une modification de couleur du papier ou sur du papier.

11. Utilisation du procédé selon la revendication 1 pour la soudure au laser.

12. Utilisation du procédé selon la revendication 1 dans la gestion de la chaleur.

13. Utilisation du procédé selon la revendication 1 pour la mise à disposition de photo-initiateurs.

14. Utilisation du procédé selon la revendication 1 pour la mise à disposition de pièges de radicaux.

15. Utilisation du procédé selon la revendication 1 pour la détection de l'oxygène.

16. Procédé pour réguler l'absorption ou la transmission d'un rayonnement électromagnétique par un matériau, **caractérisé en ce qu'**un ou plusieurs composés de formule générale (I) selon la revendication 1 sont mis en contact avec ce matériau et ces composés sont irradiés par un rayonnement électromagnétique d'une longueur d'onde de 300 à 750 nm et l'irradiation par un rayonnement électromagnétique d'une longueur d'onde de 300 à 750 nm résulte en un déplacement bathochrome du spectre d'absorption des composés de formule générale (I).

17. Procédé selon la revendication 16, **caractérisé en ce qu'**une régulation de l'absorption d'un rayonnement IR par le matériau se produit.

18. Procédé selon la revendication 17, le matériau étant utilisé pour la gestion de la chaleur ou pour l'isolation thermique.

19. Procédé selon la revendication 16, **caractérisé en ce qu'**une régulation de l'absorption de la lumière visible par le matériau se produit.

20. Procédé selon la revendication 19, le matériau étant utilisé pour la gestion de la clarté.

21. Procédé selon les revendications 16 à 20, le matériau étant une fenêtre.

22. Composés de formule générale (I) selon la revendication 1, où
R¹, R² signifient, indépendamment l'un de l'autre, en étant identiques ou différents, C₁-C₈-alkylamino, C₁-C₈-dialkylamino, arylamino, diarylamino, N-hétérocyclyle, triarylaminyle,
R³, R⁴ signifient, indépendamment l'un de l'autre, en étant identiques ou différents, C₁-C₈-alkylène, arylène
X signifient, indépendamment l'un de l'autre, en étant identiques ou différents, halogène, cyano, ou un groupe 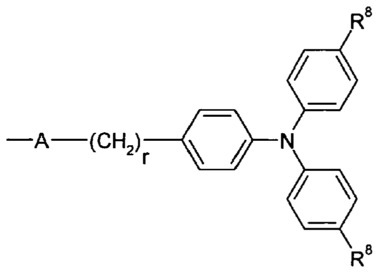 où
A signifie S, SO₂,
r vaut 2, 3, 4, 5, 6,
R⁸ signifie C₁-C₂₀-alkyle, C₁-C₂₀-alcoxy,
n, p vaut 0, 1 ou 2,
m valent 0 ou 1,
et les substituants R¹, R² pouvant à chaque fois être substitués en une position quelconque, mais pas plus de cinq fois, par C₁-C₂₀-alkyle, C₁-C₂₀-alcoxy.

23. Matériaux en contact avec des composés selon la revendication 22.

24. Polymères, laques ou verres contenant des composés selon la revendication 22.
